# EUROPEAN PATENT SPECIFICATION

(11) **EP 4 439 042 B1**
(45) Date of publication and mention of the grant of the patent: **09.07.2025**
(21) Application number: 24166783.1
(22) Date of filing: 27.03.2024
(51) Int. Cl.: G01N 15/1429, G01N 15/1434, G01N 15/14

(54) **METHOD FOR ANALYZING SPECIMEN**
VERFAHREN ZUR ANALYSE EINER PROBE
PROCÉDÉ D'ANALYSE D'UN ÉCHANTILLON

(30) Priority: 31.03.2023 JP 2023059067
(43) Date of publication of application: 02.10.2024
(73) Proprietor: SYSMEX CORPORATION, Kobe-shi Hyogo 651-0073 (JP)
(72) Inventor: TOYA, Yuji, Kobe-shi, Hyogo, 651-0073 (JP); KANEMURA, Emi, Kobe-shi, Hyogo, 651-0073 (JP)
(74) Representative: Hoffmann Eitle

(56) References cited:
- CN-A- 104 215 561
- JP-A- 2014 001 949
- US-A- 6 004 816

## Description

### FIELD OF THE INVENTION

The present invention relates to a method for analyzing a specimen.

### BACKGROUND

In analysis of a specimen containing a blood cell such as blood and body cavity fluid, normal white blood cells are classified into five subpopulations: lymphocytes, monocytes, neutrophils, eosinophils, and basophils. In the field of clinical examination, a cell that is usually hardly present in a specimen such as blood or body cavity fluid is called an abnormal cell. Detecting or classifying an abnormal cell in a specimen is extremely useful for diagnosing a health condition or a disease of a subject. U.S. Patent Application Publication No. 2012/0282601 describes detecting an atypical lymphocyte, an abnormal lymphocyte, and a blast as abnormal cells. US6004816A describes a method for classification and counting of leukocytes by flow cytometry, using a fluorescent dye that specifically binds to RNA to increase in fluorescence intensity.

### SUMMARY OF THE INVENTION

In addition to the above atypical lymphocyte, abnormal lymphocyte, and blasts, examples of abnormal cells are a DNA aneuploid white blood cell (hereinafter, "DNA aneuploid cell"), an erythroblast, an immature granulocyte, and the like. As described above, there are many types of abnormal cells, but the conventional analysis method can detect or classify only a limited type of abnormal cell. There is also an analysis method capable of coping with more types of abnormal cells by performing a plurality of measurements by changing reagents or the like. However, such an analysis method requires cost and time. An object of the present invention is to provide a means capable of coping with detection of a plurality of types of abnormal cells such as a DNA aneuploid cell and a blast in one measurement.

The present inventors have found that a plurality of types of abnormal cells can be detected by staining and measuring particles such as a blood cell in a specimen with two types of fluorescent dyes, a fluorescent dye that specifically binds to DNA and a fluorescent dye that specifically binds to RNA, and have completed the present invention.

The present invention provides a method for analyzing a specimen, including: acquiring first fluorescence information, second fluorescence information, and scattered light information generated by irradiating a particle in a measurement sample stained with a first fluorescent dye and a second fluorescent dye with light; specifying a particle having a first characteristic based on the first fluorescence information, the second fluorescence information, and the scattered light information; and outputting information indicating presence or absence of a first abnormal cell, information indicating presence or absence of a second abnormal cell, and information indicating presence or absence of a third abnormal cell based on information of the particle having a first characteristic, in which the measurement sample is prepared by mixing a specimen collected from a subject, the first fluorescent dye, and the second fluorescent dye, the first fluorescence information is fluorescence intensity from the first fluorescent dye, and the second fluorescence information is fluorescence intensity from the second fluorescent dye, and the first fluorescent dye is a fluorescent dye that specifically binds to DNA, and the second fluorescent dye is a fluorescent dye that specifically binds to RNA.

Further, the present invention provides a method for analyzing a specimen including: acquiring first fluorescence information, second fluorescence information, and scattered light information generated by irradiating a particle in a measurement sample stained with a first fluorescent dye and a second fluorescent dye with light; classifying white blood cells contained in the measurement sample into at least five subpopulations based on the first fluorescence information, the second fluorescence information, and the scattered light information; and separately detecting at least DNA aneuploid cells, an abnormal lymphocyte, and a blast based on the first fluorescence information, the second fluorescence information, and the scattered light information for a measurement sample that cannot be classified into five subpopulations, in which the measurement sample is prepared by mixing a specimen collected from a subject, the first fluorescent dye, and the second fluorescent dye, the first fluorescence information is fluorescence intensity from the first fluorescent dye, and the second fluorescence information is fluorescence intensity from the second fluorescent dye, and the first fluorescent dye is a fluorescent dye that specifically binds to DNA, and the second fluorescent dye is a fluorescent dye that specifically binds to RNA.

Furthermore, the present invention provides a staining reagent for use in the analysis method, containing a first fluorescent dye and a second fluorescent dye.

According to the present invention, there are provided an analysis method that enables detection of a plurality of types of abnormal cells, and a staining reagent for use in the method.

### BRIEF DESCRIPTION OF THE DRAWINGS

Fig. 1 is a perspective view showing an analysis system suitable for a method for analyzing a specimen of the present embodiment.
Fig. 2 is a diagram showing a fluid circuit including a specimen suction section, a sample preparation section, and a flow cytometer (FCM) detection section in a measurement unit.
Fig. 3 is a block diagram showing a configuration of the measurement unit.
Fig. 4 is a schematic diagram showing an example of a configuration of an optical system of the FCM detection section.
Fig. 5 is a schematic diagram showing that various types of light emitted when a particle passing through a flow cell is irradiated with light are received by the optical system of the FCM detection section.
Fig. 6 is a block diagram showing a configuration of an analysis unit.
Fig. 7 is a flowchart showing a flow of operation of the analysis system.
Fig. 8 is a flowchart showing procedures of measurement processing of Embodiments 1 and 2.
Fig. 9A is a flowchart showing procedures of analysis processing of Embodiment 1.
Fig. 9B is a flowchart showing procedures of analysis processing of Embodiment 1.
Fig. 9C is a flowchart showing procedures of analysis processing of Embodiment 1.
Fig. 9D is a flowchart showing procedures of analysis processing of Embodiment 1.
Fig. 10A is a schematic diagram illustrating positions at which each subpopulation of white blood cells appears in a scattergram in which the side scattered light intensity is taken on a horizontal axis and the first fluorescence intensity is taken on a vertical axis (first scattergram). Fig. 10B is a schematic diagram illustrating positions at which each subpopulation of white blood cells appears in a scattergram in which the side scattered light intensity is taken on a horizontal axis and the second fluorescence intensity is taken on a vertical axis (second scattergram).
Fig. 11A is a schematic diagram showing that the number of particle indicating a second fluorescence intensity equal to or greater than a first threshold value corresponding to second fluorescence information has increased in lymphocyte population in the second scattergram. Fig. 11B is a schematic diagram illustrating a position at which a particle indicating a higher first fluorescence intensity than that of the lymphocyte population appears in the first scattergram.

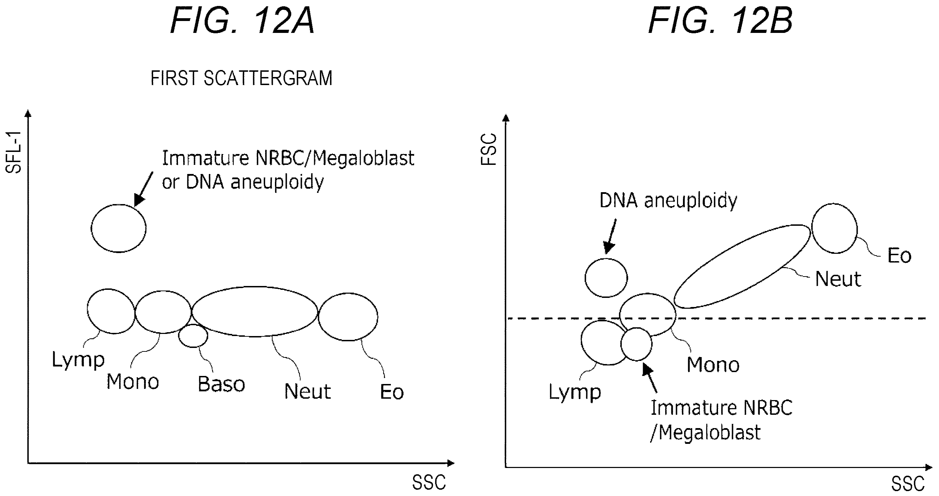

Fig. 12A is a schematic diagram showing that particles indicating a higher first fluorescence intensity than that of the lymphocyte population form a discrete population from each subpopulation of white blood cells in the first scattergram. Fig. 12B is a schematic diagram showing that in a scattergram in which the side scattered light intensity is taken on a horizontal axis and the forward scattered light intensity is taken on a vertical axis, a particle indicating a higher first fluorescence intensity than that of the lymphocyte population has appeared in a region where the forward scattered light intensity is higher than that of the lymphocyte population.
Fig. 13A is a schematic diagram illustrating a region where a particle having a second characteristic appears in the second scattergram. Fig. 13B is a schematic diagram illustrating a region where a particle having a third characteristic appears in the second scattergram.
Fig. 14 is a flowchart showing procedures of analysis processing of a modification of Embodiment 1.
Fig. 15A is a schematic diagram illustrating a position at which a lymphoblast appears in the first scattergram. Fig. 15B is a schematic diagram illustrating a position at which a myeloblast appears in the first scattergram.
Fig. 16A is a flowchart showing procedures of analysis processing of Embodiment 2.
Fig. 16B is a flowchart showing procedures of analysis processing of Embodiment 2.
Fig. 17A is a schematic diagram illustrating positions at which debris, platelet clumps, and malaria-infected red blood cells appear in the first scattergram. Fig. 17B is a schematic diagram illustrating positions at which platelet clumps and malaria-infected red blood cells appear in a scattergram in which the forward scattered light pulse width is taken on a horizontal axis and the forward scattered light intensity is taken on a vertical axis.
Fig. 18A is a schematic diagram illustrating a position at which a nucleated red blood cell appears in the first scattergram. Fig. 18B is a schematic diagram illustrating a position at which a nucleated red blood cell appears in the second scattergram.
Fig. 19 is a flowchart showing procedures of measurement processing of Embodiment 3.
Fig. 20A is a flowchart showing procedures of analysis processing of Embodiment 3.
Fig. 20B is a flowchart showing procedures of analysis processing of Embodiment 3.
Fig. 20C is a flowchart showing procedures of analysis processing of Embodiment 3.
Fig. 21A is a schematic diagram illustrating positions at which a mononuclear cell population and a polymorphonuclear cell population of white blood cells appear in the first scattergram. Fig. 21B is a schematic diagram illustrating a position at which a population of bacteria or fungi appears in the first scattergram.
Fig. 22A is a schematic diagram illustrating positions at which a lymphocyte population, a monocyte population, and a polymorphonuclear cell population of white blood cells appear in the second scattergram. Fig. 22B is a schematic diagram illustrating a position at which a population of tissue-derived cells appears in the second scattergram.
Fig. 23 is a schematic diagram illustrating a position at which a DNA aneuploid cell appears in the first scattergram.
Fig. 24A is a view showing an example of a staining reagent for use in the method for analyzing a specimen of the present embodiment. In this figure, one reagent container containing a staining reagent containing a first fluorescent dye and a second fluorescent dye is packed in a box.
Fig. 24B is a view showing an example of a staining reagent for use in the method for analyzing a specimen of the present embodiment. In this figure, a reagent container containing a reagent containing a first fluorescent dye and a reagent container containing a reagent containing a second fluorescent dye are packed in a box.
Fig. 25 shows first and second scattergrams of Reference Example 1.
Fig. 26 shows first and second scattergrams of Example 1.
Fig. 27 shows first and second scattergrams of Example 1.
Fig. 28 shows first and second scattergrams of Example 2.
Fig. 29 shows various scattergrams of Example 3.
Fig. 30 shows various scattergrams of Example 3.
Fig. 31 shows first and second scattergrams of Example 4.
Fig. 32 shows first and second scattergrams of Example 5.
Fig. 33 shows various scattergrams of Example 6.
Fig. 34 shows various scattergrams of Example 6.
Fig. 35 shows first and second scattergrams of Example 7.
Fig. 36 shows first and second scattergrams of Example 7.
Fig. 37 shows first and second scattergrams of Example 7.
Fig. 38 shows first and second scattergrams of Example 7.
Fig. 39 shows first and second scattergrams of Example 7.
Fig. 40 shows a second scattergram and a fluorescence histogram of Reference Example 2.
Fig. 41 shows images acquired by an imaging flow cytometer of Reference Example 3.
Fig. 42 shows a scattergram and a fluorescence histogram of Reference Example 4.
Fig. 43 is a first scattergram of Reference Example 4.
Fig. 44 is a graph showing a correlation between the method for analyzing a specimen of the present embodiment and a standard measurement method.
Fig. 45 shows first and second scattergrams of Example 8.
Fig. 46 shows first and second scattergrams of Example 9.
Fig. 47 shows a microscopic image and first and second scattergrams of Example 10.
Fig. 48 shows a microscopic image and first and second scattergrams of Example 11.
Fig. 49 shows first and second scattergrams and a fluorescence histogram of Example 11.

### DETAILED DESCRIPTION OF THE PREFERRED EMBODIMENTS

### (Analysis system)

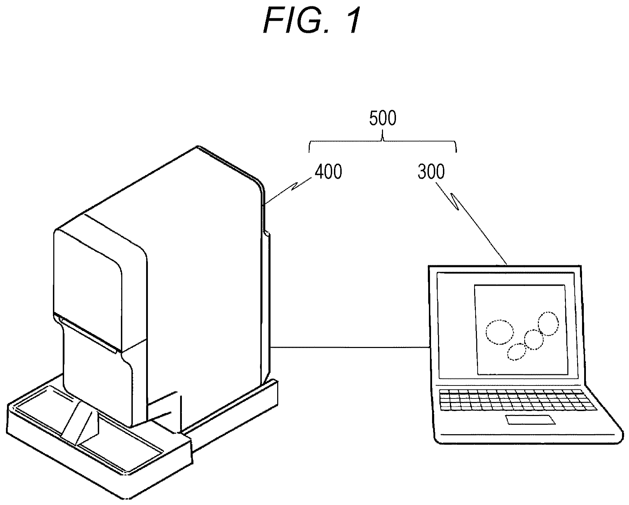

First, an analysis system suitable for a method for analyzing a specimen of the present embodiment will be described with reference to Fig. 1. An analysis system 500 includes a measurement unit 400 that is a measuring device and an analysis unit 300 that is an analyzer. The analysis unit 300 is, for example, a personal computer in which software for analyzing a specimen to be measured is incorporated. The analysis unit 300 also executes operation control of the measurement unit 400. The analysis system 500 may have a configuration in which the analysis unit 300 is provided in the measurement unit 400.

The measurement unit 400 is a unit for measuring a specimen. The measurement unit 400 includes a flow cytometer. In the measurement unit 400, a specimen and a reagent are mixed to prepare a measurement sample. For the preparation of the measurement sample, a staining reagent containing a first fluorescent dye and a second fluorescent dye and a hemolysis reagent containing a surfactant are used. Details of the specimen, the staining reagent, and the hemolysis reagent will be described later. In the analysis of particles in the specimen by this analysis system, particles in the measurement sample stained with the first and second fluorescent dyes are analyzed. The "particles in the measurement sample" refers to a tangible component contained in the measurement sample that can be individually measured by an FCM detection section 460 described later. Examples of the particles in the measurement sample include cells, platelet clumps, remnants of hemolyzed red blood cells (red blood cell ghost), lipid particles, fungi, bacteria, and the like contained in the specimen.

The measurement sample is measured by the FCM detection section 460 of the measurement unit 400. An optical signal related to fluorescence emitted from each of the first and second fluorescent dyes of the stained particles in the measurement sample and an optical signal related to scattered light emitted from the particles are acquired. The acquired optical signals are A/D converted to acquire digital data. The analysis unit 300 analyzes the digital data acquired by the measurement unit 400 to detect or classify particles in the specimen.

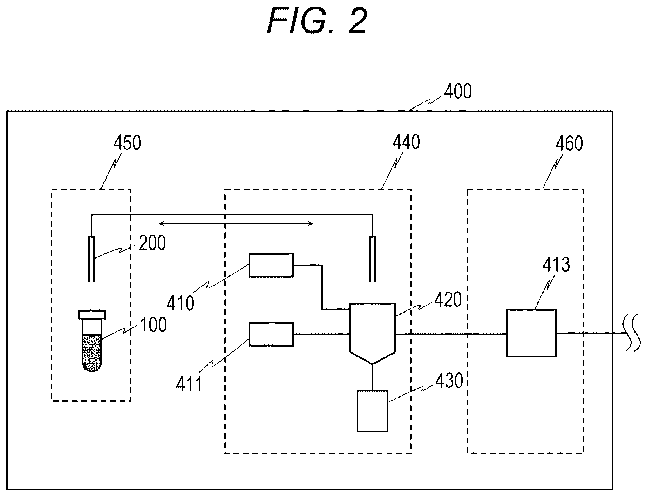

A configuration of a fluid system in the measurement unit 400 will be described with reference to Fig. 2. The measurement unit 400 includes a sample preparation section 440, a specimen suction section 450, and an FCM detection section 460. The sample preparation section 440 includes a reagent container 410, a reagent container 411, a reaction chamber 420, and a waste liquid chamber 430. For example, the reagent container 410 contains a hemolysis reagent, and the reagent container 411 contains a staining reagent. The reagent container 410 and the reagent container 411 are each connected to the reaction chamber 420 by a liquid feeding tube. The sample preparation section 440 injects the hemolysis reagent and the staining reagent into the reaction chamber 420 via each liquid feeding tube. The specimen suction section 450 includes a suction tube 200. The suction tube 200 sucks the specimen stored in a blood collection tube 100. The suction tube 200 discharges the sucked specimen to the reaction chamber 420. The reaction chamber 420 is connected to a flow cell 413 of the FCM detection section 460 by a liquid feeding tube.

The reaction chamber 420 is a container for preparing a measurement sample. In the reaction chamber 420, a specimen, a stain solution containing a first fluorescent dye and a second fluorescent dye, and a hemolysis reagent are mixed to prepare a measurement sample. The measurement sample in the reaction chamber 420 is supplied to the flow cell 413 of the FCM detection section 460 via the liquid feeding tube and measured. The FCM detection section 460 acquires various optical signals emitted from individual particles in the measurement sample. After the measurement by the FCM detection section 460 is completed, the measurement sample remaining in the reaction chamber 420 is discarded in the waste liquid chamber 430. The reaction chamber 420 is cleaned by a cleaning mechanism (not shown) before the next measurement sample is prepared.

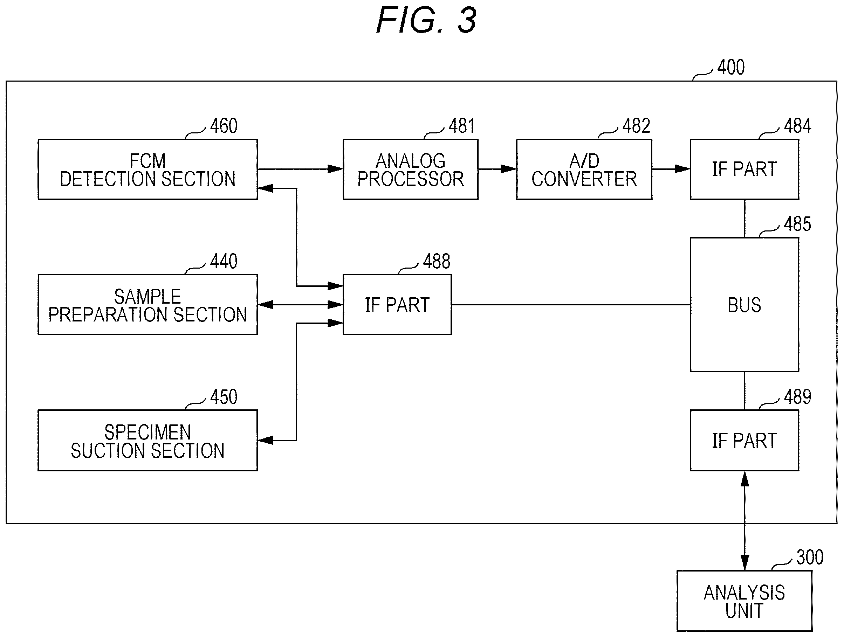

Electrical connection of each section in the measurement unit 400 will be described with reference to Fig. 3. The sample preparation section 440 and the specimen suction section 450 are connected to the FCM detection section 460 via an interface (IF) part 488. The FCM detection section 460 is connected to an analog processor 481 and an A/D converter 482. The analog processor 481 processes an analog signal output from the FCM detection section 460, and the A/D converter 482 converts an analog signal output from the analog processor 481 into a digital signal. The measurement unit 400 is connected to the analysis unit 300 via an IF part 489. An IF part 484, the IF part 488, and the IF part 489 are connected to a bus 485.

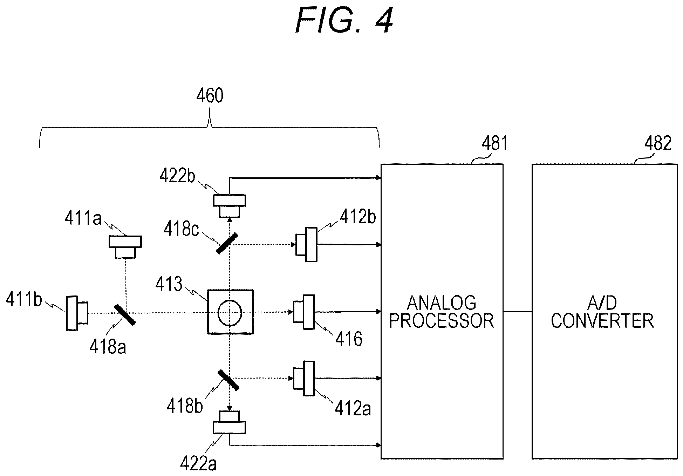

The FCM detection section 460, the analog processor 481, and the A/D converter 482 will be described with reference to Fig. 4. The FCM detection section 460 includes a first light source 411a and a second light source 411b, a flow cell 413, dichroic mirrors 418a, 418b, and 418c, side scattered light receiving elements 412a and 412b, a forward scattered light receiving element 416, and side fluorescent light receiving elements 422a and 422b. The first light source 411a and the second light source 411b emit excitation lights having wavelengths different from each other. For example, the first light source 411a emits light of a first wavelength capable of exciting the first fluorescent dye, and the second light source 411b emits light of a second wavelength capable of exciting the second fluorescent dye. The first wavelength is, for example, 315 nm or more and 490 nm or less, preferably 400 nm or more and 450 nm or less, and more preferably 400 nm or more and 410 nm or less. The second wavelength is, for example, 610 nm or more and 750 nm or less, preferably 620 nm or more and 700 nm or less, and more preferably 633 nm or more and 643 nm or less. As the first and second light sources, for example, semiconductor laser light sources can be used.

The measurement sample prepared in the reaction chamber 420 flows into the flow cell 413 of the FCM detection section 460. In the example of Fig. 4, the measurement sample flows in a direction perpendicular to a paper surface. In a state where the measurement sample is flowing into the flow cell 413, light emitted from the first light source 411a is reflected by the dichroic mirror 418a, and the light is applied to individual particles in the measurement sample flowing in the flow cell 413. The light emitted from the second light source 411b is transmitted through the dichroic mirror 418a, and the light is applied to the individual particles in the measurement sample flowing in the flow cell 413.

In the example of Fig. 4, the forward scattered light receiving element 416 is disposed to receive forward scattered light emitted from the cell based on the light emitted from the second light source 411b. Alternatively, the forward scattered light receiving element 416 may be disposed to receive forward scattered light emitted from the particles based on the light emitted from the first light source 411a. Alternatively, another light receiving element may be disposed in order to receive forward scattered light emitted from the particles based on the light emitted from the first light source 411a. The forward scattered light is, for example, scattered light having a light receiving angle of 0 degrees to about 20 degrees. The forward scattered light receiving element 416 is, for example, a photodiode. The side scattered light corresponding to light emitted from the first light source 411a (the first side scattered light) is reflected by the dichroic mirror 418b, and the first side scattered light is received by the side scattered light receiving element 412a. The side scattered light corresponding to light emitted from the second light source 411b (the second side scattered light) is reflected by the dichroic mirror 418c, and the second side scattered light is received by the side scattered light receiving element 412b. The side scattered light is, for example, scattered light having a light receiving angle of about 20 degrees to about 90 degrees. The side scattered light receiving elements 412a and 412b are, for example, photodiodes.

The side fluorescent light corresponding to light generated by exciting the first fluorescent dye (the first side fluorescent light) is transmitted through the dichroic mirror 418b, and the first side fluorescent light is received by the side fluorescent light receiving element 422a. The side fluorescent light corresponding to light generated by exciting the second fluorescent dye (the second side fluorescent light) is transmitted through the dichroic mirror 418c, and the second side fluorescent light is received by the side fluorescent light receiving element 422b. The side fluorescent light receiving elements 422a and 422b are, for example, avalanche photodiodes.

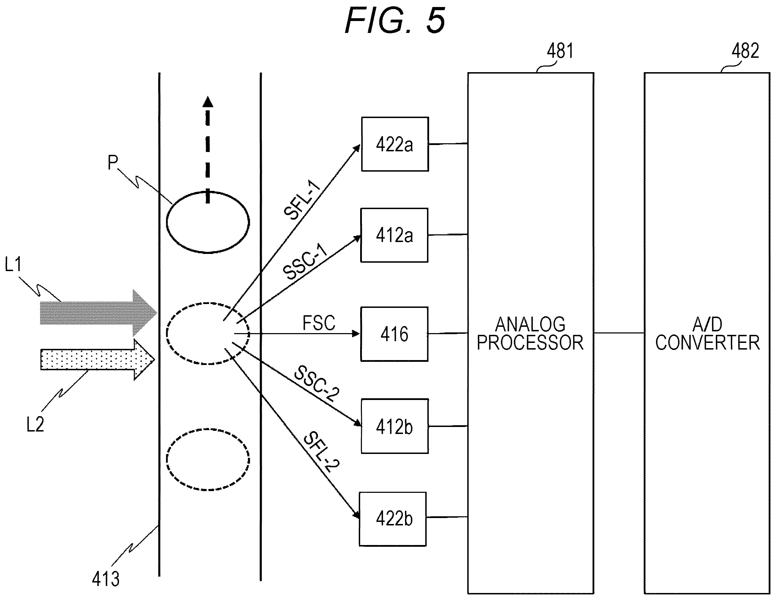

A relationship between various types of light emitted when a particle P passing through the flow cell 413 is irradiated with light and an optical system of the FCM detection section 460 will be described with reference to Fig. 5. In Fig. 5, the light emitted from the first light source 411a is light L1 having a first wavelength, and the light emitted from the second light source 411b is light L2 having a second wavelength. When the particle P passing through the flow cell 413 is irradiated with the lights L1 and L2, forward scattered light (FSC) is generated forward with respect to the traveling direction of the light. In the example of Fig. 5, since the light receiving element 416 receives the forward scattered light corresponding to the light emitted from the second light source 411b, only a second forward scattered light corresponding to the light of the second wavelength is shown, and a first forward scattered light corresponding to the light of the first wavelength is omitted. First side scattered light (SSC-1) corresponding to the light of the first wavelength and first side fluorescent light (SFL-1) excited by the light of the first wavelength are generated laterally with respect to the traveling direction of the light. Further, second side scattered light (SSC-2) corresponding to the light of the second wavelength and second side fluorescent light (SFL-2) excited by the light of the second wavelength are generated laterally with respect to the traveling direction of the light. As described above, FSC, SSC-1, SFL-1, SSC-2, and SFL-2 are received by the light receiving elements 416, 412a, 422a, 412b, and 422b, respectively. Each light receiving element outputs a wave-shaped electric signal including a pulse corresponding to received light intensity (also referred to as an analog signal). Hereinafter, an analog signal corresponding to FSC is also referred to as a "forward scattered light signal", an analog signal corresponding to SSC-1 is also referred to as a "first side scattered light signal", an analog signal corresponding to SFL-1 is also referred to as a "first fluorescence signal", an analog signal corresponding to SSC-2 is also referred to as a "second side scattered light signal", and an analog signal corresponding to SFL-2 is also referred to as a "second fluorescence signal". One pulse of each analog signal corresponds to one particle (for example, one cell).

Analog signals corresponding to various types of light are input to the analog processor 481, and processing such as noise removal and smoothing is performed. The A/D converter 482 samples analog signals output from the analog processor 481 at a predetermined sampling rate (for example, sampling at 1024 points at intervals of 10 nanoseconds, sampling at 128 points at intervals of 80 nanoseconds, sampling at 64 points at intervals of 160 nanoseconds, or the like). The A/D converter 482 digitizes the sampled analog signal to generate waveform data. The A/D converter 482 samples and digitizes five types of analog signals corresponding to individual cells flowing through the flow cell 413 to generate waveform data of the forward scattered light signal, the first side scattered light signal, the first fluorescence signal, the second side scattered light signal, and the second fluorescence signal. Furthermore, the A/D converter 482 calculates feature parameters representing morphological features of individual cell from the waveform data of each signal. Examples of such feature parameters include a peak value (height of a pulse peak), pulse width, pulse area, transmittance, Stokes shift, ratio, temporal change, values correlated thereto, and the like.

Optical information may be the feature parameter described above. The optical information includes at least one of the forward scattered light information, the first fluorescence information, the second fluorescence information, the first side scattered light information, or the second side scattered light information. The first fluorescence information is not particularly limited as long as it is information reflecting the amount of fluorescent dye that has stained DNA in a nucleated cell. The second fluorescence information is not particularly limited as long as it is information reflecting the amount of fluorescent dye that has stained RNA in a nucleated cell. As the first and second fluorescence information, a peak value of the first fluorescence signal (hereinafter, also referred to as "first fluorescence intensity") and a peak value of the second fluorescence signal (hereinafter, also referred to as "second fluorescence intensity") are respectively preferable. The first and second side scattered light information is not particularly limited as long as the side scattered light information is information reflecting internal information such as complexity of cell structure, granule characteristics, nuclear structure, and degree of lobulation. As the first and second side scattered light information, a peak value of the first side scattered light signal (hereinafter, also referred to as "first side scattered light intensity") and a peak value of the second side scattered light signal (hereinafter, also referred to as "second side scattered light intensity") are respectively preferable. The forward scattered light information is not particularly limited as long as it is information reflecting cell size. As the forward scattered light information, a peak value of the forward scattered light signal (hereinafter, also referred to as "forward scattered light intensity") is preferable.

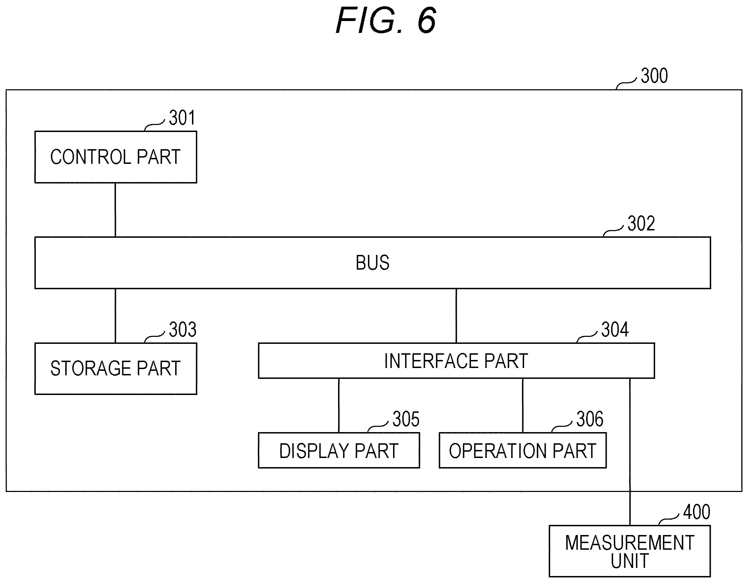

Electrical connection of each section in the analysis unit 300 will be described with reference to Fig. 6. The analysis unit 300 is electrically connected to the measurement unit 400 via an interface part 304. The interface part 304 is, for example, a USB interface. The analysis unit 300 includes a control part 301, a bus 302, a storage part 303, the interface part 304, a display part 305, and an operation part 306. The analysis unit 300 includes, for example, a personal computer (see the analysis unit 300 in Fig. 1). The analysis unit 300 controls the measurement unit 400 of the analysis system 500 by executing a program stored in the storage part 303. The analysis unit 300 analyzes the data acquired by the measurement unit 400. The analysis unit 300 displays the analysis result on the display part 305. The analysis unit 300 may classify cells based on the forward scattered light intensity, the first side scattered light intensity, the first fluorescence intensity, the second side scattered light intensity, and the second fluorescence intensity.

The storage part 303 stores, for example, a program for controlling the measurement unit 400, a program for analyzing data acquired by the measurement unit 400, and the like. The display part 305 displays, for example, an analysis result of data acquired by the measurement unit 400. The operation part 306 includes a keyboard, a mouse, or a pointing device including a touch panel.

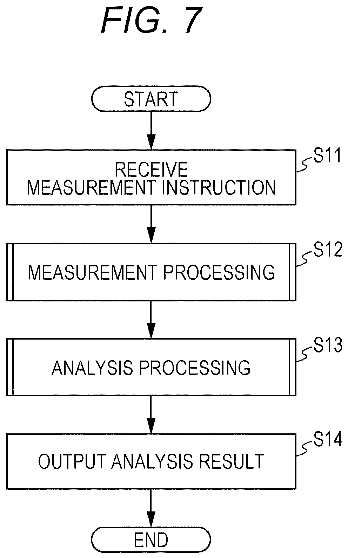

An example of the operation of the analysis system 500 will be described with reference to Fig. 7, but it is not limited to this example. In step S11, the control part 301 of the analysis unit 300 receives an instruction to start measurement from a user via the operation part 306. Upon receiving the instruction to start measurement, the control part 301 transmits instruction data for instructing the measurement start to the measurement unit 400. The measurement unit 400 receives the instruction data to execute measurement processing in step S12. In the measurement processing, the measurement unit 400 prepares a measurement sample from a specimen, and the measurement unit 400 measures the sample. Then, the measurement unit 400 transmits the data acquired by the measurement to the analysis unit 300. The analysis unit 300 executes analysis processing of step S13 based on the data received from the measurement unit 400. In the analysis processing, white blood cells are classified into subpopulations, and when abnormal cells are present in the specimen, the abnormal cells are detected. After completion of the analysis processing, the control part 301 outputs the analysis result to the display part 305 in step S14. Then, the analysis system ends the operation shown in Fig. 7. Details of the measurement processing in step S12 and the analysis processing in step S13 in Fig. 7 will be described for each of the following embodiments.

The method for analyzing a specimen of the present embodiment includes, for example, Embodiments 1 to 3 as follows. In "Embodiment 1", a measurement sample prepared from a blood sample is measured, and classification of white blood cells into subpopulations and detection of abnormal cells are performed. The abnormal cell in Embodiment 1 is a DNA aneuploid cell, an abnormal lymph, a blast, an immature erythroblast or a megaloblast, an immature granulocyte, and an atypical lymphocyte. Embodiment 1 enables distinction between DNA aneuploid cells, blasts, and abnormal lymph, which has been conventionally difficult. In "Embodiment 2", a measurement sample prepared from a blood sample is measured to specify particles that can interfere with the analysis of Embodiment 1. Then, in the same manner as in Embodiment 1, classification of white blood cells into subpopulations and detection of the abnormal cells are performed. In Embodiment 2, the particles that can interfere with the analysis of Embodiment 1 are platelet clumps, infected cells, and nucleated red blood cells. When these particles are contained in the specimen, it may affect, for example, the classification of white blood cells. For example, in Embodiment 2, by specifying particles that can interfere with such analysis and removing measurement data of the particles, it may contribute to improvement of analysis accuracy of the remaining cells. "Embodiment 3" includes measurement and analysis where the analysis system 500 is capable of making measurements of both blood samples and non-blood samples (hereinafter, the non-blood sample is referred to as "body fluid" or "body fluid sample"). In Embodiment 3, a measurement sample prepared from a blood sample or a body fluid sample is measured, and classification of white blood cells into subpopulations and detection of abnormal cells are performed.

### (Specimen and abnormal cell)

As described above, the specimens of Embodiments 1 and 2 are blood samples, and the specimen of Embodiment 3 is a blood sample or a body fluid sample. Examples of the blood sample include whole blood, a dilution of whole blood, and the like. The whole blood is, for example, peripheral blood collected from a subject. The blood sample may include an anticoagulant. Examples of the anticoagulant include ethylenediaminetetraacetic acid (EDTA), EDTA salts (for example, EDTA · 2K, EDTA · 2Na, and the like), sodium citrate, heparin, warfarin, and the like. Examples of the body fluid sample include body fluids, dilutions of body fluids, and the like. The body fluid is not particularly limited as long as it may contain a blood component. Examples of the body fluid include body cavity fluid, cerebrospinal fluid, joint fluid, peritoneal dialysis drainage, alveolar lavage fluid, and the like. Examples of the body cavity fluid include ascites, pleural effusion, pericardial fluid, and the like. The dilution of whole blood or body fluid is obtained, for example, by diluting whole blood or body fluid with a suitable aqueous solvent. Examples of the aqueous solvent include water, physiological saline, buffer solutions, and the like. The buffer solution preferably has a buffering effect at a pH near neutrality (for example, a pH of 6 or more and 8 or less).

In the present specification, the "blood component" includes a tangible component known to be contained in blood and an abnormal cell. Examples of the tangible component known to be contained in blood include blood cells usually contained in peripheral blood of a healthy person, and the like, such as white blood cells, red blood cells, and platelets. The abnormal cell refers to a tangible component that does not usually appear in blood or body fluid. The abnormal cell also includes microorganisms, infected cells, and non-cellular particles. The microorganism is not particularly limited as long as it has a size detectable by flow cytometry, and examples thereof include bacteria, fungi, and the like. Examples of the infected cell include red blood cells infected with malaria parasites (hereinafter, also referred to as "malaria-infected red blood cell"), cells infected with viruses, and the like. Examples of the non-cellular particle include platelet clumps. Platelet clumps occur in a blood collection tube due to contamination or insufficient mixing of tissue fluid during blood collection or action of EDTA.

Examples of the abnormal cells in blood include blasts, immature granulocytes, atypical lymphocytes, abnormal lymphocytes, DNA aneuploid cells, erythroblasts, megaloblasts, malaria-infected red blood cells, and platelet clumps. The "immature granulocytes" include promyelocytes, myelocytes, and metamyelocytes. The "erythroblasts" are also called nucleated red blood cells (NRBCs), and include proerythroblasts, basophilic erythroblasts, polychromatic erythroblasts, and orthochromatic erythroblasts. Among the erythroblasts, proerythroblasts, basophilic erythroblasts, and polychromatic erythroblasts are also called "immature erythroblasts". The "blasts" include myeloblasts and lymphoblasts. The "DNA aneuploid cell" is a white blood cell showing DNA aneuploidy, and has a larger or smaller amount of DNA than that of normal white blood cell. The "atypical lymphocytes" are also called reactive lymphocytes, and are lymphocytes which are activated and morphologically altered by antigen stimulation. The "abnormal lymphocytes" are morphologically altered tumorous lymphocytes, and are clonal homogeneous cells.

Examples of the abnormal cells in the body fluid include tissue-derived cells, bacteria, and fungi. The "tissue-derived cells" include, for example, histiocytes (macrophages in tissue), mesothelial cells, and tumor cells. The tissue-derived cells are also called "abnormal white blood cells" in the field of clinical examination.

It is extremely rare that a plurality of types of abnormal cells are contained in the same specimen. However, it is unknown what type of abnormal cells the specimen contains before measurement. Since the analysis processing using the above-described analysis system and reagent (described later) corresponds to detection of a plurality of types of abnormal cells, information indicating the presence and absence of a plurality of types of abnormal cells can be provided by one measurement. According to the analysis processing using the above-described analysis system and reagent (described later), it is not necessary to measure each abnormal cell using a dedicated reagent each time.

### (Measurement processing of Embodiment 1)

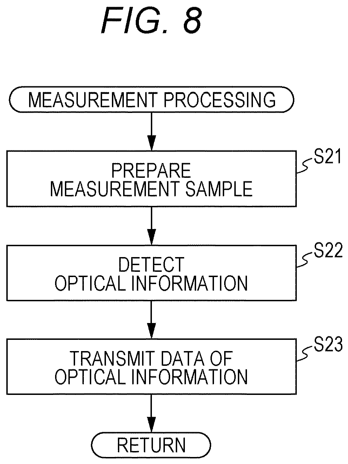

With respect to step S12 in Fig. 7, an example of measurement processing of Embodiment 1 will be described with reference to Fig. 8, but it is not limited to this example. In step S21, the control part 301 of the analysis unit 300 causes the measurement unit 400 to prepare a measurement sample from the blood sample. Specifically, under the condition of preparing a measurement sample from the blood sample, the measurement unit 400 mixes the specimen that is whole blood, the staining reagent, and the hemolysis reagent in the reaction chamber 420 to prepare a measurement sample. In step S22, the control part 301 causes the measurement unit 400 to lead the measurement sample to the FCM detection section 460, and the control part 301 executes optical measurement by a flow cytometry method. As a result, the measurement unit 400 detects optical information on individual particles in the measurement sample, for example, waveform data of FSC, SSC-1, SFL-1, SSC-2, and SFL-2. In step S23, the control part 301 causes the measurement unit 400 to transmit the optical information to the analysis unit 300, and the control part 301 ends the measurement processing. Then, the analysis unit 300 executes the analysis processing in step S13 in Fig. 7.

### (Analysis processing of Embodiment 1)

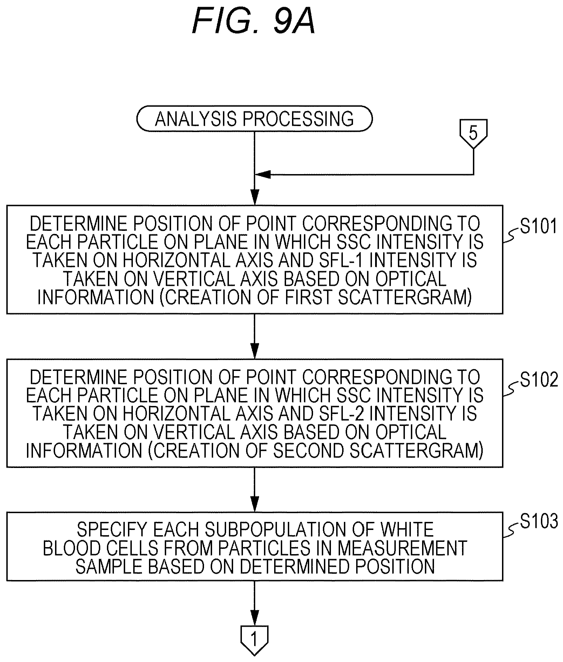

With respect to step S13 in Fig. 7, an example of analysis processing of Embodiment 1 will be described with reference to Figs. 9A to 13, but it is not limited to this example. In this analysis processing, classification of white blood cells and detection of the following abnormal cells are enabled: abnormal lymphocytes, DNA aneuploid cells, immature erythroblasts or megaloblasts, blasts, atypical lymphocytes, and immature granulocytes. In this example, the forward scattered light information and the side scattered light information are used as the scattered light information. More specifically, the forward scattered light intensity (hereinafter, also referred to as "FSC intensity") is used as the forward scattered light information. The second side scattered light intensity (hereinafter, also referred to as "SSC intensity") is used as the side scattered light information. Instead of the SSC intensity, the first side scattered light intensity may be used. The first side fluorescence intensity (hereinafter, also referred to as "SFL-1 intensity") is used as the first fluorescence information, and the second side fluorescence intensity (hereinafter, also referred to as "SFL-2 intensity") is used as the second fluorescence information. Hereinafter, a scattergram in which the SSC intensity is taken on a horizontal axis and the SFL-1 intensity is taken on a vertical axis is also referred to as a "first scattergram". Hereinafter, a scattergram in which the SSC intensity is taken on a horizontal axis and the SFL-2 intensity is taken on a vertical axis is also referred to as a "second scattergram". If necessary, the vertical axis of the first and second scattergrams may be a logarithmic scale of each fluorescence intensity. By setting the vertical axis to the logarithmic scale, a distance between a population of debris and a population of nucleated cell increases on the scattergram. Thus, by gating the population of nucleated cells on the scattergram, measurement data of debris can be excluded from measurement data of all particles in the measurement sample.

### (Classification of white blood cells based on first fluorescence information, second fluorescence information, and side scattered light information)

Referring to Fig. 9A, in step S101, the analysis unit 300 determines a position of a point corresponding to each particle on a plane in which the SSC intensity is taken on the horizontal axis and the SFL-1 intensity is taken on the vertical axis based on the acquired optical information. The analysis unit 300 may create the first scattergram based on the determined position of each point. In step S102, the analysis unit 300 determines a position of a point corresponding to each particle on a plane in which the SSC intensity is taken on the horizontal axis and the SFL-2 intensity is taken on the vertical axis based on the acquired optical information. The analysis unit 300 may create the second scattergram based on the determined position of each point. In step S103, the analysis unit 300 specifies each subpopulation of white blood cells from the particles in the measurement sample based on the determined position of each point. The subpopulations of white blood cells are a lymphocyte population, a monocyte population, a neutrophil population, an eosinophil population, and a basophil population. A lymphocyte population specified based on the side scattered light information and the first fluorescence information is hereinafter also referred to as a "first lymphocyte population". A lymphocyte population specified based on the side scattered light information and the second fluorescence information is hereinafter also referred to as a "second lymphocyte population". In the flowchart, the first lymphocyte population is denoted as "first Lymp", and the second lymphocyte population is denoted as "second Lymp". The algorithm itself for specifying each subpopulation of white blood cells is known. For example, each subpopulation of white blood cells can be specified from the particles in the measurement sample by a program installed in the analysis unit 300. As a result, white blood cells in the measurement sample are classified into subpopulations.

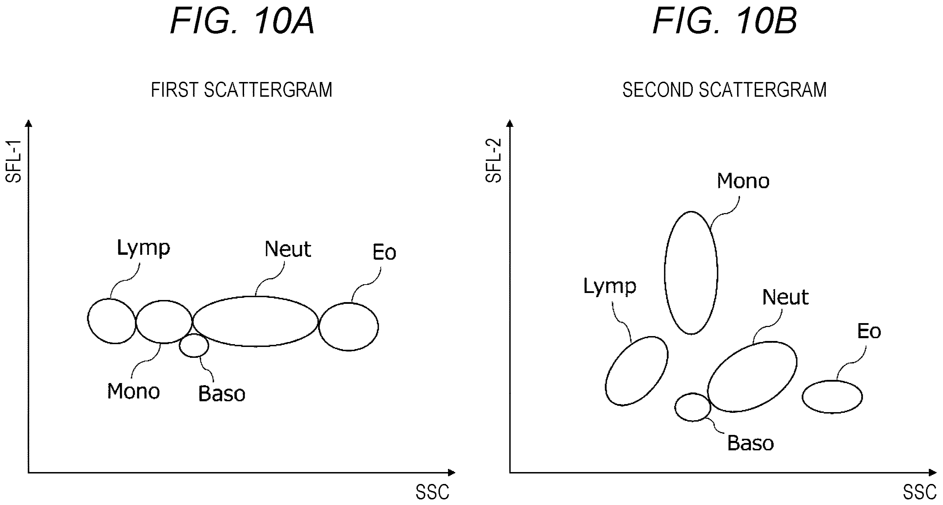

When the first and/or second scattergram has been created, each subpopulation of white blood cells may be specified based on the scattergram. The position at which each subpopulation of white blood cells appears on the scattergram is known per se. For example, as shown in A and B of Fig. 10, each subpopulation of white blood cells is distributed on each scattergram. In the figures, "Lymp" refers to the lymphocyte population, "Mono" refers to the monocyte population, "Neut" refers to the neutrophil population, "Eo" refers to the eosinophil population, and "Baso" refers to the basophil population. In A and B of Fig. 10, only a subpopulation of white blood cells is shown, and abnormal cells are not shown. In these figures, white blood cells are classified into five subpopulations: lymphocytes, monocytes, neutrophils, eosinophils, and basophils, but are not limited thereto. White blood cells may be classified into three subpopulations: lymphocytes, monocytes, and granulocytes. Alternatively, white blood cells may be classified into four subpopulations: lymphocytes, monocytes, neutrophils, and eosinophils. If necessary, the cells included in each subpopulation of white blood cells may be counted. As can be seen from A of Fig. 10, the SFL-1 intensity of each subpopulation of white blood cells is comparable. This suggests that the amount of DNA of each subpopulation of white blood cells is comparable.

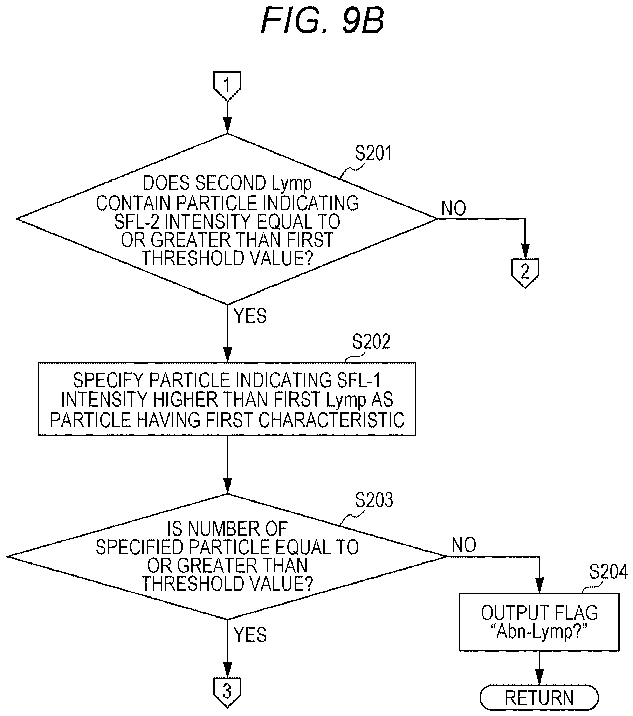

After classifying the white blood cells, the process proceeds to step S201 of Fig. 9B. The present inventors have found that among abnormal cells, in a measurement specimen containing abnormal lymphocytes, DNA aneuploid cells, blasts, immature erythroblasts, or megaloblasts, the number of particle indicating a high SFL-2 intensity tends to increase in the second lymphocyte population. In step S201, the second lymphocyte population having such a tendency is specified. Referring to Fig. 9B, in step S201, the analysis unit 300 determines whether the second lymphocyte population specified in step S103 includes a particle indicating second fluorescence information equal to or greater than a first threshold value corresponding to the second fluorescence information. The second fluorescence information is preferably the SFL-2 intensity. As the first threshold value corresponding to the second fluorescence information, for example, the minimum value of the SFL-2 intensity of all particles constituting the monocyte population (hereinafter, also referred to as "the minimum value of the SFL-2 intensity of the monocyte population") can be set. When the neutrophil population is specified, as the first threshold value corresponding to the second fluorescence information, for example, the maximum value of the SFL-2 intensity of all particles constituting the neutrophil population (hereinafter, also referred to as "the maximum value of the SFL-2 intensity of the neutrophil population") may be set.

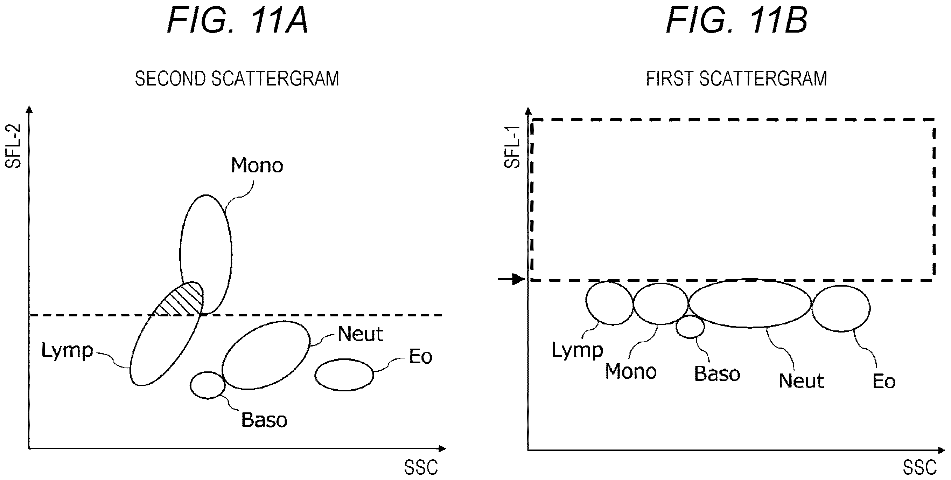

When the second scattergram has been created, the particle indicating second fluorescence information equal to or greater than the first threshold value corresponding to the second fluorescence information in the second lymphocyte population may be specified based on the scattergram. Referring to A of Fig. 11, for example, when the number of particle indicating a high SFL-2 intensity is increased in the second lymphocyte population, the particle also appears in a region where the monocyte population appears. In A of Fig. 11, a dashed line indicates the minimum value of the SFL-2 intensity of the monocyte population. Hatched lines indicate a particle indicating an SFL-2 intensity equal to or greater than the minimum value of the SFL-2 intensity of the monocyte population among the second lymphocyte population.

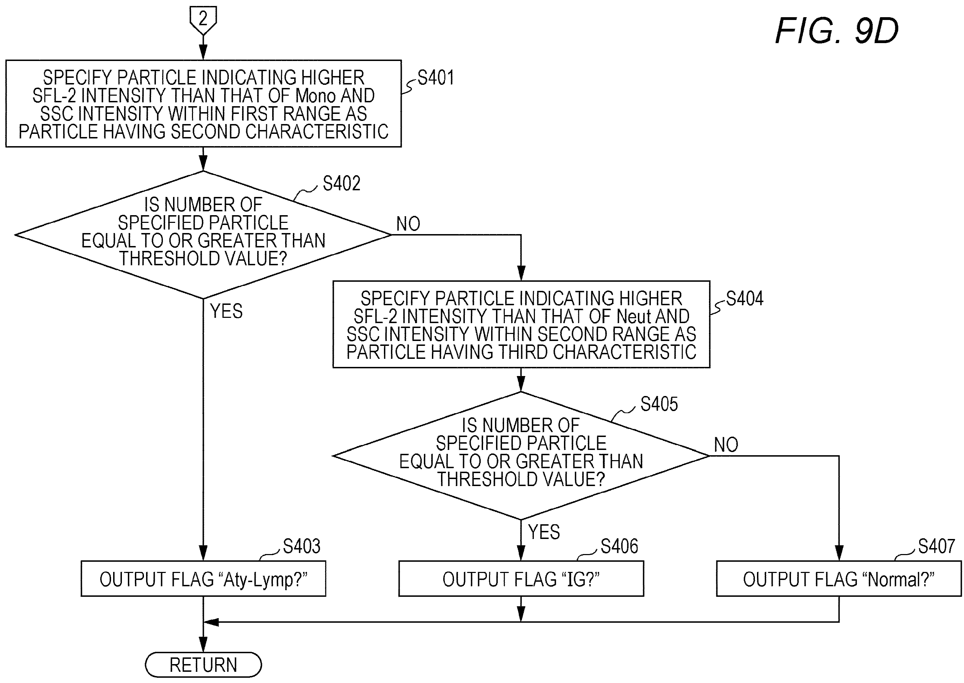

In step S201, when the analysis unit 300 determines that a particle indicating second fluorescence information equal to or greater than the first threshold value corresponding to the second fluorescence information is present in the second lymphocyte population, based on the second fluorescence information of each particle constituting the specified second lymphocyte population, the process proceeds to step S202. When the analysis unit 300 determines that a particle indicating second fluorescence information equal to or greater than the first threshold value corresponding to the second fluorescence information is not present in the second lymphocyte population, the process proceeds to step S401 of Fig. 9D.

### (Specification of particle having first characteristic)

Conventionally, it has been difficult to separately detect DNA aneuploid cells from blasts and abnormal lymphocytes. The present inventors have found that it is also difficult to distinguish DNA aneuploid cells from immature erythroblasts and megaloblasts. The present inventors have found that, as a characteristic common to a measurement sample containing DNA aneuploid cells, a measurement sample containing blasts, and a measurement sample containing immature erythroblasts or megaloblasts, the number of particle indicating a higher SFL-1 intensity than that of the first lymphocyte population increases. On the other hand, in the measurement specimen containing abnormal lymphocytes, such a particle did not tend to increase. The present inventors have found that DNA aneuploid cells, blasts, immature erythroblasts, and megaloblasts can be distinguished based on the information of the particle indicating a higher SFL-1 intensity than that of the first lymphocyte population. In the method for analyzing a specimen of the present embodiment, in order to classify DNA aneuploid cells, blasts, abnormal lymphocytes, and immature erythroblasts or megaloblasts, a particle indicating higher first fluorescence information than that of the first lymphocyte population is specified as the particle having a first characteristic. The first fluorescence information is preferably the SFL-1 intensity.

In step S202, the analysis unit 300 specifies a particle indicating higher first fluorescence information than that of the first lymphocyte population as the particle having a first characteristic, from the particles in the measurement sample, based on the first fluorescence information. The higher first fluorescence information than that of the first lymphocyte population may be, for example, a higher value than the maximum value of the SFL-1 intensity of all particles constituting the lymphocyte population (hereinafter, also referred to as "the maximum value of the SFL-1 intensity of the first lymphocyte population"). When the first scattergram has been created, the particle indicating higher first fluorescence information than that of the first lymphocyte population may be specified based on the scattergram. Referring to B of Fig. 11, for example, when the particle indicating a higher SFL-1 intensity than that of the first lymphocyte population is contained in the measurement sample, the particle appears in a region surrounded by a dashed line. In B of Fig. 11, an arrow indicates the maximum value of the SFL-1 intensity of the first lymphocyte population.

### (Detection of abnormal lymphocyte)

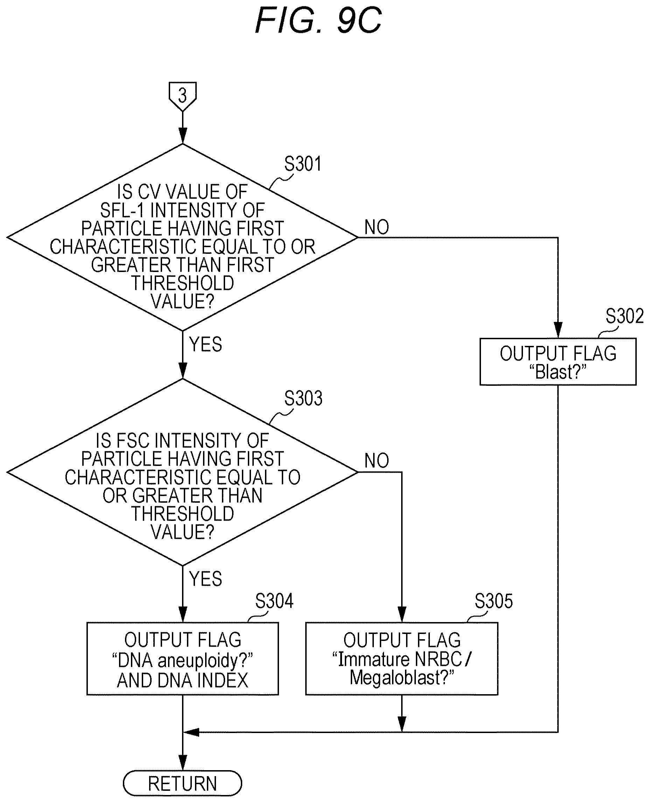

The analysis unit 300 can output information indicating the presence or absence of an abnormal lymphocyte based on information of the particle having a first characteristic. At this time, the information of the particle having a first characteristic is, for example, the number of the particle. In step S203, the analysis unit 300 counts the number of particles having a first characteristic, and the analysis unit 300 determines whether the number is equal to or greater than a corresponding threshold value. When the number of particles having a first characteristic is equal to or greater than the threshold value, the analysis unit 300 determines that no abnormal lymphocyte is contained in the measurement sample. In this case, the process proceeds to step S301 in Fig. 9C. In step S203, when the number of particles having a first characteristic is less than the threshold value, the process proceeds to step S204. The case where the number of particles having a first characteristic is less than the threshold value includes a case where the particle having a first characteristic was not present. In step S204, the analysis unit 300 specifies a particle indicating second fluorescence information equal to or greater than the first threshold value corresponding to the second fluorescence information as an abnormal lymphocyte. Then, the analysis unit 300 outputs information indicating that an abnormal lymphocyte is present. For example, a flag "Abn-Lymp?" may be output to the display part 305 as information indicating the presence of an abnormal lymphocyte. The analysis unit 300 ends the analysis processing, and the process proceeds to step S14 in Fig. 7. Not displaying the flag on the display part 305 can be information indicating the absence of an abnormal lymphocyte.

The threshold value corresponding to the number of particles having a first characteristic can be appropriately determined. For example, it may be empirically set by accumulating data of optical information obtained by measuring peripheral blood of a healthy person, peripheral blood containing abnormal lymphocytes, and peripheral blood containing DNA aneuploid cells, blasts, immature erythroblasts, or megaloblasts.

### (Detection of blast)

The present inventors have found that in a measurement sample containing DNA aneuploid cells and a measurement sample containing immature erythroblasts or megaloblasts, a particle having a first characteristic tends to appear in a region above each subpopulation of white blood cells in the vertical axis (SFL-1 intensity) direction in the first scattergram. On the other hand, in the measurement sample containing blasts, such a tendency was not observed in the particle having a first characteristic. The analysis unit 300 can output information indicating the presence or absence of a blast based on the information of the particle having a first characteristic. At this time, the information of the particle having a first characteristic is, for example, information on dispersion of first fluorescence information of the particle. The first fluorescence information is preferably the SFL-1 intensity. The information on dispersion of first fluorescence information of the particle having a first characteristic may be an index value representing variation in the first fluorescence information with respect to normal white blood cells (or at least one of subpopulations thereof) of the particle having a first characteristic. As described above, the SFL-1 intensity of each subpopulation of normal white blood cells is comparable. When the particles having a first characteristic and normal white blood cells or at least one of subpopulations thereof (preferably the first lymphocyte population) are regarded as one particle population, variation in the first fluorescence information of the particle population reflects distribution of particles having a first characteristic in the first scattergram. When the variation in the first fluorescence information is large, the particles having a first characteristic are displayed as a discrete cluster from normal white blood cells in the vertical axis (SFL-1 intensity) direction of the first scattergram. On the other hand, when the variation in the first fluorescence information is small, the particles having a first characteristic are displayed as a cluster continuous with normal white blood cells (particularly, the first lymphocyte population) in the first scattergram. That is, in the first scattergram, an appearance region of the particle having a first characteristic and an appearance region of the normal white blood cells (particularly, the first lymphocyte population) substantially overlap with each other.

The information on dispersion of first fluorescence information is preferably a value of coefficient of variation (hereinafter, also referred to as "CV") of the first fluorescence information, and more preferably a CV value of the SFL-1 intensity. The CV value of the SFL-1 intensity of the particle having a first characteristic can be calculated by dividing the standard deviation of the SFL-1 intensity of the population containing particles having a first characteristic by the average of the SFL-1 intensity of the population. Examples of the population containing particles having a first characteristic include a particle population combining particles having a first characteristic and normal white blood cells, a particle population combining particles having a first characteristic and at least one selected from a subpopulation of normal white blood cells, or a particle population combining particles having a first characteristic and a first lymphocyte population. Referring to Fig. 9C, in step S301, the analysis unit 300 determines whether the CV value of the particle having a first characteristic is equal to or greater than a first threshold value corresponding to CV. When the CV value is equal to or greater than the first threshold value, the analysis unit 300 determines that no blast is contained in the measurement sample. In this case, the process proceeds to step S303. When the CV value of the particle having a first characteristic is less than the first threshold value corresponding to CV, in step S302, the analysis unit 300 specifies the particle having a first characteristic as a blast. Then, the analysis unit 300 outputs information indicating that a blast is present. For example, a flag "Blast?" may be output to the display part 305 as information indicating the presence of a blast. Then, the analysis unit 300 ends the analysis processing, and the process proceeds to step S14 in Fig. 7. Not displaying the flag on the display part 305 can be information indicating the absence of a blast.

For example, a plurality of specimens containing at least one of DNA aneuploid cells, immature erythroblasts, or megaloblasts and a plurality of specimens containing blasts are measured, and the first threshold value corresponding to CV is set in advance based on the measurement result. Preferably, the first threshold value corresponding to CV is set between 0.05 to 0.25. For example, the first threshold value corresponding to CV is 0.05, 0.1, 0.15, 0.2, or 0.25. When the specimens containing at least one of DNA aneuploid cells, immature erythroblasts, or megaloblasts are measured, as shown in a first scattergram in A of Fig. 12, a population is formed at a position away from each subpopulation of white blood cells (indicated by an arrow in A of Fig. 12). Therefore, when the specimens containing at least one of DNA aneuploid cells, immature erythroblasts, or megaloblasts are measured, in A of Fig. 12, CV of the SFL-1 intensity of entire particles or CV of the SFL-1 intensity of particles in the lymphocyte appearance region is equal to or greater than the first threshold value. That is, when the CV of the SFL-1 intensity of entire particles or the CV of the SFL-1 intensity of particles in the lymphocyte appearance region is equal to or greater than the first threshold value, it is determined that the specimen contains at least one of DNA aneuploid cells, immature erythroblasts, or megaloblastic cells. Alternatively, when the CV of the SFL-1 intensity is less than the first threshold value, it can be determined that the specimen contains blasts (see step S301 in Fig. 9C). The entire particles refer to a particle population combining particles having a first characteristic and normal white blood cells. The particles in the lymphocyte appearance region refer to a particle population combining particles having a first characteristic and a first lymphocyte population.

### (Detection of DNA aneuploid cells and immature erythroblasts/megaloblasts)

The present inventors have found that the FSC intensity of the particle having a first characteristic in the measurement specimen containing DNA aneuploid cells is higher than that in the measurement specimen containing immature erythroblasts or megaloblasts. This is considered to be because when the FSC intensity reflects particle size, an immature erythroblast and a megaloblast are particles smaller than a DNA aneuploid cell. The analysis unit 300 can output information indicating the presence or absence of a DNA aneuploid cell based on the information of the particle having a first characteristic. The analysis unit 300 can output information indicating the presence or absence of an immature erythroblast or a megaloblast based on the information of the particle having a first characteristic. At this time, the information of the particle having a first characteristic is, for example, forward scattered light information of the particle. The forward scattered light information is preferably the FSC intensity.

In step S303, the analysis unit 300 determines whether the FSC intensity of the particle having a first characteristic is equal to or greater than a threshold value corresponding to the FSC intensity. When the FSC intensity is equal to or greater than the threshold value, in step S304, the analysis unit 300 specifies the particle having a first characteristic as a DNA aneuploid cell. Then, the analysis unit 300 outputs information indicating that a DNA aneuploid cell is present. For example, a flag "DNA aneuploidy?" may be output to the display part 305 as information indicating the presence of a DNA aneuploid cell. The analysis unit 300 may further calculate DNA index and output a value thereof. Not displaying the flag on the display part 305 can be information indicating the absence of a DNA aneuploid cell.

As shown in Reference Example 2 described later, the DNA index can be calculated based on first fluorescence information of a population specified as monocytes and lymphocytes. The DNA index is specifically calculated as follows. First, in the second scattergram, a region where monocytes and lymphocytes have appeared is gated. In this region, in addition to normal monocytes and lymphocytes, DNA aneuploid cells are included. Then, a histogram of the SFL-1 intensity is created for the particles in this region. In the histogram, a peak with a low SFL-1 intensity derived from normal monocytes and lymphocytes and a peak with a high SFL-1 intensity derived from DNA aneuploid cells. A mode of the SFL-1 intensity is acquired for the particles included in each of the two peaks. Then, the DNA index is calculated by the following equation. (DNA index) = (Mode of population with high SFL-1 intensity)/(Mode of population with low SFL-1 intensity)

In step S303, when the FSC intensity of the particle having a first characteristic is less than a corresponding threshold value, in step S305, the analysis unit 300 specifies the particle having a first characteristic as an immature erythroblast or a megaloblast. Then, the analysis unit 300 outputs information indicating that an immature erythroblast or a megaloblast is present. For example, a flag "Immature NRBC/Megaloblast?" may be output to the display part 305 as information indicating the presence of an immature erythroblast or a megaloblast. Not displaying the flag on the display part 305 can be information indicating the absence of an immature erythroblast and a megaloblast.

As the threshold value corresponding to the FSC intensity, for example, the maximum value of the FSC intensity of all particles constituting the first lymphocyte population (hereinafter, also referred to as "the maximum value of the FSC intensity of the first lymphocyte population") can be set. When the FSC intensity is equal to or greater than the threshold value, particles indicating a higher FSC intensity than that of the first lymphocyte population form a population in a region above a dashed line as shown in a scattergram in B of Fig. 12 (a horizontal axis represents the SSC intensity, and a vertical axis represents the FSC intensity). In B of Fig. 12, the dashed line indicates the maximum value of the FSC intensity of the first lymphocyte population. When a DNA aneuploid cell is present in the measurement sample, the DNA aneuploid cell appears in the region above the dashed line in the scattergram in B of Fig. 12. When an immature erythroblast or a megaloblast is present in the measurement sample, the cell appears in a region below the dashed line in the scattergram.

### (Specification of particle having second characteristic)

In step S201 of Fig. 9B, when the second lymphocyte population is determined not to include a particle indicating second fluorescence information equal to or greater than the first threshold value corresponding to the second fluorescence information, in step S401 of Fig. 9D, the analysis unit 300 specifies a particle having a second characteristic. The present inventors have found a characteristic that in a measurement sample containing atypical lymphocytes, a particle appears at a position above the monocyte population in the vertical axis (SFL-2 intensity) direction in the second scattergram. On the other hand, in the measurement sample containing immature granulocytes, such a characteristic was not observed. In the method for analyzing a specimen of the present embodiment, in order to classify atypical lymphocytes and immature granulocytes, a particle indicating higher second fluorescence information than that of the monocyte population and side scattered light information within a first range is specified as the particle having a second characteristic. The monocyte population has been specified in step S102. The second fluorescence information is preferably the SFL-2 intensity. The side scattered light information is preferably the SSC intensity.

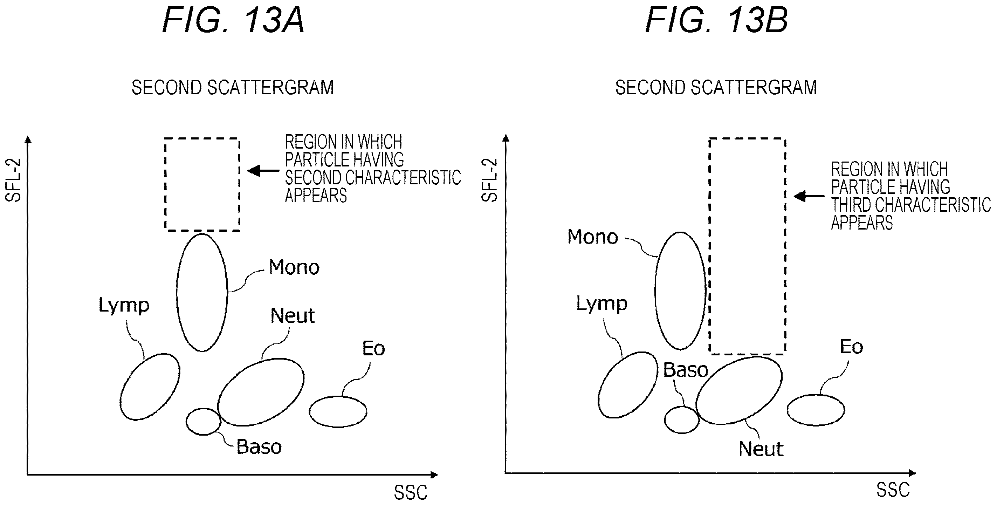

In step S401, the analysis unit 300 specifies a particle indicating a higher SFL-2 intensity than that of the monocyte population and an SSC intensity within a first range as the particle having a second characteristic. The higher SFL-2 intensity than that of the monocyte population may be, for example, a higher value than the maximum value of the SFL-2 intensity of all particles constituting the monocyte population (hereinafter, also referred to as "the maximum value of the SFL-2 intensity of the monocyte population"). With respect to the SSC intensity, the first range can be, for example, equal to or greater than a statistical representative value of the SSC intensity of the second lymphocyte population and less than or equal to a statistical representative value of the SSC intensity of the neutrophil population. The statistical representative value of the SSC intensity of one subpopulation of white blood cells is a value acquired from SSC intensity of all particles constituting the subpopulation, and examples thereof include median, average, mode, and the like. The statistical representative value of the SSC intensity of one subpopulation of white blood cells is preferably median. Alternatively, the first range may be the same SSC intensity as that of the monocyte population. That is, the first range can be equal to or greater than the minimum value of the SSC intensity of all particles constituting the monocyte population (hereinafter, also referred to as "the minimum value of the SSC intensity of the monocyte population") and less than or equal to the maximum value of the SSC intensity (hereinafter, also referred to as "the maximum value of the SSC intensity of the monocyte population"). When the second scattergram has been created, the particle having a second characteristic may be specified based on the scattergram. Referring to A of Fig. 13, when the particle having a second characteristic is contained in the measurement sample, the particle appears in a region surrounded by a dashed line. In the figure, the region surrounded by a dashed line represents a region where the SFL-2 intensity is equal to or greater than the maximum value of the SFL-2 intensity of the monocyte population and the SSC intensity is within the first range.

### (Detection of atypical lymphocyte)

The analysis unit 300 can output information indicating the presence or absence of an atypical lymphocyte based on information of the particle having a second characteristic. At this time, the information of the particle having a second characteristic is, for example, the number of the particle. In step S402, the analysis unit 300 counts the number of particles having a second characteristic, and the analysis unit 300 determines whether the number is equal to or greater than a corresponding threshold value. When the number of particles having a second characteristic is equal to or greater than the threshold value, in step S403, the analysis unit 300 specifies the particle having a second characteristic as an atypical lymphocyte. Then, the analysis unit 300 outputs information indicating that an atypical lymphocyte is present. For example, a flag "Aty-Lymp?" may be output to the display part 305 as information indicating the presence of an atypical lymphocyte. The analysis unit 300 ends the analysis processing, and the process proceeds to step S14 in Fig. 7. In step S402, when the number of particles having a second characteristic is less than the corresponding threshold value, the analysis unit 300 determines that no atypical lymphocyte is contained in the measurement sample. In this case, the process proceeds to step S404. The case where the number of particles having a second characteristic is less than the corresponding threshold value includes a case where the particle having a second characteristic was not present. Not displaying the flag on the display part 305 can be information indicating the absence of an atypical lymphocyte.

### (Specification of particle having third characteristic)

The present inventors have found that in a measurement sample containing immature granulocytes, a particle appears at a position above the neutrophil population in the vertical axis (SFL-2 intensity) direction in the second scattergram. In the method for analyzing a specimen of the present embodiment, in order to specify an immature granulocyte, a particle indicating higher second fluorescence information than that of the neutrophil population and side scattered light information within a second range is specified as the particle having a third characteristic. The neutrophil population has been specified in step S102. The second fluorescence information is preferably the SFL-2 intensity. The side scattered light information is preferably the SSC intensity.

In step S404, the analysis unit 300 specifies a particle indicating a higher SFL-2 intensity than that of the monocyte population and an SSC intensity within a first range as the particle having a third characteristic. The higher SFL-2 intensity than that of the neutrophil population can be, for example, a higher value than the maximum value of the SFL-2 intensity of the neutrophil population. With respect to the SSC intensity, the second range can be, for example, equal to or greater than the maximum value of the SSC intensity of the monocyte population and less than or equal to the maximum value of the SSC intensity of all particles constituting the neutrophil population (hereinafter, also referred to as "the maximum value of the SSC intensity of the neutrophil population"). Alternatively, the second range may be the same SSC intensity as that of the neutrophil population. That is, the second range can be equal to or greater than the minimum value of the SSC intensity of all particles constituting the neutrophil population (hereinafter, also referred to as "the minimum value of the SSC intensity of the neutrophil population") and less than or equal to the maximum value of the SSC intensity of the neutrophil population. When the second scattergram has been created, the particle having a third characteristic may be specified based on the scattergram. Referring to B of Fig. 13, when the particle having a third characteristic is contained in the measurement sample, the particle appears in a region surrounded by a dashed line. In the figure, the region surrounded by a dashed line represents a region where the SFL-2 intensity is equal to or greater than the maximum value of the SFL-2 intensity of the neutrophil population and the SSC intensity is within the second range.

### (Detection of immature granulocyte)

The analysis unit 300 can output information indicating the presence or absence of an immature granulocyte based on information of the particle having a third characteristic. At this time, the information of the particle having a third characteristic is, for example, the number of the particle. In step S405, the analysis unit 300 counts the number of particles having a third characteristic, and the analysis unit 300 determines whether the number is equal to or greater than a corresponding threshold value. When the number of particles having a third characteristic is equal to or greater than the threshold value, in step S406, the analysis unit 300 specifies the particle having a third characteristic as an immature granulocyte. Then, the analysis unit 300 outputs information indicating that an immature granulocyte is present. For example, a flag "IG?" may be output to the display part 305 as information indicating the presence of an immature granulocyte. The analysis unit 300 ends the analysis processing, and the process proceeds to step S14 in Fig. 7. In step S405, when the number of particles having a third characteristic is less than the corresponding threshold value, the analysis unit 300 determines that no immature granulocyte is contained in the measurement sample. In this case, the process proceeds to step S407. Not displaying the flag on the display part 305 can be information indicating the absence of an immature granulocyte.

In step S407, the analysis unit 300 outputs information indicating that an abnormal cell is not present in the measurement sample. For example, a flag "Normal" may be output to the display part 305. Then, the analysis unit 300 ends the analysis processing, and the process proceeds to step S14 in Fig. 7.

The threshold value corresponding to each of the number of particles having a second characteristic and the number of particles having a third characteristic can be appropriately determined. For example, it may be empirically set by accumulating data of optical information obtained by measuring peripheral blood of a healthy person, peripheral blood containing atypical lymphocytes, and peripheral blood containing immature granulocytes.

As described above, the procedures of the analysis processing of Embodiment 1 have been described based on the flowchart. From this flowchart, a relationship between the information of the particles having first, second, and third characteristics and the abnormal cell specified based on the information is extracted and described below.

When the second lymphocyte population includes a particle indicating second fluorescence information equal to or greater than the first threshold value corresponding to the second fluorescence information, a particle having a first characteristic is specified based on the first fluorescence information. When the information of the particle having a first characteristic satisfies the following conditions 1) to 3), the particle having a first characteristic is specified as a first abnormal cell. Then, as information indicating the presence or absence of a first abnormal cell, information indicating that a DNA aneuploid cell is present is output.
1) The number of particles having a first characteristic is equal to or greater than the corresponding threshold value;
2) the information on dispersion of first fluorescence information of the particle having a first characteristic is equal to or greater than a corresponding threshold value; and
3) the forward scattered light information of the particle having a first characteristic is equal to or greater than a corresponding threshold value.

Alternatively, when the information of the particle having a first characteristic satisfies the following conditions 1) and 4), the particle having a first characteristic is specified as a second abnormal cell. Then, as information indicating the presence or absence of a second abnormal cell, information indicating that a blast is present is output.
1) the number of particles having a first characteristic is equal to or greater than the corresponding threshold value; and
4) the information on dispersion of first fluorescence information of the particle having a first characteristic is less than a corresponding threshold value.

Alternatively, when the number of particles having a first characteristic is less than the corresponding threshold value, the particle indicating second fluorescence information equal to or greater than the first threshold value corresponding to the second fluorescence information is specified as a third abnormal cell. Then, as information indicating the presence or absence of a third abnormal cell, information indicating that an abnormal lymphocyte is present is output.

Alternatively, when the information of the particle having a first characteristic satisfies the following conditions 1), 2), and 5), the particle having a first characteristic is specified as a fourth abnormal cell. Then, as information indicating the presence or absence of a fourth abnormal cell, information indicating that an immature erythroblast or a megaloblast is present is output.
1) The number of particles having a first characteristic is equal to or greater than the corresponding threshold value;
2) the information on dispersion of first fluorescence information of the particle having a first characteristic is equal to or greater than the corresponding threshold value; and
5) the forward scattered light information of the particle having a first characteristic is less than a corresponding threshold value.

When the second lymphocyte population does not include a particle indicating second fluorescence information equal to or greater than the first threshold value corresponding to the second fluorescence information, a monocyte population and a particle having a second characteristic are specified based on the second fluorescence information and the side scattered light information. When the number of particles having a second characteristic is equal to or greater than a corresponding threshold value, the particle having a second characteristic is specified as a fifth abnormal cell. Then, as information indicating the presence or absence of a fifth abnormal cell, information indicating that an atypical lymphocyte is present is output.

A neutrophil population and a particle having a third characteristic are further specified based on the second fluorescence information and the side scattered light information. When the number of particles having a second characteristic is less than the corresponding threshold value and the number of particles having a third characteristic is equal to or greater than a corresponding threshold value, the particle having a third characteristic is specified as a sixth abnormal cell. Then, as information indicating the presence or absence of a sixth abnormal cell, information indicating that an immature granulocyte is present is output.

### (Modification of analysis processing of Embodiment 1)

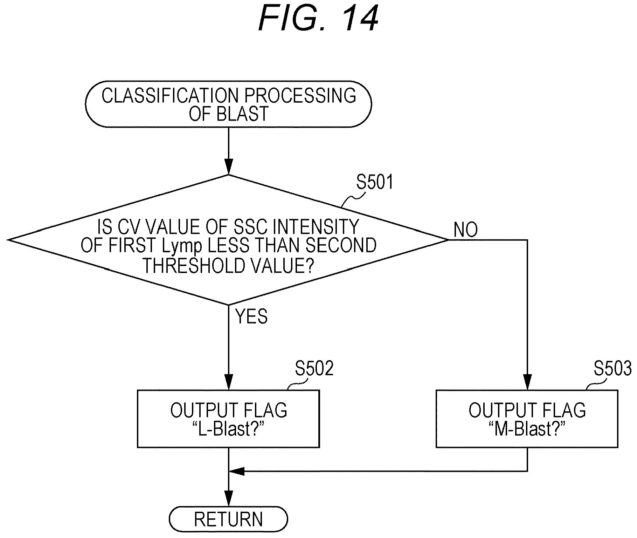

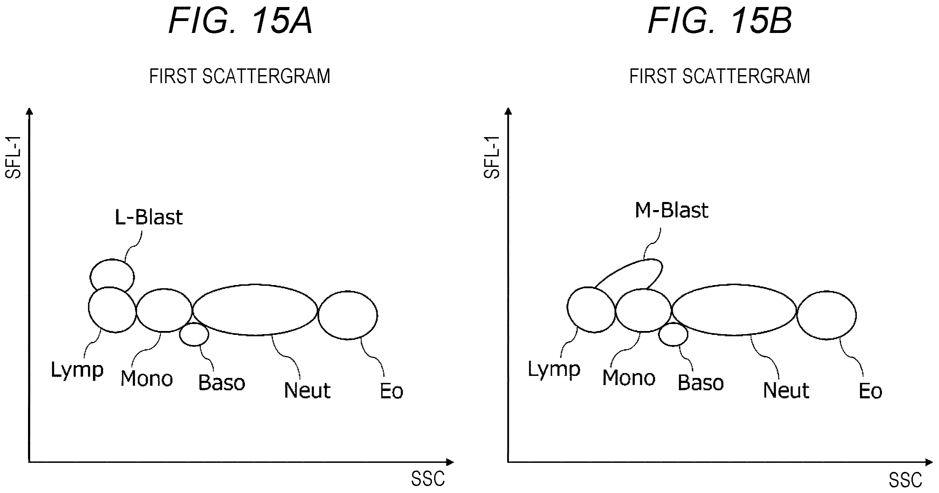

When the particle having a first characteristic is specified as a blast in step S302 in Fig. 9C, an example of analysis processing for further classifying blasts will be described with reference to Figs. 14 and 15. However, the method for analyzing a specimen of the present embodiment is not limited to this example. This analysis processing enables classification of blasts into lymphoblasts or myeloblasts.

The present inventors have found that it is possible to discriminate whether blasts are myeloblasts or lymphoblasts. Specifically, when myeloblasts are contained in the measurement sample, the first lymphocyte population tends to be distributed to the right in the horizontal axis direction in the first scattergram. On the other hand, when lymphoblasts are contained in the measurement sample, such a tendency was not observed in the first lymphocyte population. In the method for analyzing a specimen of the present embodiment, blasts are specified as lymphoblasts or myeloblasts based on the information on dispersion of first fluorescence information of the particle having a first characteristic (see step S301) and the information on dispersion of side scattered light information of the first lymphocyte population. The first fluorescence information is preferably the SFL-1 intensity. The information on dispersion of first fluorescence information is preferably a CV value of the first fluorescence information, and more preferably a CV value of the SFL-1 intensity. The side scattered light information is preferably the SSC intensity. The information on dispersion of side scattered light information may be an index value representing variation in the side scattered light information of the particle. The information on dispersion of side scattered light information is preferably a CV value of the side scattered light information, and more preferably a CV value of the SSC intensity.

### (Detection of lymphoblast)

Referring to Fig. 14, in step S501, the analysis unit 300 determines, for example, whether the CV value of the SSC intensity of the first lymphocyte population is less than a second threshold value corresponding to CV. The CV value of the SSC intensity can be calculated by dividing the standard deviation of the SSC intensity of the first lymphocyte population by the average of the SSC intensity of the first lymphocyte population. The second threshold value corresponding to CV is set in advance, for example, based on a measurement result obtained by measuring a plurality of specimens containing myeloblasts and a plurality of specimens containing lymphoblasts. Preferably, the second threshold value corresponding to CV is set between 0.3 to 0.5. For example, the second threshold value is 0.3, 0.35, 0.4, 0.45, or 0.5. When the CV value of the SSC intensity is less than the second threshold value corresponding to CV, as shown in a first scattergram in A of Fig. 15, the first lymphocyte population includes lymphoblasts, but a distribution spreading in the horizontal axis (SSC intensity) direction is less observed. In step S501, when the CV value of the SSC intensity of the first lymphocyte population is less than the second threshold value corresponding to CV, in step S502, the analysis unit 300 specifies the particle having a first characteristic as a lymphoblast. Then, the analysis unit 300 outputs information indicating that a lymphoblast is present. For example, a flag "L-Blast?" may be output to the display part 305 as information indicating the presence of a lymphoblast. Then, the analysis unit 300 ends the analysis processing, and the process proceeds to step S14 in Fig. 7. Not displaying the flag on the display part 305 can be information indicating the absence of a lymphoblast.

### (Detection of myeloblast)

When the CV value of the SSC intensity is equal to or greater than the second threshold value corresponding to CV, as shown in a first scattergram in B of Fig. 15, the first lymphocyte population includes myeloblasts, and a distribution spreading in the horizontal axis (SSC intensity) direction is observed. In step S501, when the CV value of the SSC intensity of the first lymphocyte population is equal to or greater than the second threshold value corresponding to CV, in step S503, the analysis unit 300 specifies the particle having a first characteristic as a myeloblast. Then, the analysis unit 300 outputs information indicating that a myeloblast is present. For example, a flag "M-Blast?" may be output to the display part 305 as information indicating the presence of a myeloblast. Then, the analysis unit 300 ends the analysis processing, and the process proceeds to step S14 in Fig. 7. Not displaying the flag on the display part 305 can be information indicating the absence of a myeloblast.

As described above, the procedures of the analysis processing of the modification of Embodiment 1 have been described based on the flowchart. From this flowchart, a relationship between the information of the particle having a first characteristic and the information of the first lymphocyte population, and the second abnormal cell specified based on the information is extracted and described below.

Information indicating that a lymphoblast or a myeloblast is present is output as the information indicating the presence or absence of a second abnormal cell based on the information of the particle having a first characteristic and the information of the first lymphocyte population. More specifically, when the information of the particle having a first characteristic satisfies the conditions of 1) and 4) above and the information on dispersion of side scattered light information of the first lymphocyte population is less than a corresponding threshold value, the information indicating that a lymphoblast is present is output as the information indicating the presence or absence of a second abnormal cell. Alternatively, when the information of the particle having a first characteristic satisfies the conditions of 1) and 4) above, and the information on dispersion of side scattered light information of the first lymphocyte population is equal to or greater than the corresponding threshold value, the information indicating that a myeloblast is present is output as the information indicating the presence or absence of a second abnormal cell.

### (Measurement processing and analysis processing of Embodiment 2)

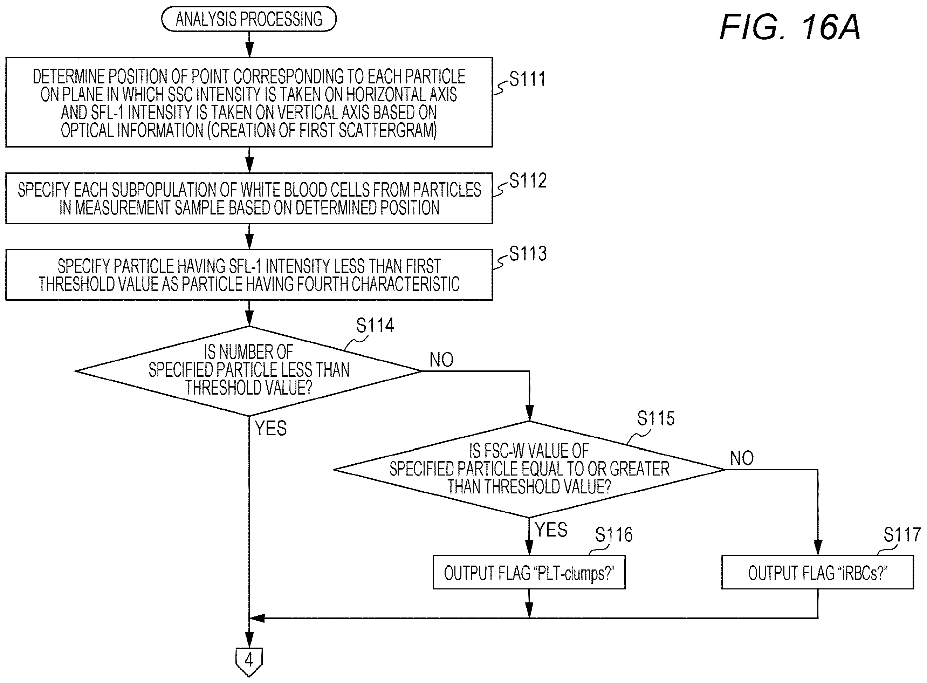

With respect to step S12 in Fig. 7, measurement processing of Embodiment 2 is similar to that described for Embodiment 1. With respect to step S13 in Fig. 7, an example of analysis processing of Embodiment 2 will be described with reference to Figs. 16A to 18, but it is not limited to this example. As described above, in the analysis processing of Embodiment 2, first, particles that can interfere with the analysis of Embodiment 1 are specified. Then, as in Embodiment 1, classification of white blood cells into subpopulations and detection of abnormal cells are performed. In Embodiment 2, the particles specified before the analysis processing of Embodiment 1 are platelet clumps, malaria-infected red blood cells, and nucleated red blood cells. Hereinafter, steps up to the step of specifying these particles will be described. The analysis processing after specifying these particles is similar to that described for Embodiment 1. In the example of analysis processing of Embodiment 2, the forward scattered light information and the side scattered light information are used as the scattered light information. More specifically, information on width of the forward scattered light signal is used as the forward scattered light information. The SSC intensity is used as the side scattered light information. Instead of the SSC intensity, the first side scattered light intensity may be used. The SFL-1 intensity is used as the first fluorescence information, and the SFL-2 intensity is used as the second fluorescence information. If necessary, the first and second scattergrams may be created. The vertical axis of these scattergrams may be a logarithmic scale of each fluorescence intensity.

### (Classification of white blood cells based on first fluorescence information and side scattered light information)

Referring to Fig. 16A, in step S111, the analysis unit 300 determines a position of a point corresponding to each particle on a plane in which the SSC intensity is taken on the horizontal axis and the SFL-1 intensity is taken on the vertical axis based on the acquired optical information. The analysis unit 300 may create the first scattergram based on the determined position of each point. In step S112, the analysis unit 300 specifies each subpopulation of white blood cells from the particles in the measurement sample based on the determined position of each point.

### (Specification of particle having fourth characteristic)

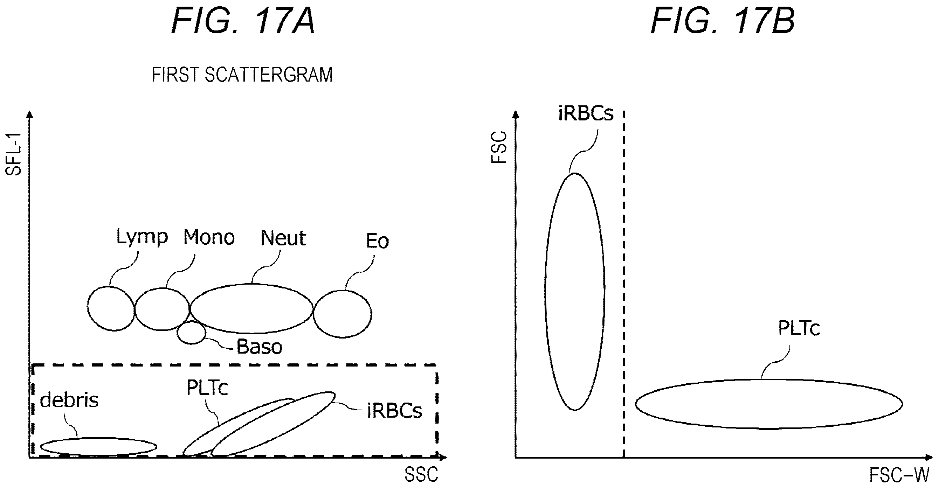

In the second scattergram, a region where the platelet clumps and the malaria-infected red blood cells appear overlaps with a region where the basophil population, the neutrophil population, and the eosinophil population appear. In the second scattergram, the platelet clumps and the malaria-infected red blood cell appear in almost the same region. Therefore, it has been difficult to specify platelet clumps and a malaria-infected red blood cell by the second scattergram. On the other hand, in the first scattergram, as shown in A of Fig. 17, white blood cells, platelet clumps and malaria-infected red blood cells can be distinguished based on the first fluorescence information. When nucleated red blood cells are also included in the specimen, nucleated red blood cells, platelet clumps and malaria-infected red blood cells can be distinguished. This is because the first fluorescence information shows a high value since white blood cells and nucleated red blood cells have nuclei, whereas the first fluorescence information shows a low value since platelet clumps and malaria-infected red blood cells do not have nuclei. In the method for analyzing a specimen of the present embodiment, in order to specify platelet clumps and a malaria-infected red blood cell, a particle in which the first fluorescence information is less than a first threshold value corresponding to the first fluorescence information is specified as a particle having a fourth characteristic. In a preferred embodiment, a particle in which the first fluorescence information is less than a first threshold value corresponding to the first fluorescence information and a side scattered light information is equal to or greater than the threshold value corresponding to the side scattered light information is specified as the particle having a fourth characteristic. The first fluorescence information is preferably the SFL-1 intensity. The side scattered light information is preferably the SSC intensity. By combining the first fluorescence information and the side scattered light information, debris, platelet clumps and malaria-infected red blood cells can be distinguished.

Referring to Fig. 16A, in step S113, the analysis unit 300 specifies a particle in which the SFL-1 intensity is less than the first threshold value corresponding to the first fluorescence information as the particle having a fourth characteristic. The SFL-1 intensity reflects the amount of DNA contained in the particle. As the first threshold value corresponding to the first fluorescence information, for example, the minimum value of the SFL-1 intensity of all particles constituting the lymphocyte population (hereinafter, also referred to as "the minimum value of the SFL-1 intensity of the first lymphocyte population") can be set. Alternatively, the minimum value of the SFL-1 intensity of all particles constituting the monocyte population (hereinafter, also referred to as "the minimum value of the SFL-1 intensity of the monocyte population") can be set as the first threshold value corresponding to the first fluorescence information. When the neutrophil population, the eosinophil population, or the basophil population has been specified, for example, the minimum value of the SFL-1 intensity of all particles constituting the neutrophil population, the eosinophil population, or the basophil population (hereinafter, also referred to as "the minimum value of the SFL-1 intensity of the neutrophil population, the eosinophil population, or the basophil population") may be set as the first threshold value corresponding to the first fluorescence information. That is, when a particle indicating an SFL-1 intensity less than such first threshold value is extracted, the particles hardly contain white blood cells. The particle in which the SFL-1 intensity is less than the first threshold value corresponding to the first fluorescence information is a particle having a smaller amount of DNA than that of white blood cells. Examples of such a particle include debris such as red blood cell ghosts, platelet clumps (PLT-clumps), and malaria-infected red blood cells (iRBCs). When the first scattergram has been created, a particle contained in a region where debris, platelet clumps, and malaria-infected red blood cells appear may be specified based on the scattergram. For example, as shown in A of Fig. 17, debris, platelet clumps (indicated as "PLTc" in the figure), and malaria-infected red blood cells (iRBCs) are distributed in a region where the SFL-1 intensity is lower than that of each subpopulation of white blood cells (a region surrounded by a dashed line). In A of Fig. 17, all of debris, platelet clumps, and malaria-infected red blood cells appear, but are not limited thereto. At least one of debris, platelet clumps, or malaria-infected red blood cells may appear in the scattergram.

The first threshold value corresponding to the first fluorescence information may be a numerical range corresponding to the first fluorescence information. Since debris is hardly stained with a fluorescent dye, for example, the lower limit of the numerical range may be set to the higher SFL-1 intensity than that of a population of debris. The upper limit of the numerical range may be the lower SFL-1 intensity than that of any subpopulation of white blood cells. By specifying a particle in which the SFL-1 intensity is within the numerical range from the particles in the measurement sample, debris can be excluded, and particles that are platelet clumps or malaria-infected red blood cells can be selected.

In step S113, the analysis unit 300 may specify, from the particles in the measurement sample, a particle in which the SFL-1 intensity is less than the first threshold value corresponding to the first fluorescence information and the SSC intensity is equal to or greater than the threshold value corresponding to the side scattered light information. As the threshold value corresponding to the side scattered light information, for example, the minimum value of the SSC intensity of the neutrophil population or the maximum value of the SSC intensity of the monocyte population may be set. Usually, the SSC intensity of the population of debris tends to be lower than the SSC intensity of the neutrophil population, platelet clumps and malaria-infected red blood cells. Therefore, when a particle indicating an SFL-1 intensity less than the first threshold value and indicating an SSC intensity less than or equal to the threshold value is extracted, the particles hardly contain not only white blood cells but also debris.

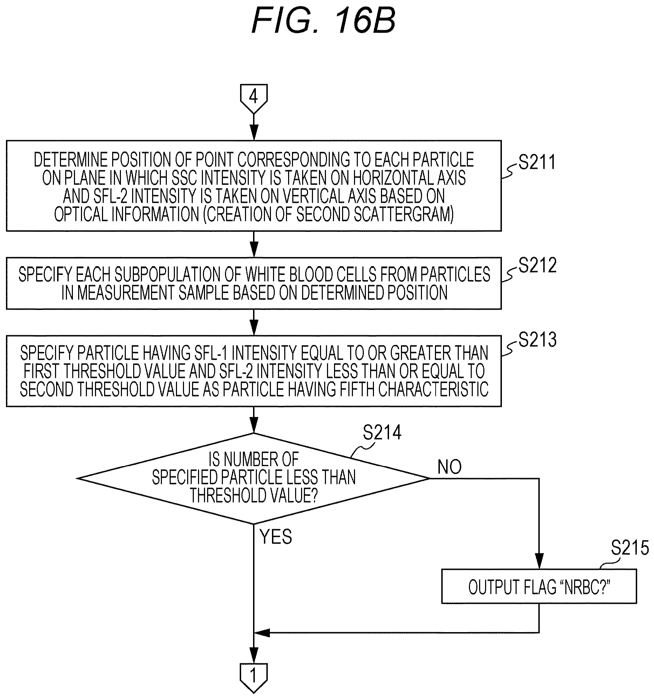

The analysis unit 300 can output information indicating the presence or absence of platelet clumps or malaria-infected red blood cells based on information of the particle having a fourth characteristic. At this time, the information of the particle having a fourth characteristic is, for example, the number and forward scattered light information of the particle. In step S114, the analysis unit 300 counts the number of particles having a fourth characteristic, and the analysis unit 300 determines whether the number is less than a corresponding threshold value. When the number of particles having a fourth characteristic is less than the threshold value, it is determined that no platelet clumps and malaria-infected red blood cell are contained in the measurement sample. In this case, the process proceeds to step S211 in Fig. 16B. The particle having a fourth characteristic may be excluded from the particles in the measurement sample by determining that the particle is debris. In step S114, when the number of particles having a fourth characteristic is equal to or greater than the corresponding threshold value, it is determined that platelet clumps or malaria-infected red blood cells are contained in the measurement sample. In this case, the process proceeds to step S115.

The threshold value corresponding to the number of particles having a fourth characteristic can be appropriately determined. For example, it may be empirically set by accumulating data of optical information obtained by measuring peripheral blood of a healthy person, peripheral blood in which platelet clumps has occurred, and peripheral blood containing malaria-infected red blood cells.

Conventionally, it has been difficult to discriminate between platelet clumps and malaria-infected red blood cells. As shown in A of Fig. 17, platelet clumps and malaria-infected red blood cells cannot be clearly distinguished by the SFL-1 intensity and the SSC intensity. However, the present inventors have found that platelet clumps and malaria-infected red blood cells can be discriminated from each other based on the forward scattered light information. In the method for analyzing a specimen of the present embodiment, in order to discriminate whether the particle having a fourth characteristic is platelet clumps or a malaria-infected red blood cell, whether the information on width of the forward scattered light signal of the particle having a fourth characteristic is equal to or greater than a corresponding threshold value is determined. The information on width of the forward scattered light signal is preferably a forward scattered light pulse width (hereinafter, also referred to as "FSC-W"). The platelet clumps are an aggregate of a plurality of platelets, and the malaria-infected red blood cell is a single particle. Therefore, there may be a difference in FSC-W between the two.

In step S115, the analysis unit 300 determines whether the FSC-W value of the particle having a fourth characteristic is equal to or greater than a threshold value corresponding to FSC-W. The analysis unit 300 may create a scattergram in which the FSC-W is taken on the horizontal axis and the FSC intensity is taken on the vertical axis as shown in B of Fig. 17. In B of Fig. 17, a dashed line indicates the threshold value corresponding to FSC-W. A population of malaria-infected red blood cells appears in a region on the left side of the dashed line on the scattergram. A population of platelet clumps appears in a region on the right side of the dashed line on the scattergram. When the FSC-W value of the particle having a fourth characteristic is equal to or greater than the threshold value corresponding to FSC-W, in step S116, the analysis unit 300 specifies the particle having a fourth characteristic as platelet clumps. Then, the analysis unit 300 outputs information indicating that platelet clumps are present. For example, a flag "PLT-clumps?" may be output to the display part 305 as information indicating the presence of platelet clumps. Thereafter, the process proceeds to step S211 in Fig. 16B. Not displaying the flag on the display part 305 can be information indicating the absence of platelet clumps.

When the FSC-W value of the particle having a fourth characteristic is less than the threshold value corresponding to FSC-W, in step S117, the analysis unit 300 specifies the particle having a fourth characteristic as a malaria-infected red blood cell. Then, the analysis unit 300 outputs information indicating that a malaria-infected red blood cell is present. For example, a flag "iRBCs?" may be output to the display part 305 as information indicating the presence of a malaria-infected red blood cell. Thereafter, the process proceeds to step S211 in Fig. 16B. Not displaying the flag on the display part 305 can be information indicating the absence of a malaria-infected red blood cell.

### (Classification of white blood cells based on second fluorescence information and side scattered light information)

Referring to Fig. 16B, in step S211, the analysis unit 300 determines a position of a point corresponding to each particle on a plane in which the SSC intensity is taken on the horizontal axis and the SFL-2 intensity is taken on the vertical axis based on the acquired optical information. The analysis unit 300 may create the second scattergram based on the determined position of each point. In step S212, the analysis unit 300 specifies each subpopulation of white blood cells from the particles in the measurement sample based on the determined position of each point.

### (Specification of particle having fifth characteristic)

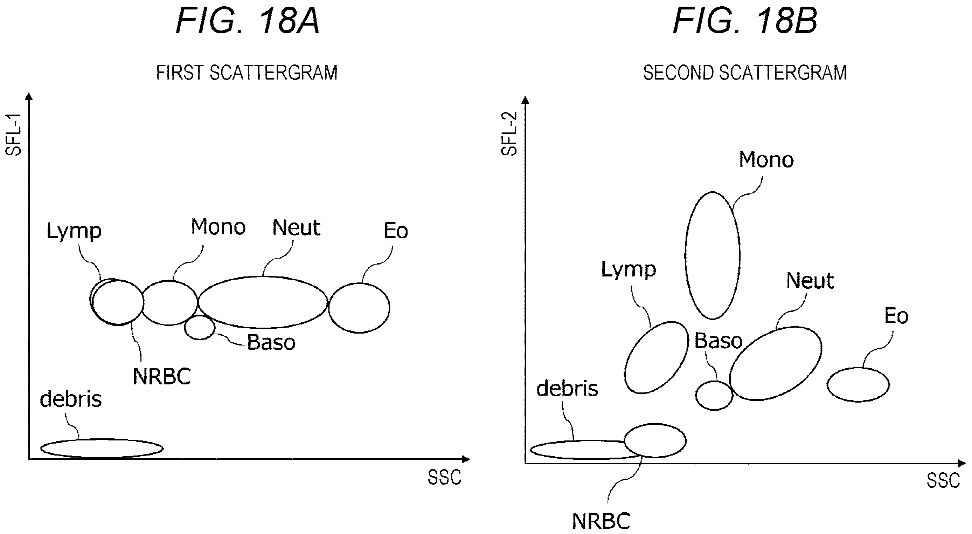

As shown in A of Fig. 18, in the first scattergram, a population of nucleated red blood cells appears at a position away from the population of debris, but a region where the nucleated red blood cell appears overlaps with a region where the lymphocyte population appears. Therefore, it has been difficult to specify a nucleated red blood cell only by the first scattergram. On the other hand, as shown in B of Fig. 18, in the second scattergram, the population of nucleated red blood cells appears at a position away from each subpopulation of white blood cells, but a region where the nucleated red blood cell appears and a region where debris appears are close to each other. Therefore, it has been difficult to specify a nucleated red blood cell only by the second scattergram. However, the present inventors have found that nucleated red blood cells can be detected based on the first fluorescence information and the second fluorescence information. In the method for analyzing a specimen according to the present embodiment, in order to detect nucleated red blood cells (NRBCs) in the measurement sample, a particle in which the first fluorescence information is equal to or greater than a first threshold value corresponding to the first fluorescence information and a second fluorescence information less than or equal to a second threshold value corresponding to the second fluorescence information is specified as a particle having a fifth characteristic. The first fluorescence information is preferably the SFL-1 intensity, and the second fluorescence information is preferably the SFL-2 intensity. The first threshold value corresponding to the first fluorescence information is as described above. By combining the first fluorescence information and the second fluorescence information, only nucleated red blood cells can be extracted. This point will be described below.

Nucleated red blood cells have almost the same amount of DNA as that of white blood cells. When nucleated red blood cells are contained in the measurement sample, particles with an SFL-1 intensity equal to or greater than the first threshold value corresponding to the first fluorescence information include nucleated red blood cells and white blood cells. Nucleated red blood cells have a smaller amount of RNA than each subpopulation of white blood cells. The SFL-2 intensity reflects the amount of RNA contained in the particle. As the second threshold value corresponding to the second fluorescence information, for example, the minimum value of the SFL-2 intensity of all particles constituting the lymphocyte population (hereinafter, also referred to as "the minimum value of the SFL-2 intensity of the second lymphocyte population") can be set. When the neutrophil population, the eosinophil population, or the basophil population has been specified, for example, the minimum value of the SFL-2 intensity of all particles constituting the neutrophil population, the eosinophil population, or the basophil population (hereinafter, also referred to as "the minimum value of the SFL-2 intensity of the neutrophil population, the eosinophil population or the basophil population") may be set as the second threshold value corresponding to the second fluorescence information. When a particle indicating an SFL-2 intensity less than such second threshold value is extracted, the particles hardly contain white blood cells. A particle in which the SFL-2 intensity is less than or equal to the second threshold value corresponding to the second fluorescence information is a particle with a smaller amount of RNA than that of white blood cells. Thus, when nucleated red blood cells are contained in the measurement sample, particles with an SFL-2 intensity less than or equal to the second threshold value corresponding to the second fluorescence information includes nucleated red blood cells and debris. Therefore, the particle in which the SFL-1 intensity is equal to or greater than the first threshold value corresponding to the first fluorescence information and the SFL-2 intensity is less than or equal to the second threshold value corresponding to the second fluorescence information include nucleated red blood cells but do not include white blood cells and debris.

Referring to Fig. 16B, in step S213, the analysis unit 300 specifies a particle in which the SFL-1 intensity is equal to or greater than the first threshold value corresponding to the first fluorescence information and the SFL-2 intensity is less than or equal to the second threshold value corresponding to the second fluorescence information as the particle having a fifth characteristic. The particle having a fifth characteristic may be specified based on the scattergram. For example, from the measurement data of all particles in the measurement sample, the measurement data of the particle indicating an SFL-1 intensity equal to or greater than the first threshold value corresponding to the first fluorescence information is extracted. As a result, the measurement data of debris can be excluded. Next, a second scattergram is created based on the data of the extracted particles. Since debris is excluded, no debris appears in the second scattergram. In the second scattergram, each subpopulation of white blood cells is distributed in a region equal to or greater than the second threshold value corresponding to the second fluorescence information. Therefore, the particle having a fifth characteristic can be specified on the second scattergram without being affected by debris and white blood cells.

### (Detection of nucleated red blood cells)

The analysis unit 300 can output information indicating the presence or absence of a nucleated red blood cell based on information of the particle having a fifth characteristic. At this time, the information of the particle having a fifth characteristic is, for example, the number of the particle. In step S214, the analysis unit 300 counts the number of specified particles, and the analysis unit 300 determines whether the number is less than a threshold value corresponding to the particles. When the number of particles having a fifth characteristic is less than the corresponding threshold value, it is determined that no nucleated red blood cell is contained in the measurement sample. In this case, the process proceeds to step S201 in Fig. 9B. In step S214, when the number of particles having a fifth characteristic is equal to or greater than the corresponding threshold value, in step S215, the analysis unit 300 specifies the particle having a fifth characteristic as a nucleated red blood cell. Then, the analysis unit 300 outputs information indicating that a nucleated red blood cell is present. For example, a flag "NRBC?" may be output to the display part 305 as information indicating the presence of a nucleated red blood cell. Thereafter, the process proceeds to step S201 in Fig. 9B. Not displaying the flag on the display part 305 can be information indicating the absence of a nucleated red blood cell.

The threshold value corresponding to the number of particles having a fifth characteristic can be appropriately determined. For example, it may be empirically set by accumulating data of optical information obtained by measuring peripheral blood of a healthy person and peripheral blood containing nucleated red blood cells.

As described above, the procedures of the analysis processing of Embodiment 2 have been described based on the flowchart. From this flowchart, a relationship between the information of the particles having fourth and fifth characteristics and the abnormal cell specified based on the information is extracted and described below.

Information indicating the presence or absence of a seventh abnormal cell and information indicating the presence or absence of an eighth abnormal cell are further output based on the information of the particle having a fourth characteristic. When the information of the particle having a fourth characteristic satisfies the following conditions 6) and 7), the particle having a fourth characteristic is specified as a seventh abnormal cell. Then, as the information indicating the presence or absence of a seventh abnormal cell, information indicating that platelet clumps are present is output.

6) The number of particles having a fourth characteristic is less than the corresponding threshold value; and

7) the information on width of the forward scattered light signal of the particle having a fourth characteristic is equal to or greater than the corresponding threshold value.

Alternatively, when the information of the particle having a fourth characteristic satisfies the following conditions 6) and 8), the particle having a fourth characteristic is specified as an eighth abnormal cell. Then, as the information indicating the presence or absence of an eighth abnormal cell, information indicating that the malaria-infected red blood cell is present is output.

6) The number of particles having a fourth characteristic is less than the corresponding threshold value; and

8) the information on width of the forward scattered light signal of the particle having a fourth characteristic is less than the corresponding threshold value.

Information indicating the presence or absence of a ninth abnormal cell is further output based on the information of the particle having a fifth characteristic. When the number of particles having a fifth characteristic is equal to or greater than the corresponding threshold value, the particle having a fifth characteristic is specified as a ninth abnormal cell. Then, as the information indicating the presence or absence of a ninth abnormal cell, information indicating that a nucleated red blood cell is present is output.

### (Analysis system used in Embodiment 3)

In Embodiment 3, the analysis system 500 capable of measuring both a blood sample and a body fluid sample are used. An example of the operation of such an analysis system 500 will be described with reference to Fig. 7, but it is not limited to this example. In step S11, the control part 301 of the analysis unit 300 receives an instruction to start measurement from a user via the operation part 306. Input reception of the instruction to start measurement is performed based on a specimen type selection screen displayed on the display part 305. The control part 301 displays a selection screen for designating the specimen type on the display part 305. On the selection screen, the user can designate, for example, either whole blood or body fluid as the specimen type. After setting the specimen type, measurement items, and the like, the user determines the start measurement displayed on the screen, whereby the measurement is performed. Note that the selection of the specimen type is not limited to this example, and for example, a specific type of body fluid such as body cavity fluid, cerebrospinal fluid, joint fluid, peritoneal dialysis drainage, or alveolar lavage fluid may be designated.

When receiving the instruction to start measurement and the designation of the specimen type, the control part 301 transmits instruction data for instructing the measurement start to the measurement unit 400. When receiving an input of designation of a body fluid as the specimen type, the control part 301 also transmits instruction data related to setting for body fluid measurement (for example, reaction time, measurement time, additional washing, and the like) to the measurement unit 400. In the case of measuring a body fluid sample, the reaction time with a reagent and the measurement time can be set to lengths different from those in the case of measuring a blood sample. For example, when the body fluid is a cerebrospinal fluid, the reaction time and the measurement time can be set longer than those in the case of measuring a blood sample. In order to suppress carryover between the blood sample and the body fluid sample and the like, it can be set such that additional cleaning is performed on a specimen suction section and a flow cell. The measurement unit 400 receives the instruction data to execute measurement processing in step S12.

### (Measurement processing of Embodiment 3)

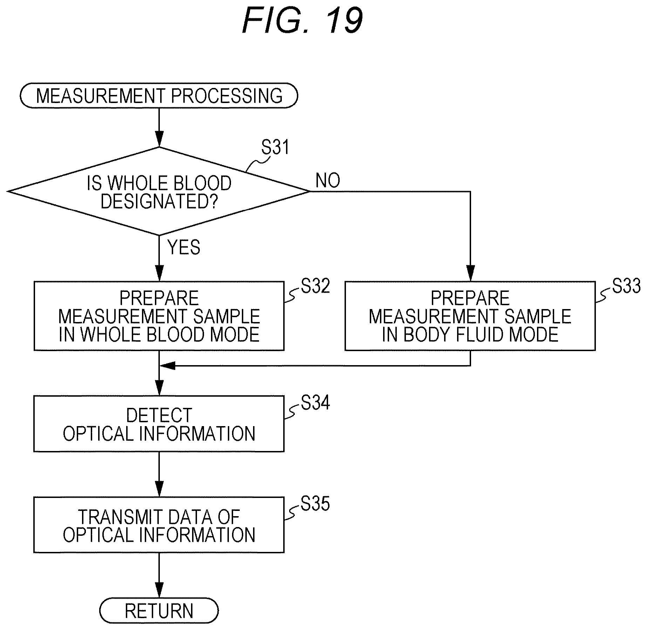

With respect to step S12 in Fig. 7, an example of measurement processing of Embodiment 3 will be described with reference to Fig. 19, but it is not limited to this example. In step S31, when the analysis unit 300 receives designation of whole blood as the specimen type, the control part 301 of the analysis unit 300 causes the measurement unit 400 to prepare a measurement sample in whole blood mode in step S32. Specifically, under the condition of preparing a measurement sample from the blood sample, the measurement unit 400 mixes the specimen that is whole blood, the staining reagent, and the hemolysis reagent in the reaction chamber 420 to prepare a measurement sample. In step S31, when the analysis unit 300 receives designation of a body fluid as the specimen type, the control part 301 causes the measurement unit 400 to prepare a measurement sample in body fluid mode in step S33. Specifically, under the condition of preparing a measurement sample from the body fluid sample, the measurement unit 400 mixes the specimen that is a body fluid, the staining reagent, and the hemolysis reagent in the reaction chamber 420 to prepare a measurement sample. In step S34, the control part 301 causes the measurement unit 400 to lead the prepared measurement sample to the FCM detection section 460, and the control part 301 executes optical measurement by a flow cytometry method. In step S35, the control part 301 causes the measurement unit 400 to transmit the optical information to the analysis unit 300, and the control part 301 ends the measurement processing. Then, the analysis unit 300 executes the analysis processing in step S13 in Fig. 7.

### (Analysis processing of Embodiment 3)

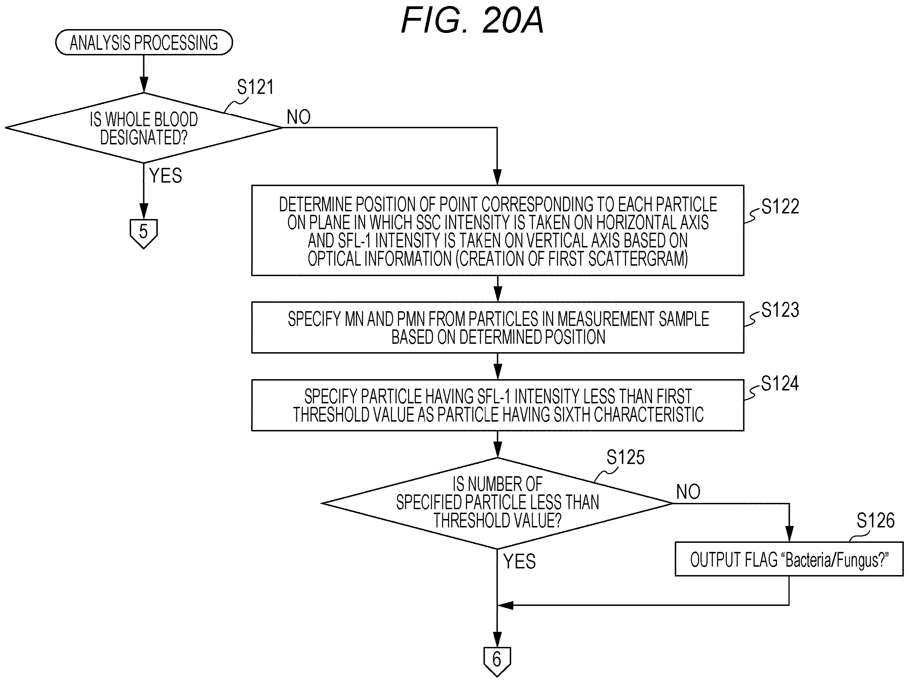

With respect to step S13 in Fig. 7, an example of analysis processing when the analysis unit 300 receives designation of a body fluid sample as the specimen type will be described with reference to Figs. 20A to 23, but it is not limited to this example. In this analysis processing, classification of white blood cells and detection of the following abnormal cells are enabled: bacteria or fungi, tissue-derived cells, and DNA aneuploid cells. The analysis processing when designation of a blood sample is received is similar to that described for Embodiments 1 and 2. In the example of analysis processing of Embodiment 3, the side scattered light information is used as the scattered light information. More specifically, the SSC intensity is used as the side scattered light information. Instead of the SSC intensity, the first side scattered light intensity may be used. The SFL-1 intensity is used as the first fluorescence information, and the SFL-2 intensity is used as the second fluorescence information. If necessary, the first and second scattergrams may be created. The vertical axis of these scattergrams may be a logarithmic scale of each fluorescence intensity.

### (Classification of white blood cells based on first fluorescence information and side scattered light information)

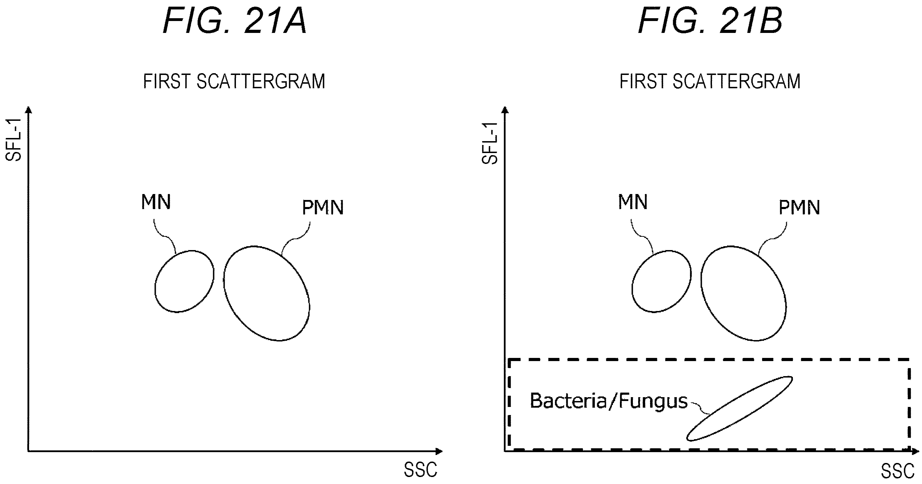

Referring to Fig. 20A, in step S121, when the analysis unit 300 receives designation of whole blood as the specimen type, the process proceeds to step S101 in Fig. 9A. Alternatively, the process may proceed to step S111 in Fig. 16A. When the analysis unit 300 receives designation of a body fluid as the specimen type, the process proceeds to step S122. In step S122, the analysis unit 300 determines a position of a point corresponding to each particle on a plane in which the SSC intensity is taken on the horizontal axis and the SFL-1 intensity is taken on the vertical axis based on the acquired optical information. The analysis unit 300 may create the first scattergram based on the determined position of each point. In step S112, the analysis unit 300 specifies a mononuclear cell population and a polymorphonuclear cell population from the particles in the measurement sample based on the determined position of each point. The mononuclear cell is a generic term for lymphocytes and monocytes. Where possible, lymphocyte and monocyte populations may be specified. The mononuclear cell population is denoted as "MN" in the flowchart. The polymorphonuclear cell is a generic term for neutrophils, eosinophils, and basophils. Where possible, at least one of a neutrophil population, an eosinophil population or a basophil population may be specified. The polymorphonuclear cell population is denoted as "PMN" in the flowchart. An algorithm itself for classifying white blood cells into a mononuclear cell population and a polymorphonuclear cell population is known. For example, white blood cells can be classified into subpopulations by a program installed in the analysis unit 300. Since the positions at which the mononuclear cell population and the polymorphonuclear cell population of white blood cells appear on the scattergram are known, each subpopulation may be specified based on the scattergram. When the first scattergram has been created, for example, as shown in A of Fig. 21, the mononuclear cell population and the polymorphonuclear cell population of white blood cells are distributed on the scattergram. However, without being limited to this figure, white blood cells may be classified into, for example, three subpopulations: lymphocytes, monocytes, and granulocytes. Alternatively, white blood cells may be classified into four subpopulations: lymphocytes, monocytes, neutrophils, and eosinophils. Alternatively, white blood cells may be classified into five subpopulations: lymphocytes, monocytes, neutrophils, eosinophils, and basophils. If necessary, the cells included in each subpopulation of white blood cells may be counted. After classifying the white blood cells, the process proceeds to step S124.

### (Specification of particle having sixth characteristic)

The present inventors have found that bacteria and fungi can be detected based on the first fluorescence information. Specifically, since bacteria and fungi have a smaller amount of DNA than that of white blood cells, the SFL-1 intensity tends to be low. In the flow cytometry method, any bacteria and fungi are treated as a kind of particles in the measurement sample, and therefore the types of bacteria and fungi are not particularly limited. In the method for analyzing a specimen of the present embodiment, a particle having a sixth characteristic is specified in order to detect bacteria and fungi in the measurement sample. The particle having a sixth characteristic is a particle in which the first fluorescence information is less than the first threshold value corresponding to the first fluorescence information. The first fluorescence information is preferably the SFL-1 intensity. In step S124, the analysis unit 300 specifies a particle in which the SFL-1 intensity is less than the first threshold value corresponding to the first fluorescence information as the particle having a sixth characteristic. The first threshold value corresponding to the first fluorescence information is as described above when white blood cells has been classified into a lymphocyte population, a monocyte population, a neutrophil population, an eosinophil population, or a basophil population. Alternatively, as the first threshold value corresponding to the first fluorescence information, for example, the minimum value of the SFL-1 intensity of all particles constituting the mononuclear cell population (hereinafter, also referred to as "the minimum value of the SFL-1 intensity of the mononuclear cell population") can be set. Alternatively, the minimum value of the SFL-1 intensity of all particles constituting the polymorphonuclear cell population (hereinafter, also referred to as "the minimum value of the SFL-1 intensity of the polymorphonuclear cell population") may be set as the first threshold value corresponding to the first fluorescence information.

### (Detection of bacteria and fungi)

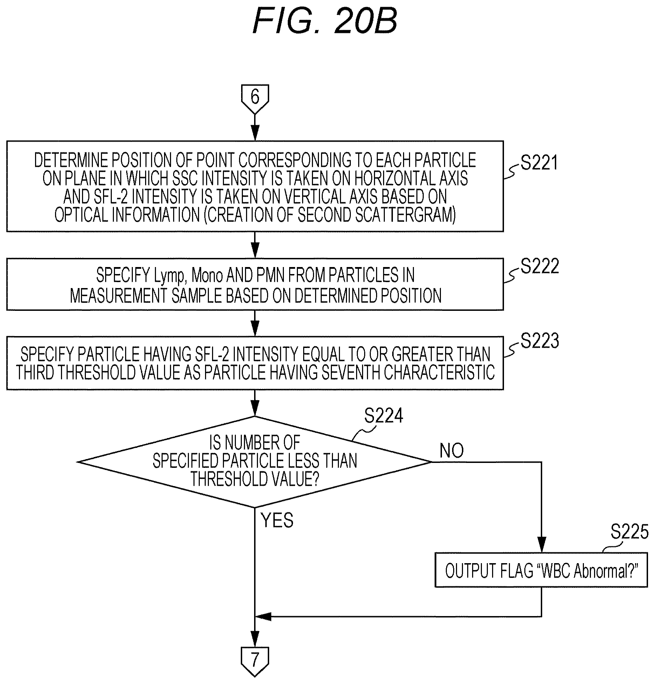

The analysis unit 300 can output information indicating the presence or absence of a bacterium or fungus based on information of the particle having a sixth characteristic. The information of the particle having a sixth characteristic is, for example, the number of the particle. In step S125, the analysis unit 300 counts the number of particles having a sixth characteristic, and the analysis unit 300 determines whether the number is less than a corresponding threshold value. When the number of particles having a sixth characteristic is less than the threshold value, it is determined that no bacterium and fungus are contained in the measurement sample. In this case, the process proceeds to step S221 in Fig. 20B. In step S125, when the number of particles having a sixth characteristic is equal to or greater than the corresponding threshold value, in step S126, the analysis unit 300 specifies the particle having a sixth characteristic as a bacterium or fungus. Then, the analysis unit 300 outputs information indicating that a bacterium or fungus is present. For example, a flag "Bacteria/Fungus?" may be output to the display part 305 as information indicating the presence of a bacterium or fungus. When the first scattergram has been created, a particle contained in a region where bacteria and fungi appear may be specified based on the scattergram. For example, as shown in B of Fig. 21, bacteria and fungi are distributed in a region where the SFL-1 intensity is lower than that of each subpopulation of white blood cells (a region surrounded by a dashed line). Thereafter, the process proceeds to step S221 in Fig. 20B. Not displaying the flag on the display part 305 can be information indicating the absence of a bacterium and a fungus.

The threshold value corresponding to the number of particles having a sixth characteristic can be appropriately determined. For example, it may be empirically set by accumulating data of optical information obtained by measuring a body fluid of a healthy person and a body fluid containing bacteria or fungi.

### (Classification of white blood cells based on second fluorescence information and side scattered light information)

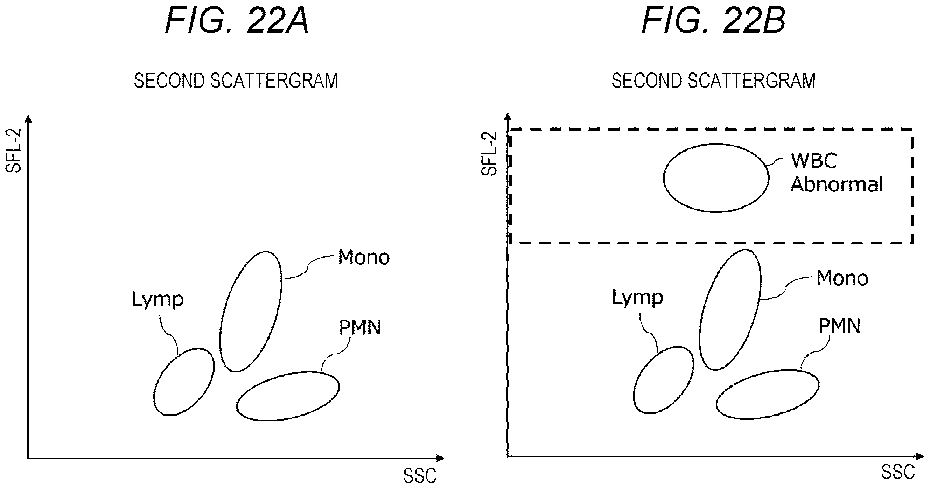

Referring to Fig. 20B, in step S221, the analysis unit 300 determines a position of a point corresponding to each particle on a plane in which the SSC intensity is taken on the horizontal axis and the SFL-2 intensity is taken on the vertical axis based on the acquired optical information. The analysis unit 300 may create the second scattergram based on the determined position of each point. In step S222, the analysis unit 300 specifies a lymphocyte population, a monocyte population, and a polymorphonuclear cell population from the particles in the measurement sample based on the determined position of each point. Where possible, at least one of a neutrophil population, an eosinophil population or a basophil population may be specified. The algorithm itself for classifying white blood cells into each subpopulation is known. For example, white blood cells can be classified into subpopulations by a program installed in the analysis unit 300. Since the positions at which the lymphocyte population, the monocyte population, and the polymorphonuclear cell population of white blood cells appear on the scattergram are known, each subpopulation may be specified based on the scattergram. When the second scattergram has been created, for example, as shown in A of Fig. 22, the lymphocyte population, the monocyte population, and the polymorphonuclear cell population of white blood cells are distributed on the scattergram. However, without being limited to this figure, white blood cells may be classified into, for example, two subpopulations: mononuclear cells and polymorphonuclear cells (granulocytes). Alternatively, white blood cells may be classified into four subpopulations: lymphocytes, monocytes, neutrophils, and eosinophils. Alternatively, white blood cells may be classified into five subpopulations: lymphocytes, monocytes, neutrophils, eosinophils, and basophils. If necessary, the cells included in each subpopulation of white blood cells may be counted. After classifying the white blood cells, the process proceeds to step S223.

### (Specification of particle having seventh characteristic)

The present inventors have found that tissue-derived cells can be detected based on the second fluorescence information. Specifically, since tissue-derived cells have a larger amount of RNA than that of white blood cells, the SFL-2 intensity of tissue-derived cells tends to be higher than that of white blood cells. Although the tissue-derived cells also have a large amount of DNA, the DNA aneuploid cells described later may also contain a large amount of DNA. Therefore, with the first fluorescence information, it has been difficult to discriminate between tissue-derived cells and DNA aneuploid cells. In the method for analyzing a specimen of the present embodiment, a particle having a seventh characteristic is specified in order to detect tissue-derived cells in the measurement sample. The particle having a seventh characteristic is a particle in which a second fluorescence information is equal to or greater than a third threshold value corresponding to the second fluorescence information. The second fluorescence information is preferably the SFL-2 intensity. In step S223, the analysis unit 300 specifies a particle in which the SFL-2 intensity is equal to or greater than the third threshold value corresponding to the second fluorescence information as the particle having a seventh characteristic. As the third threshold value corresponding to the second fluorescence information, for example, the maximum value of the SFL-2 intensity of all particles constituting the mononuclear cell population (hereinafter, also referred to as "the maximum value of the SFL-2 intensity of the mononuclear cell population") can be set. Alternatively, when the monocyte population has been specified, the maximum value of the SFL-2 intensity of the monocyte population may be set as the third threshold value corresponding to the second fluorescence information.

### (Detection of tissue-derived cells)

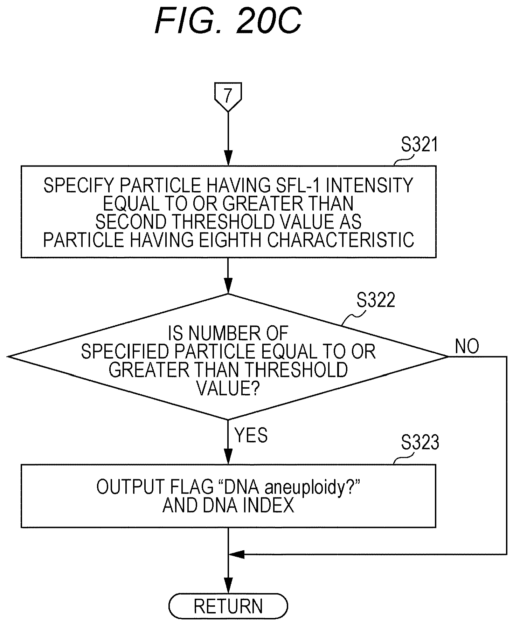

The analysis unit 300 can output information indicating the presence or absence of a tissue-derived cell based on information of the particle having a seventh characteristic. The information of the particle having a seventh characteristic is, for example, the number of the particle. In step S224, the analysis unit 300 counts the number of particles having a seventh characteristic, and the analysis unit 300 determines whether the number is less than a corresponding threshold value. When the number of particles having a seventh characteristic is less than the threshold value, it is determined that no tissue-derived cell is contained in the measurement sample. In this case, the process proceeds to step S321 in Fig. 20C. In step S224, when the number of particles having a seventh characteristic is equal to or greater than the corresponding threshold value, in step S225, the analysis unit 300 specifies the particle having a seventh characteristic as a tissue-derived cell. Then, the analysis unit 300 outputs information indicating that a tissue-derived cell is present. For example, a flag "WBC Abnormal?" may be output to the display part 305 as information indicating the presence of a tissue-derived cell. The number of particles having a seventh characteristic may be output to the display part 305 as "HF-BF count value" (the number of cells appearing in the high fluorescence region). When the second scattergram has been created, a particle contained in a region where tissue-derived cells appear may be specified based on the scattergram. For example, as shown in B of Fig. 22, the tissue-derived cells are distributed in a region where the SFL-2 intensity is higher than the maximum value of the SFL-2 intensity of the monocyte population (a region surrounded by a dashed line). The process then proceeds to step S321 of Fig. 20C. Not displaying the flag on the display part 305 can be information indicating the absence of a tissue-derived cell.

### (Specification of particle having eighth characteristic)

The present inventors have found that a DNA aneuploid cell can be detected based on the first fluorescence information. Specifically, since DNA aneuploid cells have a larger amount of DNA than that of white blood cells, the SFL-1 intensity of DNA aneuploid cells tend to be higher than that of white blood cells. In the method for analyzing a specimen of the present embodiment, a particle having an eighth characteristic is specified in order to detect a DNA aneuploid cell in the measurement sample. The particle having an eighth characteristic is a particle in which the first fluorescence information is equal to or greater than a second threshold value corresponding to the first fluorescence information. The first fluorescence information is preferably the SFL-1 intensity. In step S321, the analysis unit 300 specifies a particle in which the SFL-1 intensity is equal to or greater than the second threshold value corresponding to the first fluorescence information as the particle having an eighth characteristic. As the second threshold value corresponding to the first fluorescence information, for example, the maximum value of the SFL-1 intensity of all particles constituting the mononuclear cell population (hereinafter, also referred to as "the maximum value of the SFL-1 intensity of the mononuclear cell population") can be set. Alternatively, when the polymorphonuclear cell population has been specified, the maximum value of the SFL-1 intensity of the polymorphonuclear cell population may be set as the second threshold value corresponding to the first fluorescence information.

### (Detection of DNA aneuploid cell)

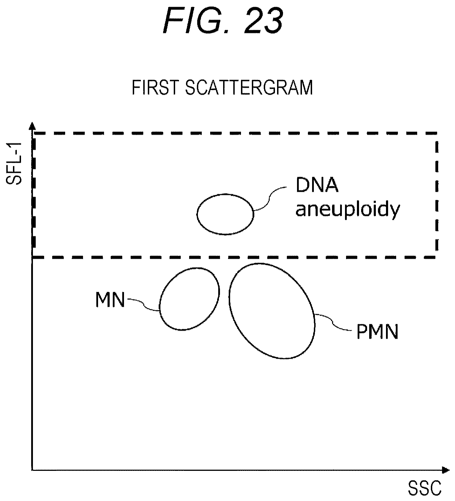

The analysis unit 300 can output information indicating the presence or absence of a DNA aneuploid cell based on information of the particle having an eighth characteristic. The information of the particle having an eighth characteristic is, for example, the number of the particle. In step S322, the analysis unit 300 counts the number of particles having an eighth characteristic, and the analysis unit 300 determines whether the number is equal to or greater than a corresponding threshold value. When the number of tissue-derived cells is acquired in step S224, it is preferable to subtract the number of tissue-derived cells from the number of particles acquired in step S322. This is because tissue-derived cells also have a large amount of DNA. That is, when tissue-derived cells and DNA aneuploid cells are contained in the measurement sample, the particles having an eighth characteristic may contain not only DNA aneuploid cells but also tissue-derived cells. When the number of particles having an eighth characteristic is less than the corresponding threshold value, it is determined that no DNA aneuploid cell is contained in the measurement sample. In this case, the analysis unit 300 ends the analysis processing, and the process proceeds to step S14 in Fig. 7. In step S322, when the number of particles having an eighth characteristic is equal to or greater than the corresponding threshold value, in step S323, the analysis unit 300 specifies the particle having an eighth characteristic as a DNA aneuploid cell. Then, the analysis unit 300 outputs information indicating that a DNA aneuploid cell is present. For example, a flag "DNA aneuploidy?" may be output to the display part 305 as information indicating the presence of a DNA aneuploid cell. The analysis unit 300 may further calculate DNA index and output a value thereof. The DNA index is as described above. When the first scattergram has been created, a particle contained in a region where DNA aneuploid cells appear may be specified based on the scattergram. For example, as shown in Fig. 23, the DNA aneuploid cell is distributed in a region where the SFL-1 intensity is higher than the maximum value of the SFL-1 intensity of the mononuclear cell population (a region surrounded by a dashed line). The analysis unit 300 ends the analysis processing, and the process proceeds to step S14 in Fig. 7. Not displaying the flag on the display part 305 can be information indicating the absence of a DNA aneuploid cell.

As described above, the procedures of the analysis processing of Embodiment 3 have been described based on the flowchart. From this flowchart, a relationship between the information of the particles having sixth, seventh, and eighth characteristics and the abnormal cell specified based on the information is extracted and described below.

At least one particle selected from the particle having a sixth characteristic, the particle having a seventh characteristic, and the particle having an eighth characteristic is specified based on the first fluorescence information and the second fluorescence information. When the information of the particle having a sixth characteristic is obtained, information indicating the presence or absence of a tenth abnormal cell is output based on the information. When the number of particles having a sixth characteristic is equal to or greater than the corresponding threshold value, the particle having a sixth characteristic is specified as a tenth abnormal cell. Then, as the information indicating the presence or absence of a tenth abnormal cell, information indicating that a bacterium or fungus is present is output.

When the information of the particle having a seventh characteristic is obtained, information indicating the presence or absence of an eleventh abnormal cell is output based on the information. When the number of particles having a seventh characteristic is equal to or greater than the corresponding threshold value, the particle having a seventh characteristic is specified as an eleventh abnormal cell. Then, as the information indicating the presence or absence of an eleventh abnormal cell, information indicating that a tissue-derived cell is present is output.

When the information of the particle having an eighth characteristic is obtained, information indicating the presence or absence of a twelfth abnormal cell is output based on the information. When the number of particles having an eighth characteristic is equal to or greater than the corresponding threshold value, the particle having an eighth characteristic is specified as a twelfth abnormal cell. Then, as the information indicating the presence or absence of a twelfth abnormal cell, information indicating that a DNA aneuploid cell is present is output.

Referring to Fig. 7, when the analysis processing is ended, the control part 301 outputs the analysis result to the display part 305 in step S14, and the control part 301 ends the processing. As described above, the processing of analyzing whether various abnormal cells are contained in the measurement sample has been individually described with reference to Figs. 9A to 23. However, some of these analysis processing may be performed, or all the analysis processing may be performed.

The analysis result screen output to the display part 305 may include, for example, the number of white blood cells, the number of each subpopulation of white blood cells (lymphocytes, monocytes, neutrophils, eosinophils, and basophils), and the number of detected abnormal cells. In addition to these pieces of information, the screen may include a scattergram created based on the optical information. The screen may further include a flag based on the analysis result.

As described above, the method for analyzing a specimen of the present embodiment can provide a medical worker such as a doctor or a medical technician with a result of classifying white blood cells and an analysis result of abnormal cells. The medical worker can determine, for example, whether or not to further examine the specimen from the acquired analysis result. The preparation of the measurement sample and analysis of the specimen described above are all performed in vitro.

### (Fluorescent dye)

Each fluorescent dye in the staining reagent used for preparing the measurement sample will be described. The first fluorescent dye is a fluorescent dye that specifically binds to DNA, and the second fluorescent dye is a fluorescent dye that specifically binds to RNA. The second fluorescent dye is a fluorescent dye having maximum absorption in a wavelength range different from that of the first fluorescent dye. That is, the second fluorescent dye is a fluorescent dye that emits fluorescence of a detectable wavelength separately from the fluorescence from the first fluorescent dye. Each of the first fluorescent dye and the second fluorescent dye can be appropriately selected from known fluorescent dyes having a property of binding to nucleic acid of blood cells such as white blood cells. As described above, since the first and second fluorescent dyes themselves stain nucleic acids of blood cells, in the method for analyzing a specimen of the present embodiment, it is not necessary to stain blood cells using antibodies labeled with these fluorescent dyes. Therefore, the first and second fluorescent dyes do not contain an antibody.

The first fluorescent dye and the second fluorescent dye are excited by light emitted from a light source included in the flow cytometer. The first fluorescent dye and the second fluorescent dye have fluorescence emission maxima in different wavelength ranges.

When a flow cytometer including two light sources is used as in the measurement unit 400, the light emitted from each light source is two types of light: light of a first wavelength capable of exciting the first fluorescent dye and light of a second wavelength capable of exciting the second fluorescent dye. The second wavelength is different from the first wavelength. The wavelength can be appropriately determined according to the type of fluorescent dye. For example, the first wavelength is 315 to 490 nm, preferably 400 to 450 nm, and more preferably 400 to 410 nm. The second wavelength is 610 to 750 nm, preferably 620 to 700 nm, and more preferably 633 to 643 nm.

The first fluorescent dye is a dye having a maximum absorption in a wavelength range of 400 to 490 nm and emitting fluorescence by being excited by absorbing light in the wavelength range, and the first fluorescent dye is preferably a dye having a property of binding to DNA of cells. Examples thereof include fluorescent dyes having an acridine skeleton, 4',6-diamidino-2-phenylindole dihydrochloride (DAPI), Hoechst 3342, Hoechst 33258, and Hoechst 334580 of Hoechst series, and the like.

Examples of the fluorescent dye having an acridine skeleton include proflavin, 9-aminoacridine, acridine orange, Acridine Yellow G, acriflavin, Basic Yellow 9, lactic acid ethacridine, Euchrysine GG (Euchrysine GGNX), proflavin hemisulfate, 3,6-bis(dimethylamino)acridine (Rhoduline Orange), and 3,6-diamino-2,7,10-trimethylacridinium chloride. Among them, Acridine Yellow G is preferable. Alternatively, a commercially available fluorescent dye may be used as the first fluorescent dye.

The second fluorescent dye is a dye having a maximum absorption in a wavelength range of 610 to 750 nm and emitting fluorescence by being excited by absorbing light in the wavelength range, and the first fluorescent dye is preferably a dye having a property of specifically binding to RNA of cells. Examples thereof include propidium iodide, ethidium bromide, ethidium-acridine heterodimer, ethidium diazide, ethidium homodimer-1, ethidium homodimer-2, ethidium monoazide, trimethylenebis[[3-[[4-[[(3-methylbenzothiazol-3-ium)-2-yl]methylene]-1,4-dihydroquinolin]-1-yl]propyl]dimethylaminium] · tetraiodide (TOTO-1), 4-[(3-methylbenzothiazol-2(3H)-ylidene)methyl]-1-[3-(trimethylaminio)propyl]quinolinium · diiodide (TO-PRO-1), N,N,N',N'-tetramethyl-N,N'-bis[3-[4-[3-[(3-methylbenzothiazol-3-ium)-2-yl]-2-propenylidene]-1,4-dihydroquinolin-1-yl]propyl]-1,3-propanediaminium · tetraiodide (TOTO-3), 2-[3-[[1-[3-(trimethylaminio)propyl]-1,4-dihydroquinolin]-4-ylidene]-1-propenyl]-3-methylbenzothiazol-3-ium · diiodide (TOPRO-3), fluorescent dyes represented by the following general formula (V), combinations thereof, and the like.

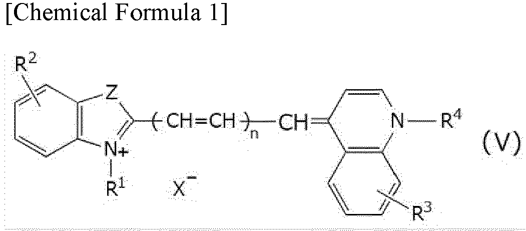

In the formula (V), R¹ and R⁴ are a hydrogen atom, a methyl group, an ethyl group, or an alkyl group having 6 to 18 carbon atoms, and when one of R¹ and R⁴ is an alkyl group having 6 to 18 carbon atoms, the other is a hydrogen atom, a methyl group, or an ethyl group. R² and R³ are the same as or different from each other, and R² and R³ are a methyl group, an ethyl group, a methoxy group, or an ethoxy group. Z is a sulfur atom, an oxygen atom, or a carbon atom having a methyl group. n is 0, 1, 2, or 3. X⁻ is an anion.

In the formula (V), the alkyl group having 6 to 18 carbon atoms may be linear or branched. Among the alkyl groups having 6 to 18 carbon atoms, an alkyl group having 6, 8 or 10 carbon atoms is preferable.

In the formula (V), examples of anion X⁻ include halogen ions such as F⁻, Cl⁻, Br⁻ and I⁻, CF₃SO₃⁻, BF₄⁻, ClO₄⁻, and the like.

As the fluorescent dye represented by the formula (V), a fluorescent dye represented by the following formula is preferable.

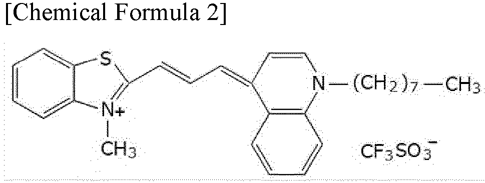

A commercially available staining reagent containing the second fluorescent dye alone may be used. Examples thereof include Fluorocell WDF (Sysmex Corporation), Stromatolyzer 4DS (Sysmex Corporation), and the like.

The first fluorescent dye and the second fluorescent dye are preferably used as solutions. The solvent is not particularly limited as long as it can dissolve each of the fluorescent dyes described above. Examples thereof include water, organic solvents, and mixtures thereof. The organic solvent is preferably a solvent that can be mixed with water, and examples thereof include alcohols having 1 to 6 carbon atoms, ethylene glycol, diethylene glycol, polyethylene glycol, dimethyl sulfoxide (DMSO), and the like.

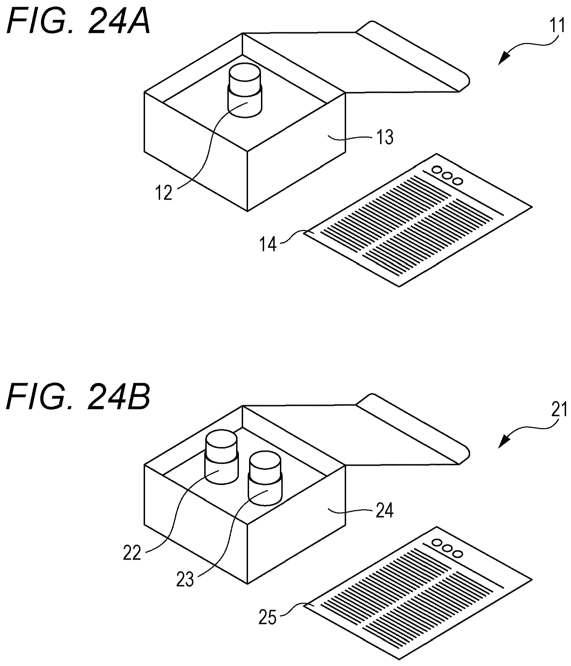

For the preparation of the measurement sample, it is preferable to use a staining reagent containing a first fluorescent dye and a second fluorescent dye. The staining reagent containing a first fluorescent dye and a second fluorescent dye may be stored in one reagent container. The reagent container containing the staining reagent may be packed in a box. The box may contain an attached document. Composition of the staining reagent, structure of each fluorescent dye, usage, storage method, and the like may be described in the attached document. Referring to Fig. 24A, 11 denotes the staining reagent of the present embodiment, 12 denotes a reagent container containing the staining reagent containing a first fluorescent dye and a second fluorescent dye, 13 denotes a packing box, and 14 denotes an attached document. Alternatively, the staining reagent containing a first fluorescent dye and a second fluorescent dye may be in a form in which a reagent containing a first fluorescent dye and a reagent containing a second fluorescent dye are stored in separate reagent containers. Referring to Fig. 24B, 21 denotes the staining reagent according to the present embodiment, 22 denotes a first reagent container containing a reagent containing a first fluorescent dye, 23 denotes a second reagent container containing a reagent containing a second fluorescent dye, 24 denotes a packing box, and 25 denotes an attached document.

The concentration of the first fluorescent dye in the staining reagent can be appropriately determined according to the type of fluorescent dye. The concentration of the first fluorescent dye is, for example, 0.01 mg/L or more, preferably 0.1 mg/L or more, and more preferably 0.5 mg/L. The concentration of the first fluorescent dye is, for example, 100 mg/L or less, preferably 75 mg/L or less, and more preferably 50 mg/L or less. The concentration of the second fluorescent dye in the staining reagent is the same as that of the first fluorescent dye.

One embodiment relates to a use of a first fluorescent dye and a second fluorescent dye for production of a staining reagent. The "staining reagent", the "first fluorescent dye", and the "second fluorescent dye" are as described above.

### (Hemolysis reagent)

The hemolysis reagent used for preparing the measurement sample will be described. The hemolysis reagent contains a surfactant, and is used in combination with a staining reagent containing a first fluorescent dye and a second fluorescent dye. The surfactant can hemolyze red blood cells in the specimen and damage cell membranes of cells other than red blood cells to such an extent that the first fluorescent dye and the second fluorescent dye can transmit therethrough. Examples of the surfactant include nonionic surfactants, cationic surfactants, and combinations thereof. The hemolysis reagent preferably contains a nonionic surfactant.

Examples of the nonionic surfactant include those represented by the following formula (I):

R¹-R²-(CH₂CH₂O)ₙ-H (I)

wherein R¹ is an alkyl group, an alkenyl group or an alkynyl group having 8 or more and 25 or less carbon atoms; R² is an oxygen atom, -(COO)-, or a group represented by the following formula (II): 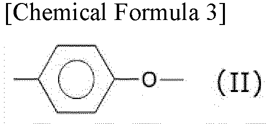 and; n is 23 or more and 25 or less, or 30.

In the formula (I), n is preferably 23 or 25, and more preferably n is 23. When n is 23 or more and 25 or less, the concentration of the nonionic surfactant represented by the formula (I) in the hemolysis reagent is 1700 ppm or more, and preferably 1750 ppm or more. When n is 23 or more and 25 or less, the concentration of the nonionic surfactant represented by the formula (I) in the measurement sample is 2300 ppm or less, and preferably 2200 ppm or less.

When n is 30, the concentration of the nonionic surfactant represented by the formula (I) in the hemolysis reagent is 1900 ppm or more, preferably 2000 ppm or more, and more preferably 2100 ppm or more. When n is 30, the concentration of the nonionic surfactant represented by the formula (I) in the measurement sample is 2300 ppm or less, and preferably 2200 ppm.

Specific examples of the nonionic surfactant represented by the formula (I) include polyoxyethylene alkyl ethers, polyoxyethylene sterol, polyoxyethylene castor oil, polyoxyethylene sorbitol fatty acid esters, polyoxyethylene alkyl amines, polyoxyethylene polyoxypropylene alkyl ethers, combinations thereof, and the like. Among them, polyoxyethylene alkyl ethers are preferable. The polyoxyethylene alkyl ether is preferably at least one selected from polyoxyethylene (23) cetyl ether, polyoxyethylene (25) cetyl ether, polyoxyethylene (30) cetyl ether, and a group thereof. Polyoxyethylene (23) cetyl ether, polyoxyethylene (25) cetyl ether, and a combination thereof are more preferable, and polyoxyethylene (23) cetyl ether is further preferable. The nonionic surfactant contained in the hemolysis reagent may be one kind or two or more kinds. The hemolysis reagent may further contain a nonionic surfactant other than the nonionic surfactant represented by the formula (I).

The hemolysis reagent may further include a cationic surfactant. Examples of the cationic surfactant include quaternary ammonium salt type surfactants, pyridium salt type surfactants, and combinations thereof. As the quaternary ammonium salt type surfactant, for example, a surfactant having 9 to 30 carbon atoms in total represented by the following formula (III) is preferable. The cationic surfactant contained in the hemolysis reagent may be one kind or two or more kinds.

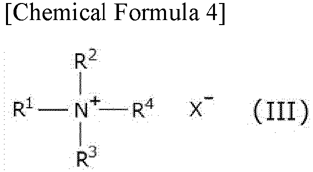

In the formula (III), R¹ is an alkyl group or alkenyl group having 6 to 18 carbon atoms; R² and R³ are the same as or different from each other, and R² and R³ are an alkyl group or alkenyl group having 1 to 4 carbon atoms; R⁴ is an alkyl group or alkenyl group having 1 to 4 carbon atoms, or a benzyl group, and X⁻ is a halogen ion.

In the formula (III), R¹ is preferably an alkyl group or alkenyl group having 6, 8, 10, 12 or 14 carbon atoms, and R¹ is particularly preferably a straight chain alkyl group. More specific examples of R¹ include an octyl group, a decyl group, and a dodecyl group. R² and R³ are the same as or different from each other, and R² and R³ are preferably a methyl group, an ethyl group, or a propyl group. R⁴ is preferably a methyl group, an ethyl group, or a propyl group.

Examples of the pyridium salt type surfactant include surfactants represented by formula (IV).

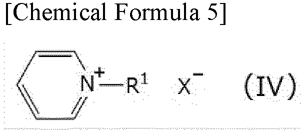

In the formula (IV), R¹ is an alkyl group or alkenyl group having 6 to 18 carbon atoms; and X⁻ is a halogen ion.

In the formula (IV), R¹ is preferably an alkyl group or alkenyl group having 6, 8, 10, 12 or 14 carbon atoms, and R¹ is particularly preferably a straight chain alkyl group. More specific examples of R¹ include an octyl group, a decyl group, and a dodecyl group.

The concentration of the cationic surfactant in the hemolysis reagent can be appropriately selected depending on the type of surfactant. The concentration of the cationic surfactant is 10 ppm or more. The concentration of the cationic surfactant is preferably 400 ppm or more, more preferably 500 ppm or more, and further preferably 600 ppm or more. The cationic surfactant concentration is 10000 ppm or less. The concentration of the cationic surfactant is preferably 1000 ppm or less, more preferably 800 ppm or less, and further preferably 700 ppm or less.

The hemolysis reagent may contain a buffer substance for keeping the pH constant. Examples thereof include inorganic acid salts, organic acid salts, Good's buffers, combinations thereof, and the like. Examples of the inorganic acid salt include phosphates, borates, combinations thereof, and the like. Examples of the organic acid salt include citrates, malates, combinations thereof, and the like. Examples of the Good's buffer include MES, Bis-Tris, ADA, PIPES, Bis-Tris-Propane, ACES, MOPS, MOPSO, BES, TES, HEPES, HEPPS, Tricine, Tris, Bicine, TAPS, combinations thereof, and the like.

The hemolysis reagent may further contain an aromatic organic acid. In the present specification, the aromatic organic acid means an acid having at least one aromatic ring in the molecule and a salt thereof. Examples of the aromatic organic acid include aromatic carboxylic acids, aromatic sulfonic acids, and the like. Examples of the aromatic carboxylic acid include phthalic acid, benzoic acid, salicylic acid, hippuric acid, salts thereof, combinations thereof, and the like. Examples of the aromatic sulfonic acid include p-aminobenzenesulfonic acid, benzenesulfonic acid, salts thereof, combinations thereof, and the like. The aromatic organic acid contained in the hemolysis reagent may be one kind or two or more kinds. The aromatic organic acid may exhibit a buffering action. When an aromatic organic acid exhibiting a buffering action is used, the addition of a buffering agent is optional, and the aromatic organic acid may be combined with the above buffering agent.

When the hemolysis reagent contains an aromatic organic acid, the concentration of the aromatic organic acid is not particularly limited, and is preferably 20 mM or more and more preferably 25 mM or more from the viewpoint of classification ability between monocytes and lymphocytes. The concentration of the aromatic organic acid contained in the hemolysis reagent is preferably 50 mM or less, and more preferably 45 mM or less.

The hemolysis reagent is preferably a liquid reagent. The solvent is not particularly limited as long as it can dissolve each component such as the surfactant. Examples of the solvent include water, organic solvents, and mixtures thereof. The organic solvent is preferably a solvent that can be mixed with water, and examples thereof include alcohols having 1 to 6 carbon atoms, ethylene glycol, diethylene glycol, polyethylene glycol, DMSO, and the like.

The pH of the hemolysis reagent is not particularly limited, and the pH is preferably 5.5 or more. The pH is more preferably 5.7 or more, and further preferably 5.9 or more. The pH is preferably 7.2 or less. The pH is more preferably 6.9 or less, and further preferably 6.6 or less. For adjusting the pH, a known base (such as sodium hydroxide) or acid (such as hydrochloric acid) can be used.

In the hemolysis reagent, the osmotic pressure is not particularly limited, and is preferably 150 mOsm/kg or less, more preferably 130 mOsm/kg or less, and most preferably 110 mOsm/kg or less from the viewpoint of hemolysis efficiency of red blood cells. An appropriate osmotic pressure regulator may be added to regulate the osmotic pressure. Examples of the osmotic pressure regulator include sugars, amino acids, organic solvents, sodium chloride, combinations thereof, and the like.

As the hemolysis reagent, a commercially available hemolysis reagent for measuring blood cells may be used. Examples thereof include Lysercell WDF (Sysmex Corporation), Lysercell WDFII (Sysmex Corporation), and the like.

The first fluorescent dye and the second fluorescent dye are mixed with the specimen and the hemolysis reagent so that the concentrations (final concentrations) in the measurement sample are each within a predetermined range. The preferred final concentration of the first fluorescent dye in the measurement sample is 1000 ppm or less, preferably 100 ppm or less, and more preferably 10 ppm or less. The preferred final concentration of the first fluorescent dye in the measurement sample is 0.001 ppm or more, preferably 0.01 ppm or more, and more preferably 0.1 ppm or more. The preferred final concentration of the second fluorescent dye in the measurement sample is 1000 ppm or less, preferably 100 ppm or less, and more preferably 10 ppm or less. The preferred final concentration of the second fluorescent dye in the measurement sample is 0.001 ppm or more, preferably 0.01 ppm or more, and more preferably 0.1 ppm or more.

The mixing ratio of the hemolysis reagent, the staining reagent and the specimen is, for example, preferably 1000 : 1 or more : 1 or more in volume ratio. The mixing ratio is more preferably 1000 : 10 or more : 10 or more, and further preferably 1000 : 15 or more : 15 or more. The mixing ratio of the hemolysis reagent, the staining reagent and the specimen is preferably, for example, 1000 : 50 or less : 50 or less in volume ratio. The mixing ratio is more preferably 1000 : 30 or less : 30 or less, and further preferably 1000 : 25 or less : 25 or less. The mixing ratio of the staining reagent and the specimen may be the same or different.

### (Embodiment of flow cytometer with one light source)

The light may be emitted from one light source. When the light is emitted from one light source, the light emitted from the light source is light capable of exciting both the first fluorescent dye and the second fluorescent dye. As such light, light including a plurality of wavelengths is preferable, and examples thereof include white light. Alternatively, when the first fluorescent dye and the second fluorescent dye have maximum absorption in a wavelength range close to an extent that they can be excited by light of one wavelength, the light emitted from the light source may be the light of one wavelength. For example, when one of the first fluorescent dye and the second fluorescent dye has a maximum absorption in a wavelength range of 400 to 520 nm and the other has a maximum absorption in a wavelength range of 300 to 420 nm, both fluorescent dyes can be excited by light having a central wavelength at 400 to 420 nm, for example, light of 455 nm. Alternatively, for example, when the maximum absorption of the first fluorescent dye and the second fluorescent dye is within a wavelength range of 630 to 660 nm, one of the first fluorescent dye and the second fluorescent dye emits fluorescence having a peak in a wavelength range of 660 to 670 nm, and the other emits fluorescence having a peak in a wavelength range longer than 670 nm, both fluorescent dyes can be excited by light having a central wavelength at 630 to 655 nm, for example, light of 633 nm, and light generated from each fluorescent dye can be detected separately.

When the measurement sample is irradiated with light having one wavelength, for example, light having a wavelength of 633 nm, a combination is considered in which the maximum absorption of the first fluorescent dye and the second fluorescent dye is within a wavelength range of 630 to 660 nm, the second fluorescent dye emits fluorescence having a peak in a wavelength range of 660 to 670 nm, and the first fluorescent dye emits fluorescence having a peak in a wavelength range longer than 670 nm. As such a combination, for example, DRAQ5, DRAQ7 or DRAQ9 (BioStatus Limited) can be used as the first fluorescent dye, and the fluorescent dye represented by the above formula (V) can be used as the second fluorescent dye.

Hereinbelow, the present invention will be described in detail by examples, but the present invention is not limited to these examples.

### EXAMPLES

In the following Reference Examples and Examples, the method for analyzing a specimen of the present embodiment was performed using the following hemolysis reagent, staining reagent, and analyzer. Details are as described in [Measurement method] below.

### [Hemolysis reagent]

As the hemolysis reagent, Lysercell WDFII (Sysmex Corporation) was used.

### [Staining reagent]

### (First fluorescent dye)

As the first fluorescent dye, Acridine Yellow G (Kanto Chemical Co., Inc.) was used.

### (Second fluorescent dye)

As the second fluorescent dye, Dye Compound A described in U.S. Patent No. 6004816 was used. Dye Compound A was a compound in which R¹ is a methyl group, R² and R³ are a hydrogen atom, R⁴ is n-octyl, n is 1, Z is a sulfur atom, and X⁻ is CF₃SO₃⁻ in the above formula (V). The structural formula was as follows.

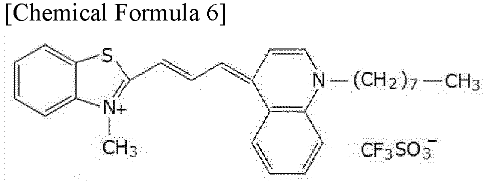

As described in U.S. Patent No. 6004816, Dye Compound A could be obtained by the following steps. One equivalent of 3-methyl-2-methylbenzothiazolium methanesulfate and three equivalents of N,N-diphenylformamidine were stirred in acetic acid for 1.5 hours with heating on an oil bath at 90°C. The reaction solution was poured into hexane, and a red oily matter was further suspended in and washed with hexane to remove acetic acid. The crude product was recrystallized with ethyl acetate-hexane (yield 48%). To the recrystallized product, one equivalent of 1-octyl lepidinium trifluorate and pyridine were added, and the mixture was stirred for 3 hours with heating on an oil bath at 90°C. The reaction solution was concentrated, and the remaining blue crude product was purified with methanol-chloroform by flash chromatography to obtain Dye Compound A as a dark blue powder (yield: 62%). The results of the obtained physical property tests (TLC, 1H-NMR, MASS, and the like) of Dye Compound A are described in U.S. Patent No. 6004816. Maximum absorption spectrum of Dye Compound A was 629 nm.

Dye Compound A (27.5 mg) and Acridine Yellow G (25 mg) were dissolved in special grade ethylene glycol (1 L) to prepare a staining reagent.

### [Analyzer]

A multi-item automatic blood cell analyzer XN-1000 (Sysmex Corporation) was modified as follows and used as an analyzer. The XN-1000 included hardware illustrated in Figs. 1 to 3, and FCM detection section 460 included a semiconductor laser light source that emits red (633 nm) light, a detector for red forward scattered light, a detector for red side scattered light, and a detector that receives fluorescence (light of 660 nm or more) excited by the red light. The XN-1000 was modified by adding a semiconductor laser light source that emits blue-violet (405 nm) light, a detector for blue-violet scattered light, and a fluorescence detector that detects fluorescence (450 to 600 nm) excited by blue-violet light. An FCM detection section of the modified XN-1000 had a configuration of Fig. 4. By connecting the XN-1000 to a computer having installed analysis software for flow cytometry Flowjo (trademark) (BD Biosciences), measurement data detected for scattered light information of red and blue-violet and fluorescence information generated from the first fluorescent dye and the second fluorescent dye were analyzed, and a desired scattergram was displayed.

### [Measurement method]

Preparation and measurement of a measurement sample were performed according to a manual attached to XN-1000 (Sysmex Corporation) except that the staining reagent prepared as described above was used in place of Fluorocell WDF (Sysmex Corporation) which is a staining reagent for XN-1000. Data analysis was performed by Flowjo (trademark). A measurement sample was prepared by mixing 1000 µL of Lysercell WDFII, 17 µL of a specimen, and 20 µL of the staining reagent. The dilution ratio of the staining reagent in the measurement sample was 51.85, and the final concentrations of the first fluorescent dye and the second fluorescent dye in the measurement sample were 0.53 ppm and 0.48 ppm, respectively.

### Reference Example 1: Fractionation of nucleated cells and non-nucleated components in normal specimen of blood sample

As a normal specimen of a blood sample, peripheral blood obtained from a healthy person was used. A measurement sample prepared from this specimen was measured by the above measuring device to acquire a first fluorescence information, a second fluorescence information, and a scattered light information. The first fluorescence information was fluorescence intensity (hereinafter referred to as "blue-violet fluorescence intensity") from the first fluorescent dye. The second fluorescence information was fluorescence intensity (hereinafter referred to as "red fluorescence intensity") from the second fluorescent dye. The scattered light information was a side scattered light intensity obtained by irradiating particles in the measurement sample with a red laser. Based on the acquired optical information, a scattergram in which the side scattered light intensity was taken on the horizontal axis and the blue-violet fluorescence intensity was taken on the vertical axis (first scattergram) was created. A scattergram in which the side scattered light intensity was taken on the horizontal axis and the red fluorescence intensity was taken on the vertical axis (second scattergram) was created.

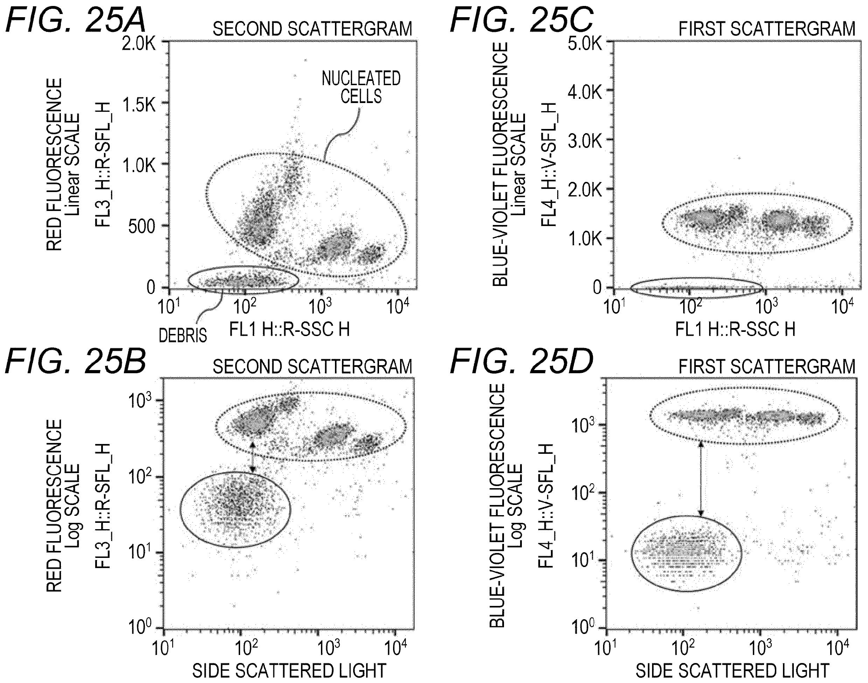

The created scattergrams are shown in Fig. 25. In Fig. 25, A is the second scattergram in which the vertical axis represents the red fluorescence intensity (linear scale), B is the second scattergram in which the vertical axis represents the red fluorescence intensity (logarithmic scale), C is the first scattergram in which the vertical axis represents the blue-violet fluorescence intensity (linear scale), and D is the first scattergram in which the vertical axis represents the blue-violet fluorescence intensity (logarithmic scale). In each scattergram, a particle group surrounded by a dashed ellipse was nucleated cells (white blood cells), and a particle group surrounded by a solid ellipse was non-nucleated components. The nucleated cells were mainly white blood cells. The non-nucleated components were debris including red blood cell ghosts, platelets, and the like. For the scattergrams in B and D of Fig. 25, the ratio of the fluorescence intensity of nucleated cells to the fluorescence intensity of non-nucleated components (WBC/Debris ratio) was calculated. The WBC/Debris ratio of B was 8.9, and the WBC/Debris ratio of D was 92.7. It was found that fractionation performance between nucleated cells and non-nucleated components was improved by the fluorescence from the first fluorescent dye.

### Example 1: Analysis of normal specimen of blood sample

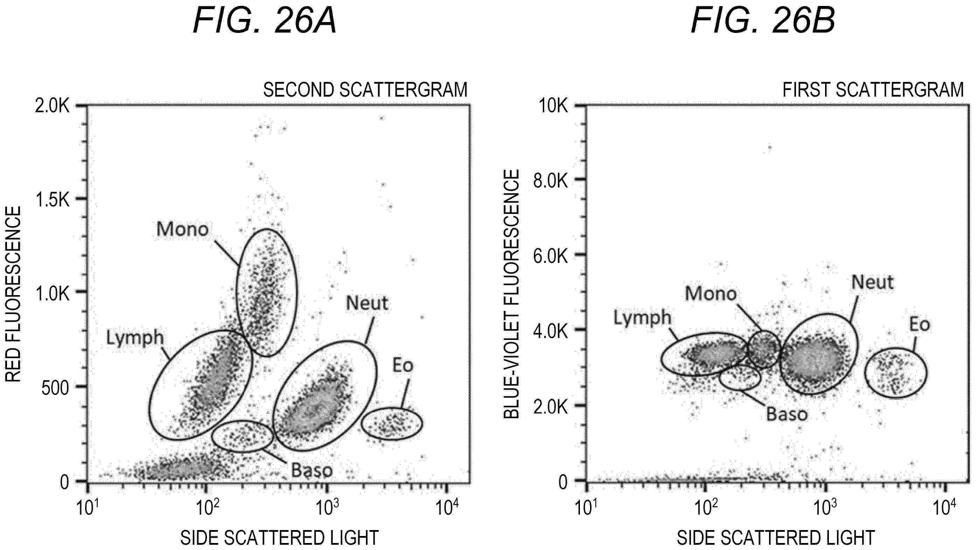

As a normal specimen of a blood sample, peripheral blood obtained from a healthy person was used. Measurement samples prepared from this specimen were measured, and first and second scattergrams were created. The created scattergrams are shown in Fig. 26. In the second scattergram shown in A of Fig. 26, white blood cells were classified into five subpopulations, that is, a lymphocyte population (Lymph), a monocyte population (Mono), a neutrophil population (Neut), an eosinophil population (Eo), and a basophil population (Baso). In the first scattergram shown in B of Fig. 26, the above five populations appeared in a region having substantially the same fluorescence intensity. From the first scattergram, no abnormal cells were detected.

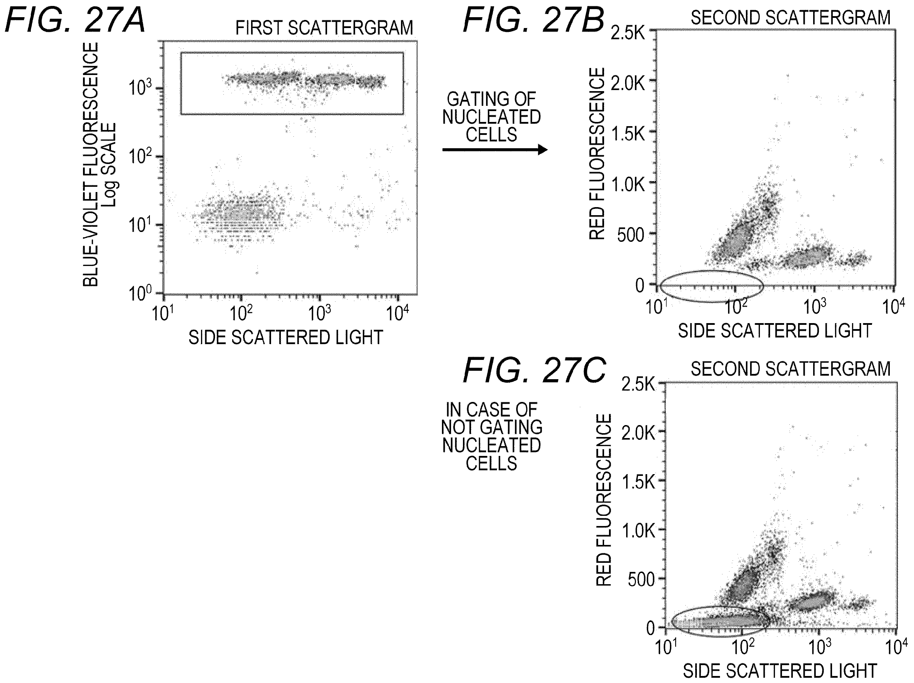

On the first scattergram (the vertical axis is logarithmic scale), a population of nucleated cells included in a region having a high blue-violet fluorescence intensity was sorted (gated), and the population was plotted on the second scattergram. The created scattergrams are shown in Fig. 27. In the first scattergram shown in A of Fig. 27, a population of nucleated cells (white blood cells) that appeared in a region surrounded by a solid square was gated. In the second scattergram shown in B of Fig. 27, the gated population of nucleated cells was classified into five subpopulations. A solid ellipse indicates a region where debris appears, but no debris appeared in the second scattergram of B. In the second scattergram shown in C of Fig. 27, a result of classifying all particles in the measurement sample without gating the population of nucleated cells in the first scattergram is shown. In C of Fig. 27, debris appeared in a region indicated by a solid ellipse. As described above, an influence of non-nucleated components such as debris can be excluded by gating the population of nucleated cells in the first scattergram. As a result, it was shown that white blood cells can be more accurately classified in the second scattergram.

### Example 2: Analysis of specimen containing platelet clumps (PLT-clumps)

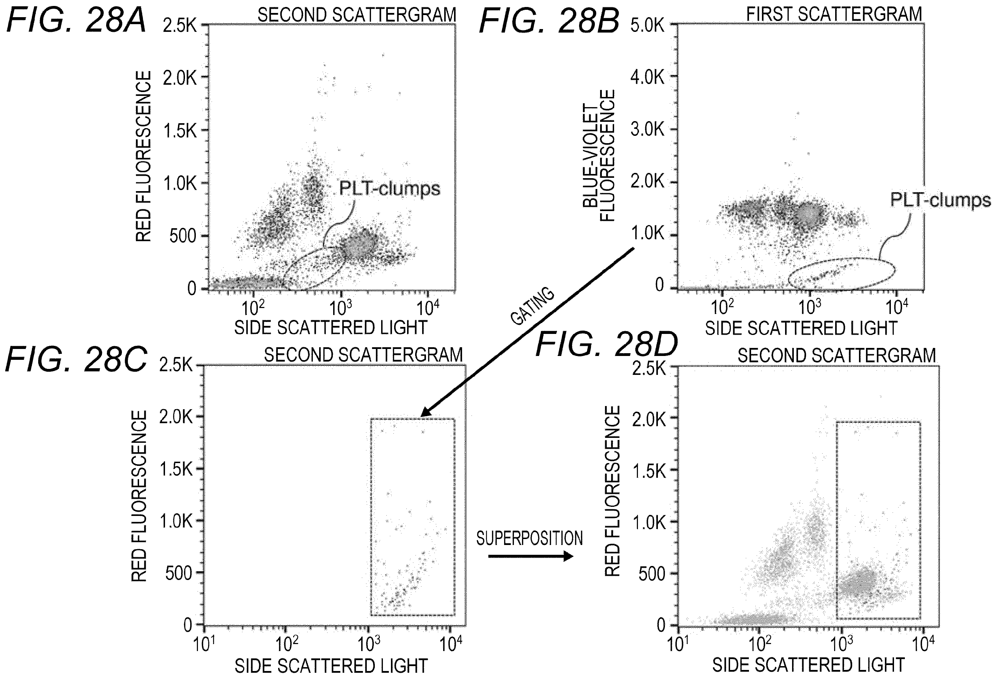

As a specimen, peripheral blood in which platelet clumps had occurred in a blood collection tube was used. Measurement samples prepared from this specimen were measured, and first and second scattergrams were created. The created scattergrams are shown in Fig. 28. In the second scattergram shown in A of Fig. 28, as described later, a region where the platelet clumps appeared overlapped with a region where the neutrophil population and the basophil population appeared. In the first scattergram shown in B of Fig. 28, a population suspected of having PLT-clumps appeared in a region surrounded by a dashed ellipse. In a normal specimen, a particle group is hardly observed in this region (see B of Fig. 26). As described above, when a predetermined number or more of particles are present in a region where the blue-violet fluorescence intensity of the first scattergram is low, a population of the particles is suspected to be PLT-clumps. Therefore, it was shown that among particle groups present in the region where the blue-violet fluorescence intensity of the first scattergram is low, a population having the same scattered light intensity as the neutrophil and eosinophil populations can be defined as PLT-clumps.

In the first scattergram shown in B of Fig. 28, the population defined as PLT-clumps was gated, and the population was plotted on the second scattergram. The created second scattergram is shown in C of Fig. 28. The population defined as PLT-clumps appeared in a region surrounded by a dashed square. The scattergram in C of Fig. 28 was superimposed on the original second scattergram (A of Fig. 28). This is shown in D of Fig. 28. As can be seen from the scattergram in D of Fig. 28, the region surrounded by a dashed square, that is, the region where the population defined as PLT-clumps had appeared overlapped with a region where the neutrophil population and the eosinophil population appeared. Therefore, it was difficult to detect and classify PLT-clumps only by the second scattergram. On the other hand, it was shown that PLT-clumps can be detected or classified by the first scattergram.

### Example 3: Distinction between platelet clumps (PLT-clumps) and infected red blood cells (iRBC)

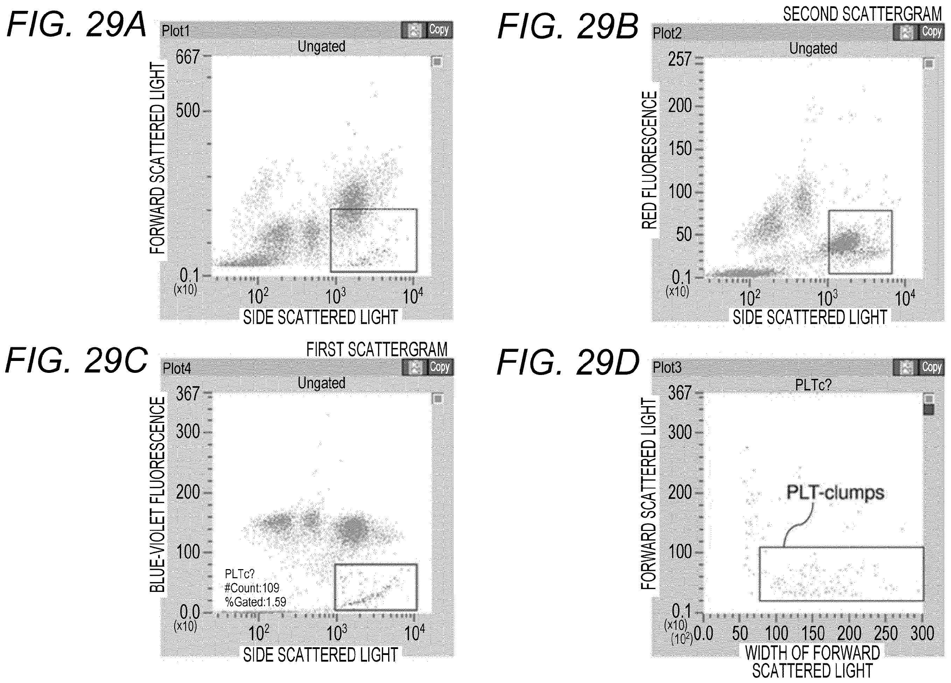

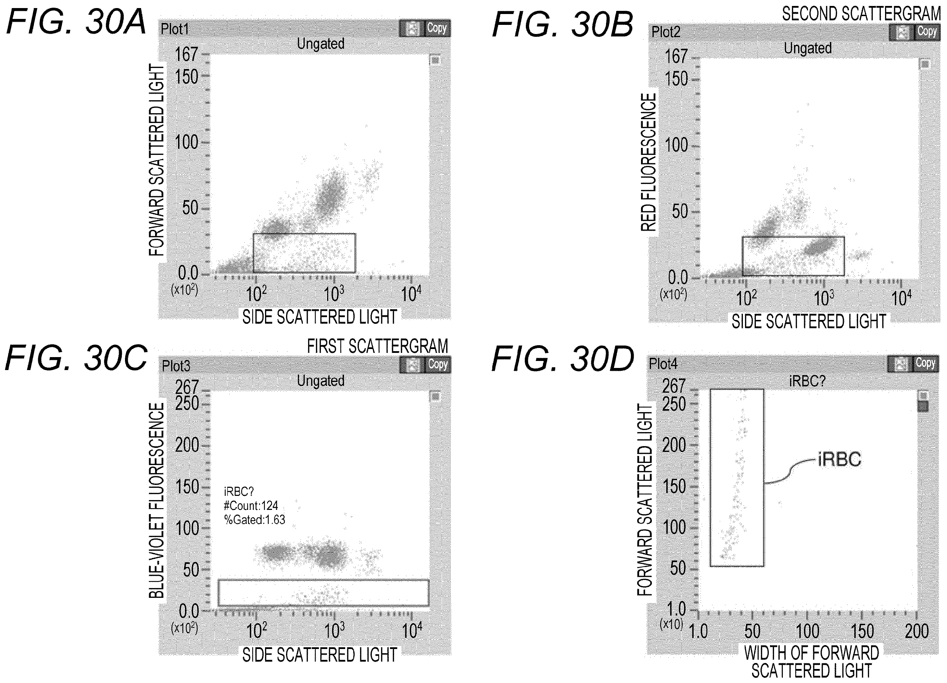

As a specimen containing PLT-clumps, peripheral blood in which platelet clumps had occurred in the blood collection tube was used. As a specimen containing iRBC, cultured malaria added human blood was used. The cultured malaria added human blood was acquired by infecting blood of a healthy person from which white blood cells had been removed in vitro with malaria parasites and culturing the blood for a predetermined period. A measurement sample prepared from each specimen was measured. In the measurement, in addition to blue-violet fluorescence intensity, red fluorescence intensity, and side scattered light intensity, intensity and width of forward scattered light signal were acquired. For each specimen, a scattergram was created based on the acquired information. The scattergrams for specimens containing PLT-clumps are shown in Fig. 29. The scattergrams for specimens containing iRBC are shown in Fig. 30. In Figs. 29 and 30, A is a scattergram in which the side scattered light intensity is taken on a horizontal axis and the forward scattered light intensity is taken on a vertical axis, B is a second scattergram, C is a first scattergram, D is a scattergram in which the forward scattered light width is taken on a horizontal axis and the forward scattered light intensity is taken on a vertical axis.

In the scattergrams in A and B of Fig. 29, a population of PLT-clumps appeared in a region surrounded by a solid square. However, it was difficult to detect the population of PLT-clumps due to the distribution of subpopulations of white blood cells. Similarly, in the scattergrams in A and B of Fig. 30, a population of iRBC appeared in a region surrounded by a solid square, but it was difficult to detect the population of iRBC. In the first scattergram in C of Fig. 29, a population suspected of having PLT-clumps appeared in a region where the blue-violet fluorescence intensity is low surrounded by a solid square. In the first scattergram in C of Fig. 30, a population suspected of having iRBC appeared in a region where the blue-violet fluorescence intensity is low surrounded by a solid square. When C of Fig. 29 was compared with C of Fig. 30, it was difficult to distinguish between PLT-clumps and iRBC by the first scattergram. Since PLT-clumps are aggregates, the width of the particle tends to be large. However, since iRBCs are malaria-infected red blood cells, they do not aggregate. It is known that the value of the forward scattered light width increases as the width of the particle passing through a flow cell is larger. Therefore, in the first scattergram, the population suspected of having PLT-clumps and the population suspected of having iRBC were gated, and plotted on the scattergrams in D of Figs. 29 and 30. In the scattergram in D of Fig. 29, the population suspected of having PLT-clumps appeared in a region where the value of the forward scattered light width is high surrounded by a solid square. On the other hand, in the scattergram in D of Fig. 30, the population suspected of having iRBC appeared in a region where the value of the forward scattered light width is low surrounded by a solid square. As described above, it was shown that PLT-clumps and iRBC can be distinguished by gating a particle group appeared in a region where the blue-violet fluorescence intensity of the first scattergram is low, and plotting the particle group on the scattergram in which the forward scattered light width is taken on a horizontal axis and the forward scattered light intensity is taken on a vertical axis.

### Example 4: Analysis of specimen containing immature granulocytes (IG)

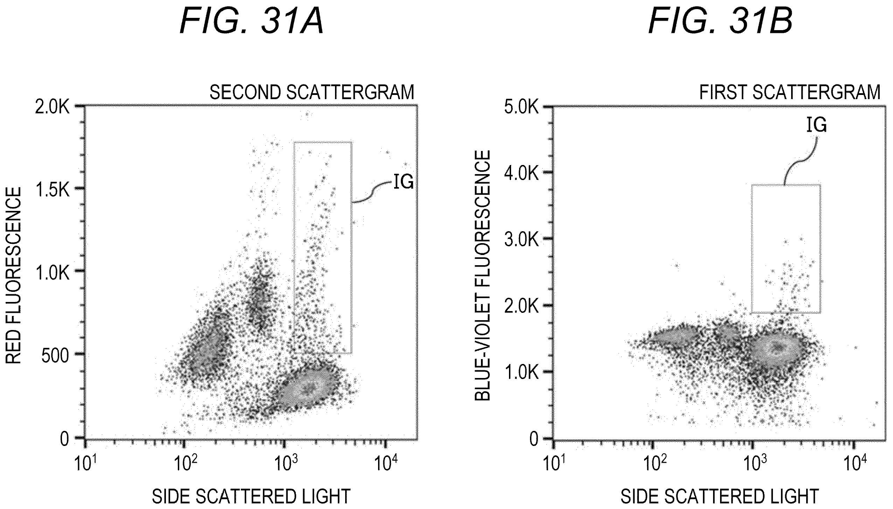

As a specimen containing IG, peripheral blood containing immature granulocytes was used. Measurement samples prepared from this specimen were measured, and first and second scattergrams were created. The created scattergrams are shown in Fig. 31. In the second scattergram shown in A of Fig. 31, normal white blood cells were classified into at least three subpopulations: lymphocytes, monocytes, and neutrophils. In A of Fig. 31, in the population classified as lymphocytes, particles having a red fluorescence intensity equal to or greater than the threshold value were hardly observed. The number of particles having a higher red fluorescence intensity than that of the monocyte population and having the same scattered light intensity as that of the monocyte population was small. However, a large number of particles having a higher red fluorescence intensity than that of the neutrophil population and having the same scattered light intensity as that of the neutrophil population appeared in a region surrounded by a solid square. Since this particle group was not observed in the normal specimen, the particle group appeared in the region was defined as an IG cluster.

Also in the first scattergram shown in B of Fig. 31, normal white blood cells were classified into at least three subpopulations: lymphocytes, monocytes, and neutrophils. In the first scattergram, particles having a higher blue-violet fluorescence intensity than that of the monocyte population and having the same scattered light intensity as that of the monocyte population were hardly observed. However, a large number of particles having a higher blue-violet fluorescence intensity than that of the neutrophil population and having the same scattered light intensity as that of the neutrophil population appeared in a region surrounded by a solid square. The particle group appeared in the region was defined as an IG cluster. As described above, it was shown that in the first or second scattergram, a region where the fluorescence intensity is higher than that of the neutrophil population and the scattered light intensity is the same as that of the neutrophil population can be defined as a region where the IG cluster appears. It was shown that when a predetermined number or more of particles appear in this defined region, the particles can be detected or classified as IG.

### Example 5: Classification of specimens containing nucleated red blood cells (NRBC)

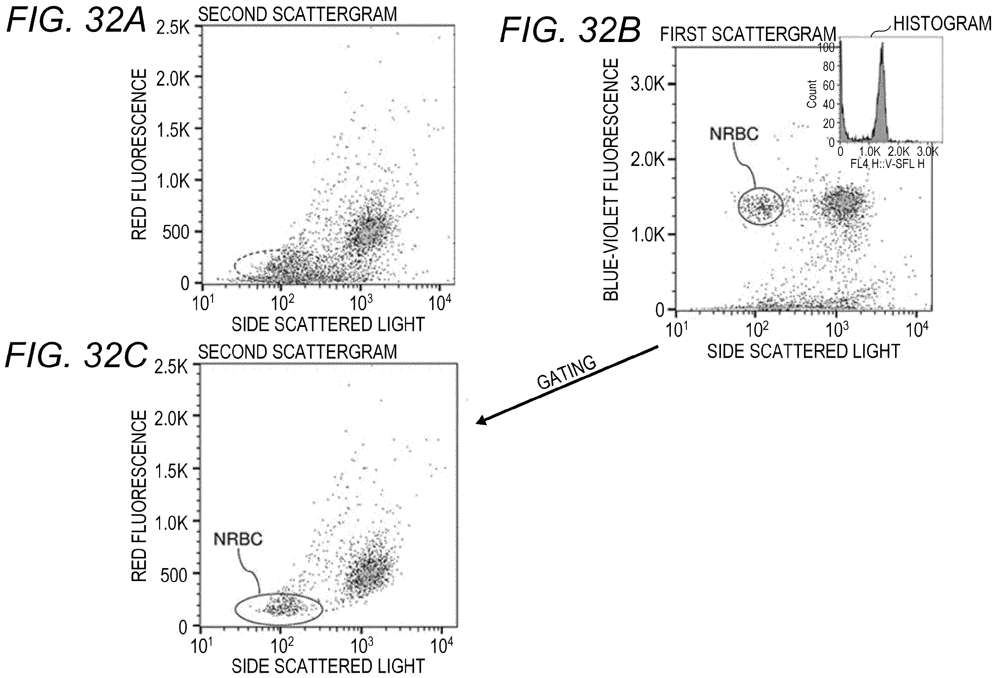

As a specimen containing NRBC, peripheral blood containing erythroblasts was used. Measurement samples prepared from this specimen were measured, and first and second scattergrams were created. The created scattergrams are shown in Fig. 32. In the second scattergram shown in A of Fig. 32, erythroblasts appeared in a region surrounded by a dashed ellipse, but overlapped with debris. Therefore, it was difficult to distinguish between erythroblasts and debris, and erythroblasts could not be detected and classified. On the other hand, in the first scattergram shown in B of Fig. 32, erythroblasts appeared in a region surrounded by a solid ellipse. However, since lymphocytes also appear in this region, it was difficult to detect and classify erythroblasts. On the other hand, debris appeared in a region where the blue-violet fluorescence intensity of the first scattergram was low. As can be seen from the first scattergram, a population of erythroblasts could be clearly distinguished from the population of debris by the blue-violet fluorescence intensity. Therefore, a region where the high blue-violet fluorescence intensity was high in the first scattergram was gated and plotted on the second scattergram. The created second scattergram is shown in C of Fig. 32. In the scattergram in C of Fig. 32, erythroblasts appeared in a region surrounded by a solid ellipse. As can be seen from this scattergram, since debris particles were excluded, it became possible to detect and classify erythroblasts. A scattergram similar to that in C of Fig. 32 was obtained also in a case where a particle group having a high blue-violet fluorescence intensity was gated in a histogram of the blue-violet fluorescence intensity, instead of the gating in the first scattergram, and plotted on the second scattergram. It was shown that in the scattergram in C of Fig. 32, a region where the red fluorescence intensity is lower than that of the lymphocyte population can be defined as a region where the NRBC cluster appears. It was shown that when a predetermined number or more of particles appear in this defined region, the particles can be detected or classified as NRBCs.

### Example 6: Distinction between immature NRBC/Megaloblast and DNA aneuploid cell (DNA aneuploidy)

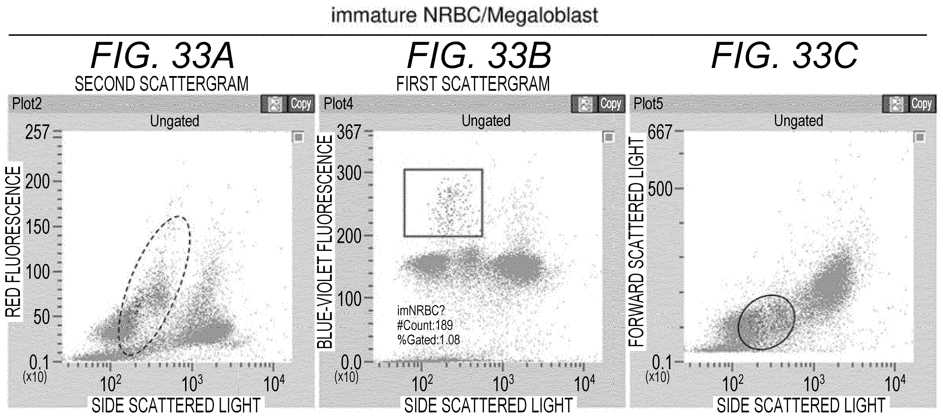

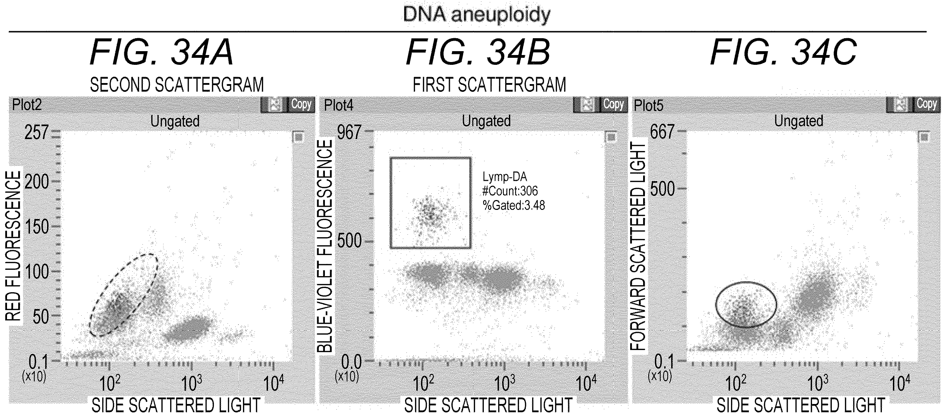

As a specimen containing immature NRBC/Megaloblast, peripheral blood containing erythroblasts (NRBC) was used. As a specimen containing DNA aneuploid cell, peripheral blood containing DNA aneuploid cells was used. A measurement sample prepared from each specimen was measured. In the measurement, in addition to blue-violet fluorescence intensity, red fluorescence intensity, and side scattered light intensity, intensity of forward scattered light signal was acquired. For each specimen, a scattergram was created based on the acquired information. The scattergrams for specimens containing immature NRBC/Megaloblast are shown in Fig. 33. The scattergrams for specimens containing DNA aneuploid cell are shown in Fig. 34. In Figs. 33 and 34, A is a second scattergram, B is a first scattergram, and C is a scattergram in which the side scattered light intensity is taken on a horizontal axis and the forward scattered light intensity is taken on a vertical axis.

In the second scattergram shown in A of Fig. 33, the immature NRBC/Megaloblast appeared in a region surrounded by a dashed ellipse, but overlapped with the lymphocyte population. Therefore, the immature NRBC/Megaloblast could not be detected and classified. In the first scattergram shown in B of Fig. 33, a large number of particles indicating a higher blue-violet fluorescence intensity than that of the lymphocyte population and having the same scattered light intensity as that of the lymphocyte or monocyte population appeared in a region surrounded by a solid square. This group of particles was immature NRBC/Megaloblast. The CV value of blue-violet fluorescence intensity was calculated for the particle population combining this particle group and normal white blood cells. Since the calculated CV value was equal to or greater than the threshold value, the immature NRBC/Megaloblast was a cluster separated from the lymphocyte population and the monocyte population in the first scattergram.

Also in the second scattergram shown in A of Fig. 34, as in A of Fig. 33, DNA aneuploid cells appeared in a region surrounded by a dashed ellipse, but overlapped with the lymphocyte population. Therefore, DNA aneuploid cell could not be detected and classified. Also in the first scattergram shown in B of Fig. 34, as in B of Fig. 33, a large number of particles indicating a higher blue-violet fluorescence intensity than that of the lymphocyte population and having the same scattered light intensity as that of lymphocytes appeared in a region surrounded by a solid square. This group of particles was DNA aneuploid cell. The CV value of blue-violet fluorescence intensity was calculated for a particle population combining this particle group and normal white blood cells. The calculated CV value was equal to or greater than the threshold value. Thus, DNA aneuploid cell was a cluster separated from the lymphocyte population. Comparing B of Fig. 33 with B of Fig. 34, it was difficult to distinguish between immature NRBC/Megaloblast and DNA DNA aneuploid cell by the first scattergram.

In the scattergram shown in C of Fig. 33, the immature NRBC/Megaloblast appeared in a region surrounded by a solid ellipse. As can be seen from this scattergram, the cluster of immature NRBC/Megaloblast overlapped with the lymphocyte population. That is, the forward scattered light intensity of immature NRBC/Megaloblast was almost the same as the forward scattered light intensity of lymphocytes. On the other hand, in the scattergram shown in C of Fig. 34, DNA aneuploid cell appeared in a region surrounded by a solid ellipse. As can be seen from this scattergram, the cluster of DNA aneuploid cellsappeared in a region where the forward scattered light intensity was higher than that of the lymphocyte population. A comparison between C of Fig. 33 and C of Fig. 34 showed that immature NRBC/Megaloblast and DNA aneuploid cell can be distinguished.

From Figs. 33 and 34, it was shown that in the first scattergram, when a predetermined number or more of particles appear in a region where the blue fluorescence intensity is higher than that of the lymphocyte population and the scattered light intensity is the same as that of the lymphocyte population or the monocyte population and the CV value of blue-violet fluorescence intensity of the particles is equal to or greater than the threshold value, the particles are suspected of being immature NRBC/Megaloblast or DNA aneuploid cell. Then, it was shown that when the forward scattered light intensity of the particles is less than the threshold value, the particles can be defined as immature NRBC/Megaloblast. It was shown that when the forward scattered light intensity of the particles is equal to or greater than the threshold value, the particles can be defined as DNA aneuploid cells.

### Example 7: Detection and classification of abnormal white blood cell groups

As abnormal white blood cells, four types of atypical lymphocytes, abnormal lymphocytes, myeloblasts, and lymphoblasts are mainly known. Abnormal lymphocytes include tumorous cells having no abnormality in the amount of DNA and DNA aneuploid cells. In Example 7, specimens containing various abnormal white blood cells were analyzed.

### (1) Analysis of specimen containing atypical lymphocytes (Aty-Lymph)

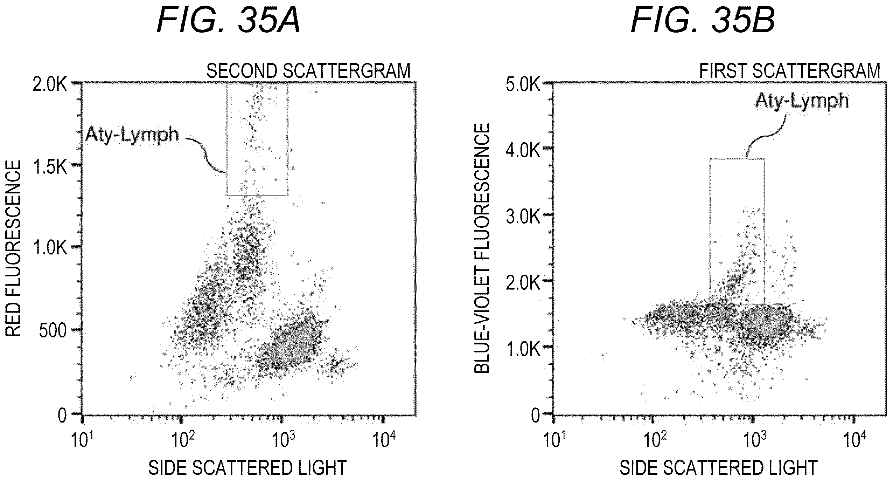

As a specimen containing Aty-Lymph, peripheral blood containing atypical lymphocytes was used. Measurement samples prepared from this specimen were measured, and first and second scattergrams were created. The created scattergrams are shown in Fig. 35. In the second scattergram shown in A of Fig. 35, normal white blood cells were classified into at least three subpopulations: lymphocytes, monocytes, and neutrophils. In A of Fig. 35, in the population classified as lymphocytes, particles having a red fluorescence intensity equal to or greater than the threshold value were hardly observed. Particles having a higher red fluorescence intensity than that of the neutrophil population and having the same scattered light intensity as that of the neutrophil population were hardly observed. However, a large number of particles having a higher red fluorescence intensity than that of the monocytes population and having the same scattered light intensity as that of the monocytes population appeared in a region surrounded by a solid square. Since this particle group was not observed in the normal specimen, the particle group appeared in the region was defined as an Aty-Lymph cluster.

Also in the first scattergram shown in B of Fig. 35, normal white blood cells were classified into at least three subpopulations: lymphocytes, monocytes, and neutrophils. In the first scattergram, particles having a blue-violet fluorescence intensity equal to or greater than the corresponding threshold value were hardly observed in the lymphocyte population. Particles having a higher blue-violet fluorescence intensity than that of the neutrophil population and having the same scattered light intensity as that of the neutrophil population were also hardly observed. However, a large number of particles having a higher blue-violet fluorescence intensity than that of the monocytes population and having the same scattered light intensity as that of the monocytes population appeared in a region surrounded by a solid square. The particle group appeared in the region was defined as an Aty-Lymph cluster. As described above, it was shown that in the first or second scattergram, a region where the fluorescence intensity is higher than that of the monocyte population and the scattered light intensity is the same as that of the monocyte population can be defined as a region where the Aty-Lymph cluster appears. It was shown that when a predetermined number or more of particles appear in this defined region, the particles can be detected or classified as Aty-Lymph.

### (2) Classification of specimens containing abnormal lymphocytes (Abn-Lymph)

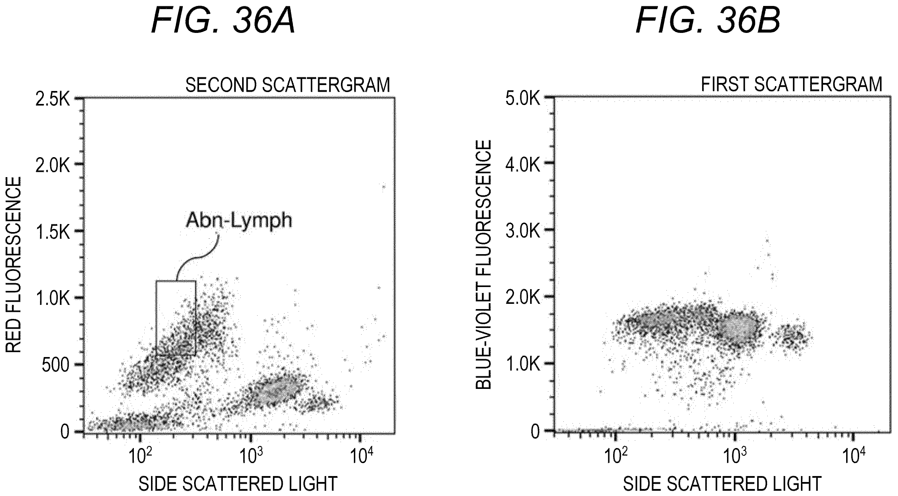

As a specimen containing Abn-Lymph, peripheral blood containing abnormal lymphocytes other than DNA aneuploid cells was used. Measurement samples prepared from this specimen were measured, and first and second scattergrams were created. The created scattergrams are shown in Fig. 36. In the second scattergram shown in A of Fig. 36, normal white blood cells were classified into at least four subpopulations: lymphocytes, monocytes, neutrophils, and eosinophils. In A of Fig. 36, in the population classified as lymphocytes, a large number of particles having a red fluorescence intensity equal to or greater than the threshold value appeared in a region surrounded by a solid square. In the first scattergram shown in B of Fig. 36, normal white blood cells were classified into at least four subpopulations: lymphocytes, monocytes, neutrophils, and eosinophils. In the first scattergram, particles having a higher red fluorescence intensity than that of the lymphocyte population were hardly observed. Therefore, it was suggested that almost no cells having an abnormal amount of DNA were present in the specimen. In the second scattergram, a particle group appeared in the region surrounded by a solid square, that is, a region where the red fluorescence intensity was equal to or greater than the threshold value and the scattered light intensity was the same as that of the lymphocyte population was defined as an Abn-Lymph cluster. It was shown that when a predetermined number or more of particles appear in this defined region, the particles can be detected or classified as Abn-Lymph.

### (3) Analysis of specimen containing DNA aneuploid cell

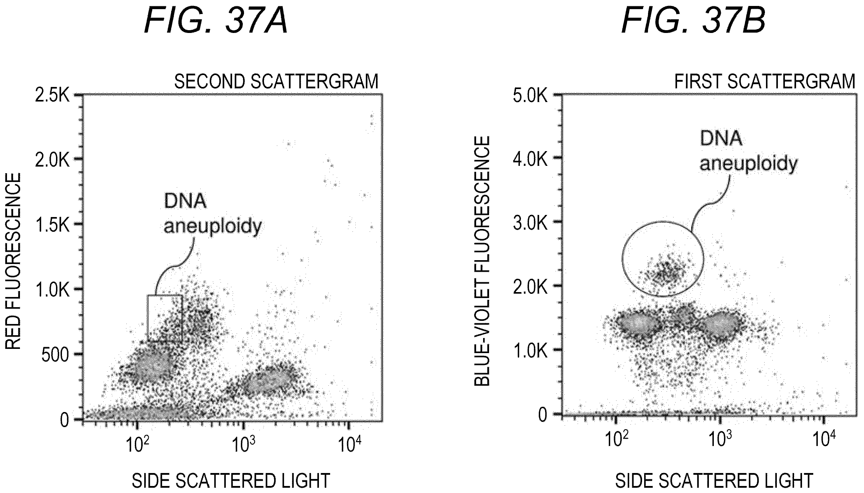

As a specimen containing DNA aneuploid cell, peripheral blood containing DNA aneuploid cells was used. Measurement samples prepared from this specimen were measured, and first and second scattergrams were created. The created scattergrams are shown in Fig. 37. In the second scattergram shown in A of Fig. 37, normal white blood cells were classified into at least three subpopulations: lymphocytes, monocytes, and neutrophils. In A of Fig. 37, in the population classified as lymphocytes, a large number of particles having a red fluorescence intensity equal to or greater than the threshold value appeared in a region surrounded by a solid square. Comparing A of Fig. 37 with A of Fig. 36, the distribution of the particles in the second scattergrams was substantially the same. Therefore, it was difficult to distinguish whether the abnormal white blood cell in the specimen is Abn-Lymph or DNA aneuploid cell by the second scattergram.

In the first scattergram shown in B of Fig. 37, normal white blood cells were classified into at least four subpopulations: lymphocytes, monocytes, neutrophils, and eosinophils. In the first scattergram, a large number of particles having a higher blue-violet fluorescence intensity than that of the lymphocyte population appeared in a region surrounded by a solid ellipse. This group of particles was shown to contain a greater amount of DNA than that of normal white blood cells. The CV value of blue-violet fluorescence intensity was calculated for the particle population combining this particle group and normal white blood cells. The calculated CV value was equal to or greater than the threshold value. Therefore, it was suggested that this particle group was discrete from each subpopulation of normal white blood cells. In fact, in B of Fig. 37, the particles appeared in the region surrounded by a solid ellipses were a cluster separated from the lymphocyte population and the monocyte population. Therefore, it was suggested that the particle group appeared in the region surrounded by a solid ellipses was DNA aneuploid cell.

As described above, in the second scattergram, a particle group appeared in a region where the red fluorescence intensity was equal to or greater than the threshold value and the scattered light intensity was the same as that of the lymphocyte population is suspected to be Abn-Lymph or DNA aneuploid cell. However, it was shown that in the first scattergram, when a predetermined number or more of particles appear in a region where the blue-violet fluorescence intensity is higher than that of the lymphocyte population, and the CV value of blue-violet fluorescence intensity of the particle group is equal to or greater than the threshold value, the particle group can be detected or classified as DNA aneuploid cell.

### (4) Analysis of specimen containing myeloblasts (M-Blast)

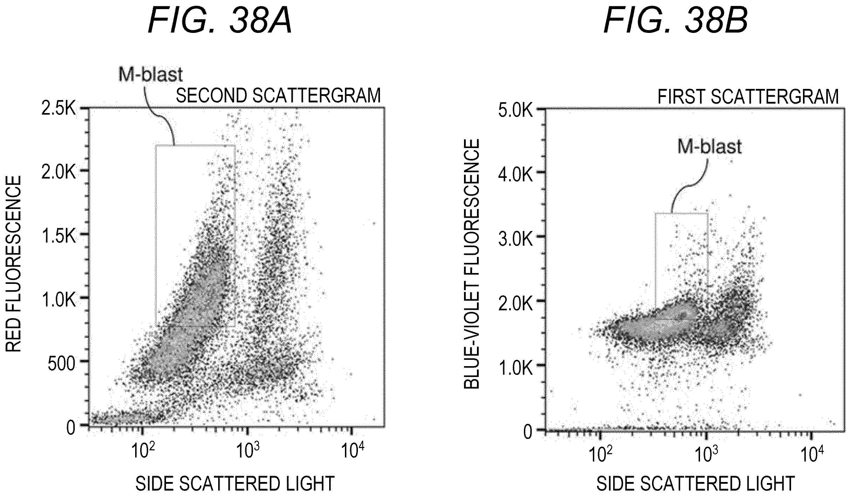

As a specimen containing M-Blast, peripheral blood containing myeloblasts was used. Measurement samples prepared from this specimen were measured, and first and second scattergrams were created. The created scattergrams are shown in Fig. 38. In the second scattergram shown in A of Fig. 38, the lymphocyte population and the monocyte population were overlapped, and a large population was observed. The neutrophil population and the eosinophil population were classified. A large number of particles having a higher red fluorescence intensity than that of the lymphocyte population were observed in a region surrounded by a solid square. The particles appeared in this region contained myeloblasts, but as can be seen from A of Fig. 38, the particle group of myeloblasts overlapped with the monocyte population.

In the first scattergram shown in B of Fig. 38, a large population in which the lymphocyte population and the monocyte population were overlapped was observed. The neutrophil population and the eosinophil population were classified. In the first scattergram, a large number of particles having a higher blue-violet fluorescence intensity than that of the lymphocyte population were observed in a region surrounded by a solid square. These particles were shown to contain a greater amount of DNA than that of normal white blood cells. The CV value of blue-violet fluorescence intensity was calculated for the particle population combining the particles, lymphocytes, and monocytes in the region surrounded by a solid square in the first scattergram. The calculated CV value was less than the threshold value. Therefore, it was suggested that the particles in the region surrounded by a solid square are a cluster continuous with the lymphocyte population and the monocyte population. This was as the distribution of the particles shown in the first scattergram. The CV value of side scattered light intensity was calculated for the lymphocyte population in the first scattergram. The calculated CV value was equal to or greater than the threshold value. From these, it was shown that particles in the region surrounded by a solid square, that is, a region where the blue fluorescence intensity is higher than that of the lymphocyte population and the side scattered light intensity is the same as that of the lymphocyte population and the monocyte population can be defined as myeloblasts.

### (5) Analysis of specimen containing lymphoblasts (L-Blast)

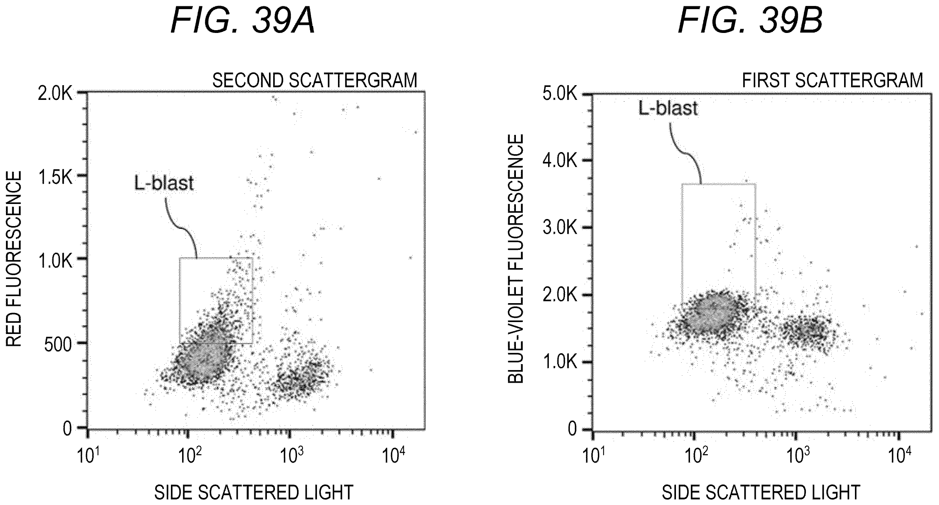

As a specimen containing L-Blast, peripheral blood containing lymphoblasts was used. Measurement samples prepared from this specimen were measured, and first and second scattergrams were created. The created scattergrams are shown in Fig. 39. In the second scattergram shown in A of Fig. 39, the lymphocyte population and the neutrophil population were classified. In the lymphocyte population, a large number of particles having a red fluorescence intensity equal to or greater than the threshold value appeared in a region surrounded by a solid square. The particles appeared in this region contained lymphoblasts, but as can be seen from A of Fig. 39, the particle group of lymphoblasts overlapped with the lymphocyte population.

In the first scattergram shown in B of Fig. 39, the lymphocyte population and the neutrophil population were classified. A large number of particles having a higher blue-violet fluorescence intensity than that of the lymphocyte population were observed in a region surrounded by a solid square. These particles were shown to contain a greater amount of DNA than that of normal white blood cells. The CV value of blue-violet fluorescence intensity was calculated for the particle population combining the particles and lymphocytes in the region surrounded by a solid square in the first scattergram. The calculated CV value was less than the threshold value. Therefore, it was suggested that the particles in the region surrounded by a solid square are a cluster continuous with the lymphocyte population. This was as the distribution of the particles shown in the first scattergram. The CV value of side scattered light intensity was calculated for the lymphocyte population in the first scattergram. The calculated CV value was less than the threshold value. From these, it was shown that particles in a region surrounded by a solid square, that is, a region where the blue fluorescence intensity is higher than the lymphocyte population and the side scattered light intensity is the same as the lymphocyte population can be defined as lymphoblasts.

### Reference Example 2: Acquisition of DNA index of DNA aneuploid cell

When DNA aneuploid cell is detected or classified by the method for analyzing a specimen of the present embodiment, a DNA index can be acquired using a histogram of fluorescence intensity from the first fluorescent dye. The DNA index is a ratio of the amount of DNA of DNA aneuploid cells to the amount of DNA of normal white blood cells. The DNA index may suggest the grade of lymphoma. Hereinafter, a method for acquiring a DNA index will be described.

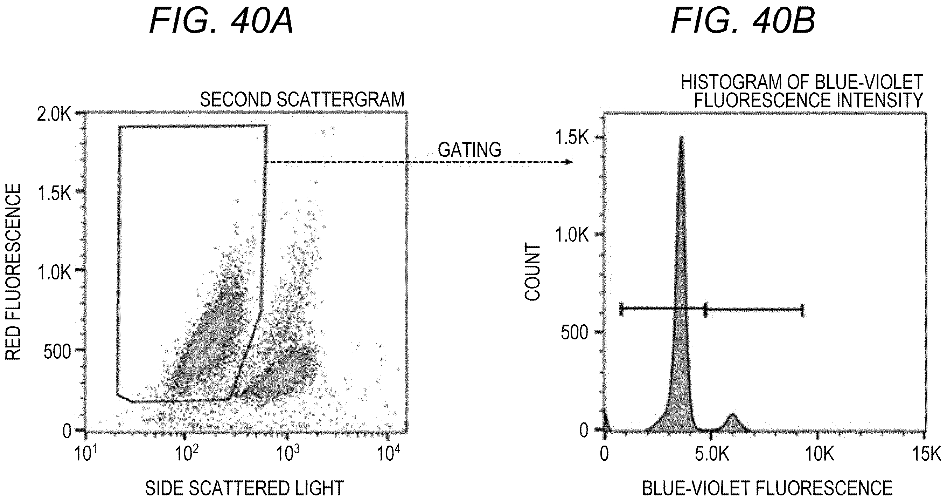

As a specimen containing DNA aneuploid cell, peripheral blood of a patient with mantle cell lymphoma was used. As in (3) of Example 7, a measurement sample prepared from the peripheral blood was measured, and a second scattergram was created. Referring to A of Fig. 40, in the second scattergram, a region where the monocyte population and the lymphocyte population had appeared (a region surrounded by a solid line) was gated. Particles in this region were analyzed with a histogram of blue-violet fluorescence intensity (hereinafter, also referred to as "V-SFL"). In the histogram in B of Fig. 40, two peaks were observed. The peak with lower V-SFL (left peak) was called "first peak", and the peak with higher V-SFL (right peak) was called "second peak". All particles constituting the first peak were defined as "V-SFL normal population", and all particles constituting the second peak were defined as "V-SFL high value population". The amount of DNA of normal white blood cells is substantially constant, and as can be seen from the first scattergram of the normal specimen, the range of the blue fluorescence intensity indicated by the normal white blood cells is also known. Therefore, in B of Fig. 40, it was suggested that the V-SFL normal population includes normal lymphocytes and monocytes, and it was suggested that the V-SFL high value population includes DNA aneuploid cells. Using mode as the representative value of V-SFL of each population, the DNA index was calculated by the following equation. In the example of Fig. 40, the DNA index was 1.67. (DNA index) = (Mode of V-SFL high value population)/(Mode of V-SFL normal population)

### Reference Example 3: Staining site of cell by fluorescent reagent

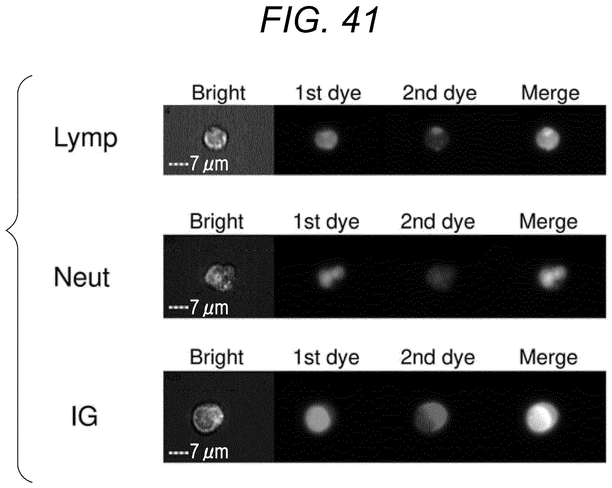

Using the hemolysis reagent and the staining reagent, a measurement sample was prepared from peripheral blood containing immature granulocytes. The cells in the measurement sample were imaged by an imaging flow cytometer, and staining sites in lymphocytes, neutrophils, and immature granulocytes were confirmed. The results are shown in Fig. 41. In the figure, "Bright" represents a bright field image," 1st dye" represents a fluorescence image by the first fluorescent dye, "2nd dye" represents a fluorescence image by the second fluorescent dye, and "Merge" represents superposition of the fluorescence image by the first fluorescent dye and the fluorescence image by the second fluorescent dye. As shown in Fig. 41, it was found that immature granulocytes (IG) were more strongly stained with the first fluorescent dye and the second fluorescent dye than lymphocytes (Lymp) and neutrophils (Neut). This suggests that immature granulocytes have higher amounts of DNA and RNA than lymphocytes and neutrophils.

### Reference Example 4: Acquisition of DNA abnormality (%)

A total of 16 specimens including normal specimens and DNA aneuploid cells were analyzed by the method for analyzing a specimen of the present embodiment and a standard measurement method to acquire DNA abnormality (%). The standard measurement method was a measurement method by The Standardization Committee of the Japan Cytometry Society described in Takamoto S. et al., Guidelines for flow cytometric analysis of DNA aneuploidy, Cytometry Research, vol. 19, no. 1, pp. 1-9, 2009. In the standard measurement method, a stain solution containing propidium iodide (PI) as the first fluorescent dye was used. Composition of the stain solution was 50 µg/mL PI, 0.1% Triton (trademark) X-100, 0.1% disodium citrate trihydrate, and 1 mg/mL RNase.

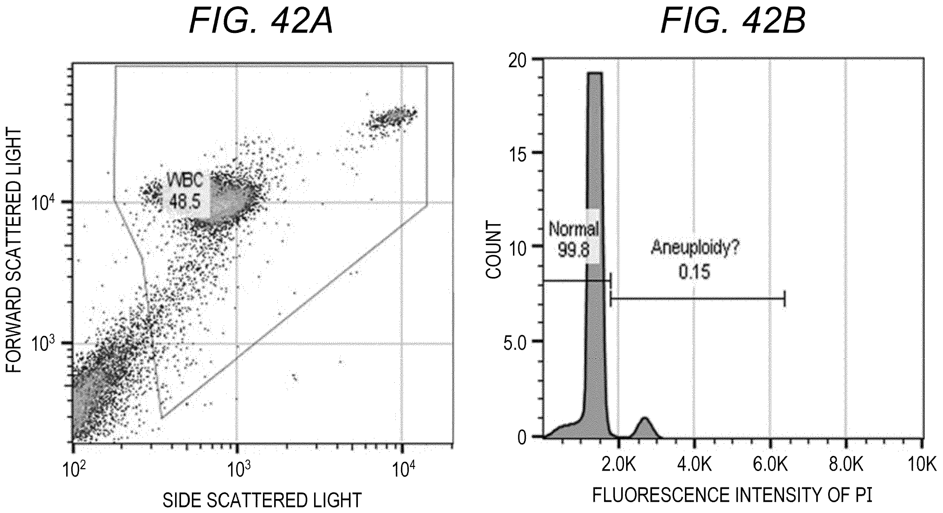

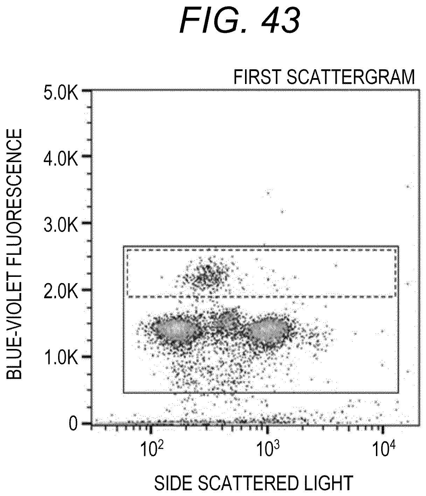

In the standard measurement method, a stain solution (1 mL) was mixed with whole blood (50 µL), and the mixture was allowed to stand for 30 minutes or more to obtain a measurement sample. The obtained measurement sample was measured with the above analyzer. A scattergram in which the side scattered light intensity is taken on a horizontal axis and the forward scattered light intensity is taken on a vertical axis was created by Flowjo (trademark). Referring to A of Fig. 42, on this scattergram, a region not containing debris and containing white blood cells (a region surrounded by a solid line) was gated, a histogram of the fluorescence intensity of PI for white blood cells was created, and white blood cells were counted. Referring to B of Fig. 42, a population showing a high fluorescence intensity was gated and counted on this histogram. In the method for analyzing a specimen of the present embodiment, a measurement sample prepared from each specimen was measured, and a first scattergram was created. Referring to Fig. 43, on the first scattergram, a region not containing debris and containing white blood cells (a region surrounded by a solid line) was gated, and particles in the region were counted. Among the particles in this region, a population having a high blue fluorescence intensity (a region surrounded by a dashed line) was gated and counted. Using the number of cells counted by the standard measurement method and the method for analyzing a specimen of the present embodiment, the DNA abnormality (%) of each specimen was calculated by the following equation. (DNA abnormality) = {(Number of cells showing high fluorescence intensity of first fluorescent dye)/(Number of white blood cells)} × 100

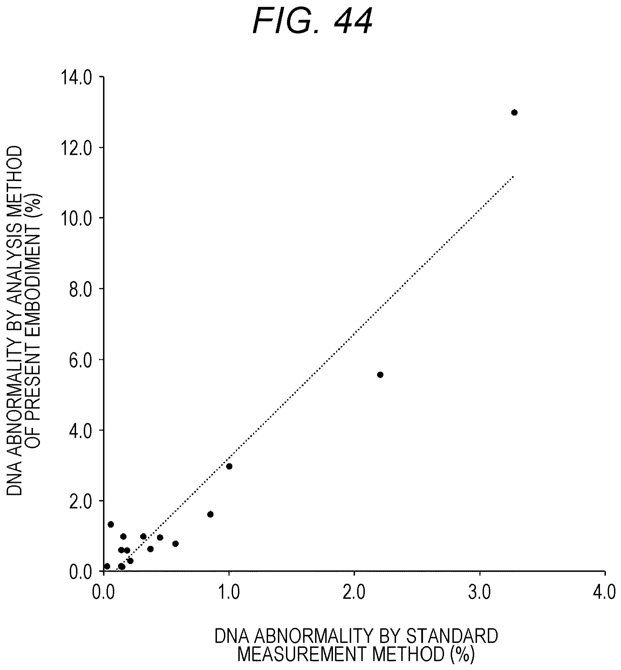

The values of DNA abnormalities (%) obtained by the standard measurement method and the method for analyzing a specimen of the present embodiment were plotted to obtain a regression line (y = 3.5122x - 0.3086). Correlation coefficient (r) was 0.961, and determination coefficient (r²) was 0.9241. Referring to Fig. 44, it was shown that the method for analyzing a specimen of the present embodiment has a high correlation with the standard measurement method.

### Example 8: Analysis of specimen containing infected red blood cells (iRBC)

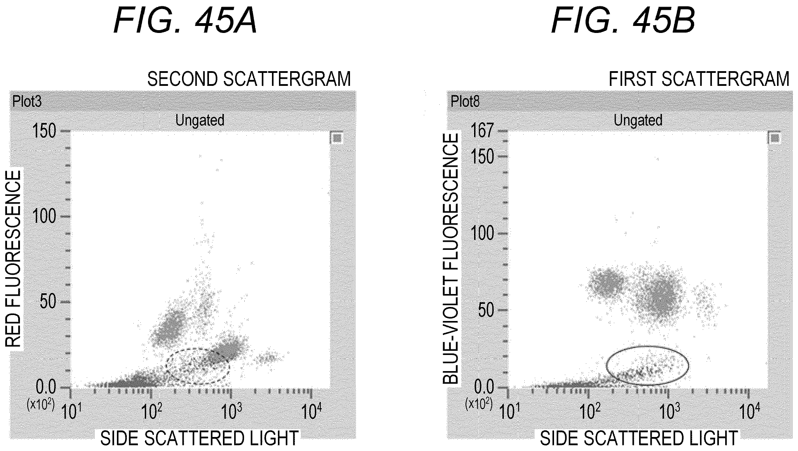

As a specimen containing iRBC, cultured malaria added human blood was used. Measurement samples prepared from this specimen were measured, and first and second scattergrams were created. The created scattergrams are shown in Fig. 45. In the second scattergram shown in A of Fig. 45, particles having a higher red fluorescence intensity and side scattered light intensity than those of a population of debris appeared in a region surrounded by a dashed ellipse. The particles appeared in this region contained iRBC, but as can be seen from A of Fig. 45, the particle group of iRBC overlapped with the neutrophil population. In the first scattergram shown in B of Fig. 45, a particle group of iRBC appeared in a region surrounded by a solid ellipse. Unlike the second scattergram, the particle group of iRBC in the first scattergram did not overlap with the subpopulation of white blood cells. Therefore, it was shown that in the first scattergram, a particle group appeared in a region where the blue fluorescence intensity and the side scattered light intensity are higher than those of the population of debris can be defined as an iRBC cluster. The distinction between iRBC and PLT-clumps is as described in Example 3.

### Example 9: Analysis of normal specimen of non-blood sample

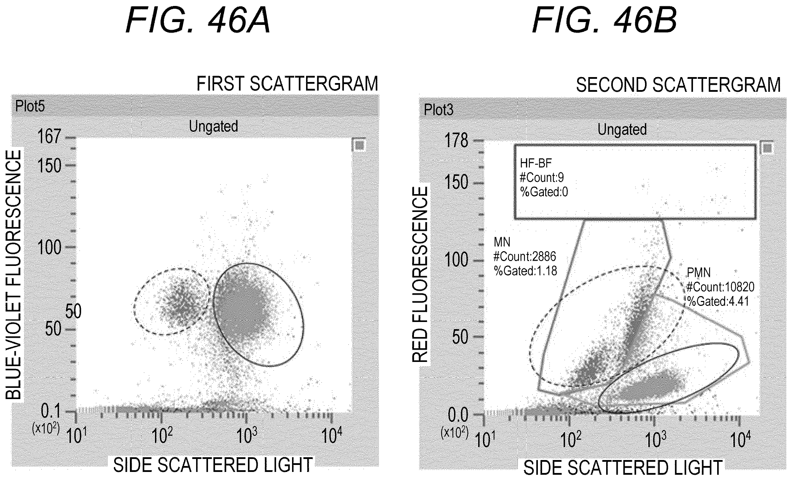

As a normal specimen of a non-blood sample, a pleural effusion known not to contain abnormal cells was used. Measurement samples prepared from this specimen were measured, and first and second scattergrams were created. The created scattergrams are shown in Fig. 46. In the first scattergram shown in A of Fig. 46, a population of mononuclear cells (monocytes and lymphocytes) appeared in a region surrounded by a dashed ellipse, and a population of polymorphonuclear cells (neutrophils, eosinophils and basophils) appeared in a region surrounded by a solid line ellipse. These populations were distant from the population of debris that appeared in a region where the blue fluorescence intensity was low. Therefore, from the first scattergram, it was shown that nucleated cells and debris are distinguished, so that only nucleated cells can be gated. It was shown that a population of nucleated cells appeared in a region where the side scattered light intensity is low can be defined as mononuclear cells, and a population of nucleated cells appeared in a region where the side scattered light intensity is high can be defined as polymorphonuclear cells. In the second scattergram shown in B of Fig. 46, the monocyte population and the lymphocyte population appeared in a region surrounded by a dashed ellipse, and the population of polymorphonuclear cells appeared in a region surrounded by a solid ellipse.

### Example 10: Analysis of specimen containing tissue-derived cells

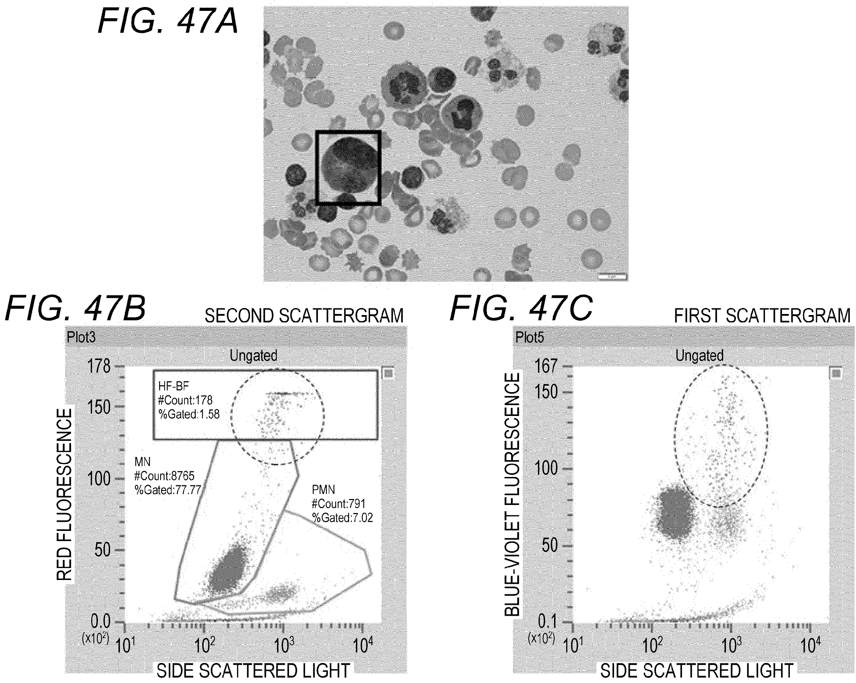

As a specimen containing tissue-derived cells, a pleural effusion confirmed to contain mesothelial cells was used. When this specimen was stained with May-Giemsa and observed with a microscope, a mesothelial cell was observed in a region surrounded by a solid square as shown in a microscopic image in A of Fig. 47. Measurement samples prepared from this specimen were measured, and first and second scattergrams were created. The created scattergrams are shown in Fig. 47. In both the second scattergram shown in B of Fig. 47 and the first scattergram shown in C, mesothelial cell particles appeared in a region surrounded by a dashed ellipse. Therefore, it was shown that in the first scattergram or the second scattergram, a particle group appeared in a region where the fluorescence intensity is high can be defined as a tissue-derived cell cluster.

### Example 11: Analysis of specimen containing bacteria or fungi

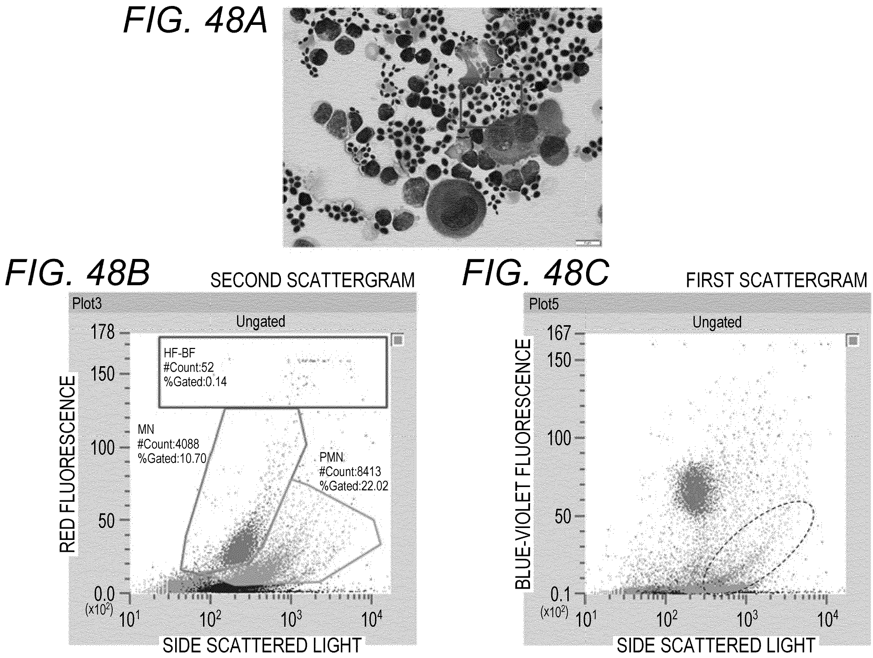

As a specimen containing bacteria or fungi, ascites confirmed to contain fungi was used. When this specimen was stained with May-Giemsa and observed with a microscope, fungi were observed in a region surrounded by a solid square as shown in a microscopic image in A of Fig. 48. Measurement samples prepared from this specimen were measured, and first and second scattergrams were created. In the second scattergram shown in B of Fig. 48, a particle group of fungi was overlapped with the neutrophil population and could not be detected. On the other hand, in the first scattergram shown in C of Fig. 48, a particle group of fungi appeared in a region surrounded by a dashed ellipse. Therefore, it was shown that in the first scattergram, a particle group appeared in a region where the blue-violet fluorescence intensity is higher than that of the population of debris and the blue fluorescence intensity is lower than that of white blood cells can be defined as a cluster of bacteria or fungi.

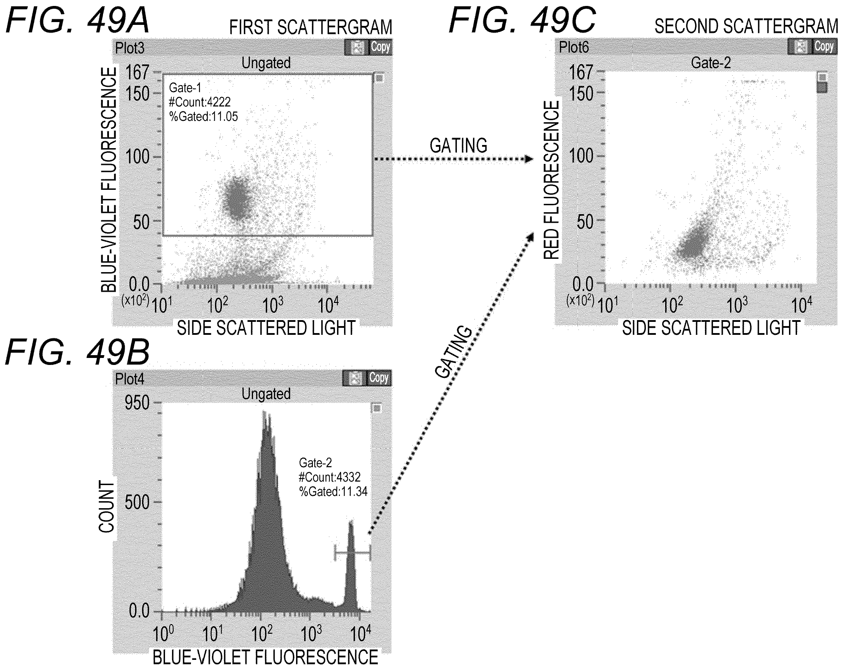

Even when the cluster of bacteria or fungi was detected in the specimen, white blood cells could be detected and classified by excluding them. As shown in A of Fig. 49, in a first scattergram of the specimen containing fungi, by gating a region containing white blood cells and having a high blue fluorescence intensity, the population of debris and the cluster of fungi were excluded. As shown in B of Fig. 49, in a histogram of the blue fluorescence intensity, also by gating the population having a high blue fluorescence intensity, the population of debris and the cluster of fungi could be excluded. By plotting the gated particle groups on a second scattergram, the population of white blood cells could be detected and classified as shown in C of Fig. 49.

## Claims

1. A method for analyzing a specimen, comprising:
acquiring first fluorescence information, second fluorescence information, and scattered light information generated by irradiating a particle in a measurement sample stained with a first fluorescent dye and a second fluorescent dye with light;
specifying a particle having a first characteristic based on the first fluorescence information, the second fluorescence information, and the scattered light information; and
outputting information indicating presence or absence of a first abnormal cell, information indicating presence or absence of a second abnormal cell, and information indicating presence or absence of a third abnormal cell based on information of the particle having a first characteristic,
wherein the measurement sample is prepared by mixing a specimen collected from a subject, the first fluorescent dye, and the second fluorescent dye,
the first fluorescence information is fluorescence intensity from the first fluorescent dye, and the second fluorescence information is fluorescence intensity from the second fluorescent dye, and
the first fluorescent dye is a fluorescent dye that specifically binds to DNA, and the second fluorescent dye is a fluorescent dye that specifically binds to RNA.

2. The analysis method according to claim 1, wherein the specimen is a blood sample.

3. The analysis method according to claim 2, wherein in the acquiring, side scattered light information and forward scattered light information are acquired as the scattered light information.

4. The analysis method according to claim 3, wherein
in the specifying, each subpopulation of white blood cells comprising a first lymphocyte population is specified from particles in the measurement sample based on the side scattered light information and the first fluorescence information, and
each subpopulation of white blood cells comprising a second lymphocyte population is specified from particles in the measurement sample based on the side scattered light information and the second fluorescence information.

5. The analysis method according to claim 4, wherein
in the specifying, when the second lymphocyte population comprises a particle indicating second fluorescence information equal to or greater than a first threshold value corresponding to the second fluorescence information, the particle having a first characteristic is specified based on the first fluorescence information, and
the particle having a first characteristic is a particle indicating the first fluorescence information higher than that of the first lymphocyte population.

6. The analysis method according to claim 5, wherein information of the particle having a first characteristic comprises a number of particle, information on dispersion of first fluorescence information, and forward scattered light information.

7. The analysis method according to claim 6, wherein
in the outputting, when the particle having a first characteristic satisfies following conditions 1) to 3):
1) a number of the particle having a first characteristic is equal to or greater than a corresponding threshold value;
2) the information on dispersion of first fluorescence information of the particle having a first characteristic is equal to or greater than a corresponding threshold value; and
3) the forward scattered light information of the particle having a first characteristic is equal to or greater than a corresponding threshold value,
the particle having a first characteristic is specified as the first abnormal cell, and
information indicating that a DNA aneuploid cell is present is output as the information indicating presence or absence of a first abnormal cell.

8. The analysis method according to claim 7, wherein the information on dispersion of first fluorescence information is a CV value of first fluorescence intensity, and the forward scattered light information is forward scattered light intensity.

9. The analysis method according to claim 7, further comprising: calculating a DNA index of the DNA aneuploid cell; and outputting the DNA index.

10. The analysis method according to claim 6, wherein
in the outputting, when the information of the particle having a first characteristic satisfies following conditions 1) and 4):
1) a number of the particle having a first characteristic is equal to or greater than a corresponding threshold value; and
4) the information on dispersion of first fluorescence information of the particle having a first characteristic is less than a corresponding threshold value,
the particle having a first characteristic is specified as the second abnormal cell, and
information indicating that a blast is present is output as the information indicating presence or absence of a second abnormal cell.

11. The analysis method according to claim 10, wherein the information on dispersion of first fluorescence information is a CV value of first fluorescence intensity.

12. The analysis method according to claim 6, wherein
in the outputting, when a number of the particle having a first characteristic is less than a corresponding threshold value, the particle having a first characteristic is specified as the third abnormal cell, and
information indicating that an abnormal lymphocyte is present is output as the information indicating presence or absence of a third abnormal cell.

13. The analysis method according to claim 6, wherein
in the outputting, when information indicating presence or absence of a fourth abnormal cell is further output, and
the information of the particle having a first characteristic satisfies following conditions 1), 2), and 5):
1) a number of the particle having a first characteristic is equal to or greater than a corresponding threshold value;
2) the information on dispersion of first fluorescence information of the particle having a first characteristic is equal to or greater than a corresponding threshold; and
5) the forward scattered light information of the particle having a first characteristic is less than a corresponding threshold value,
the particle having a first characteristic is specified as the fourth abnormal cell, and
information indicating that an immature erythroblast or a megaloblast is present is output as the information indicating presence or absence of a fourth abnormal cell.

14. The analysis method according to claim 13, wherein the information on dispersion of first fluorescence information is a CV value of first fluorescence intensity, and the forward scattered light information is forward scattered light intensity.

15. The analysis method according to claim 10, wherein
the blast is a lymphoblast or a myeloblast, and
information indicating that a lymphoblast or a myeloblast is present is output as the information indicating presence or absence of a second abnormal cell based on the information of the particle having a first characteristic and the information of the first lymphocyte population.

16. The analysis method according to claim 15, wherein
in the outputting,
when the particle having a first characteristic satisfies the conditions 1) and 4) and information on dispersion of side scattered light information of the first lymphocyte population is less than a corresponding threshold value, information indicating that a lymphoblast is present is output as the information indicating presence or absence of a second abnormal cell, and
when the particle having a first characteristic satisfies the conditions 1) and 4) and the information on dispersion of side scattered light information of the first lymphocyte population is equal to or greater than the corresponding threshold value, information indicating that a myeloblast is present is output as the information indicating presence or absence of a second abnormal cell.

17. The analysis method according to claim 16, wherein the information on dispersion of side scattered light information is a CV value of side scattered light intensity.

## Patentansprüche

1. Verfahren zur Analyse einer Probe, umfassend:
Erfassen von ersten Fluoreszenzinformationen, zweiten Fluoreszenzinformationen und Streulichtinformationen, die durch Bestrahlung eines Partikels in einer mit einem ersten Fluoreszenzfarbstoff und einem zweiten Fluoreszenzfarbstoff gefärbten Messprobe mit Licht erzeugt werden;
Spezifizieren eines Partikels mit einer ersten Charakteristik auf Grundlage der ersten Fluoreszenzinformation, der zweiten Fluoreszenzinformation und der Streulichtinformation; und
Ausgeben von Informationen, die das Vorhandensein oder die Abwesenheit einer ersten abnormalen Zelle anzeigen, von Informationen, die das Vorhandensein oder die Abwesenheit einer zweiten abnormalen Zelle anzeigen, und von Informationen, die das Vorhandensein oder die Abwesenheit einer dritten abnormalen Zelle anzeigen, auf Grundlage von Informationen über das Partikel mit einer ersten Eigenschaft,
wobei die Messprobe durch Mischen einer von einer Person entnommenen Probe, des ersten Fluoreszenzfarbstoffs und des zweiten Fluoreszenzfarbstoffs hergestellt wird,
die erste Fluoreszenzinformation die Fluoreszenzintensität des ersten Fluoreszenzfarbstoffs ist und die zweite Fluoreszenzinformation die Fluoreszenzintensität des zweiten Fluoreszenzfarbstoffs ist, und
der erste Fluoreszenzfarbstoff ein Fluoreszenzfarbstoff ist, der spezifisch an DNA bindet, und der zweite Fluoreszenzfarbstoff ein Fluoreszenzfarbstoff ist, der spezifisch an RNA bindet.

2. Analyseverfahren nach Anspruch 1, wobei die Probe eine Blutprobe ist.

3. Analyseverfahren nach Anspruch 2, wobei bei der Erfassung seitliche Streulichtinformationen und Vorwärtsstreulichtinformationen als die Streulichtinformationen erfasst werden.

4. Analyseverfahren nach Anspruch 3, wobei
bei der Spezifizierung jede Subpopulation weißer Blutzellen, die eine erste Lymphozytenpopulation umfasst, aus Partikeln in der Messprobe auf Grundlage der seitlichen Streulichtinformation und der ersten Fluoreszenzinformation spezifiziert wird, und
jede Subpopulation weißer Blutzellen, die eine zweite Lymphozytenpopulation umfasst, aus Partikeln in der Messprobe auf Grundlage der seitlichen Streulichtinformationen und der zweiten Fluoreszenzinformationen spezifiziert wird.

5. Analyseverfahren nach Anspruch 4, wobei
bei der Spezifizierung, wenn die zweite Lymphozytenpopulation ein Partikel umfasst, das eine zweite Fluoreszenzinformation anzeigt, die gleich oder größer als ein erster Schwellenwert ist, der der zweiten Fluoreszenzinformation entspricht, der Partikel mit einer ersten Charakteristik auf Grundlage der ersten Fluoreszenzinformation spezifiziert wird, und
das Partikel mit einer ersten Charakteristik ein Partikel ist, das die erste Fluoreszenzinformation höher als die der ersten Lymphozytenpopulation anzeigt.

6. Analyseverfahren nach Anspruch 5, wobei die Information des Partikels mit einer ersten Charakteristik eine Partikelanzahl, Information über die Verteilung der ersten Fluoreszenzinformation und Vorwärtsstreulichtinformation umfasst.

7. Analyseverfahren nach Anspruch 6, wobei
bei der Ausgabe, wenn der Partikel mit einer ersten Charakteristik die folgenden Bedingungen 1) bis 3) erfüllt:
1) eine Anzahl der Partikel mit einer ersten Charakteristik gleich oder größer als ein entsprechender Schwellenwert ist;
2) die Information über die Verteilung der ersten Fluoreszenzinformation des Partikels mit einer ersten Charakteristik gleich oder größer als ein entsprechender Schwellenwert ist; und
3) die Vorwärtsstreulichtinformation des Partikels mit einer ersten Charakteristik gleich oder größer als ein entsprechender Schwellenwert ist,
der Partikel mit einer ersten Charakteristik als erste abnorme Zelle spezifiziert wird, und
die Information, die anzeigt, dass eine aneuploide DNA-Zelle vorhanden ist, als die Information ausgegeben wird, die das Vorhandensein oder Nichtvorhandensein einer ersten abnormalen Zelle anzeigt.

8. Analyseverfahren nach Anspruch 7, wobei die Information über die Verteilung der ersten Fluoreszenzinformation ein CV-Wert der ersten Fluoreszenzintensität ist und die Vorwärtsstreulichtinformation die Intensität des vorwärts gestreuten Lichts ist.

9. Analyseverfahren nach Anspruch 7, weiter umfassend: Berechnen eines DNA-Indexes der aneuploiden DNA-Zelle; und Ausgeben des DNA-Indexes.

10. Analyseverfahren nach Anspruch 6, wobei
bei der Ausgabe, wenn die Information des Partikels mit einer ersten Charakteristik die folgenden Bedingungen 1) und 4) erfüllt:
1) eine Anzahl der Partikel mit einer ersten Charakteristik gleich oder größer als ein entsprechender Schwellenwert ist; und
4) die Information über die Verteilung der ersten Fluoreszenzinformation des Partikels mit einer ersten Charakteristik kleiner als ein entsprechender Schwellenwert ist,
der Partikel mit einer ersten Charakteristik als zweite anormale Zelle spezifiziert wird, und
die Information, dass ein Blast vorhanden ist, als Information über das Vorhandensein oder Nichtvorhandensein einer zweiten anormalen Zelle ausgegeben wird.

11. Analyseverfahren nach Anspruch 10, wobei die Information über die Verteilung der ersten Fluoreszenzinformation ein CV-Wert der ersten Fluoreszenzintensität ist.

12. Analyseverfahren nach Anspruch 6, wobei
bei der Ausgabe, wenn eine Anzahl des Partikels mit einer ersten Charakteristik kleiner als ein entsprechender Schwellenwert ist, der Partikel mit einer ersten Charakteristik als die dritte abnormale Zelle spezifiziert wird, und
die Information, dass ein anormaler Lymphozyt vorhanden ist, als die Information ausgegeben wird, die das Vorhandensein oder Nichtvorhandensein einer dritten anormalen Zelle anzeigt.

13. Analyseverfahren nach Anspruch 6, wobei
bei der Ausgabe, wenn Informationen, die das Vorhandensein oder Nichtvorhandensein einer vierten abnormalen Zelle anzeigen, weiter ausgegeben werden, und
die Information des Partikels mit einer ersten Charakteristik die folgenden Bedingungen 1), 2) und 5) erfüllt:
1) eine Anzahl der Partikel mit einer ersten Charakteristik gleich oder größer als ein entsprechender Schwellenwert ist;
2) die Information über die Verteilung der ersten Fluoreszenzinformation des Partikels mit einer ersten Charakteristik gleich oder größer als ein entsprechender Schwellenwert ist; und
5) die Vorwärtsstreulichtinformation des Partikels mit einer ersten Charakteristik kleiner ist als ein entsprechender Schwellenwert,
der Partikel mit einer ersten Charakteristik als vierte abnorme Zelle spezifiziert wird, und
die Information, dass ein unreifer Erythroblast oder ein Megaloblast vorhanden ist, als Information über das Vorhandensein oder Nichtvorhandensein einer vierten abnormen Zelle ausgegeben wird.

14. Analyseverfahren nach Anspruch 13, wobei die Information über die Verteilung der ersten Fluoreszenzinformation ein CV-Wert der ersten Fluoreszenzintensität ist und die Vorwärtsstreulichtinformation die Intensität des vorwärts gestreuten Lichts ist.

15. Analyseverfahren nach Anspruch 10, wobei
der Blast ein Lymphoblast oder ein Myeloblast ist, und
die Information, die anzeigt, dass ein Lymphoblast oder ein Myeloblast vorhanden ist, als die Information ausgegeben wird, die das Vorhandensein oder Nichtvorhandensein einer zweiten abnormalen Zelle anzeigt, auf Grundlage der Information des Partikels mit einer ersten Charakteristik und der Information der ersten Lymphozytenpopulation.

16. Analyseverfahren nach Anspruch 15, wobei
bei der Ausgabe,
wenn der Partikel mit einer ersten Charakteristik die Bedingungen 1) und 4) erfüllt und die Information über die Streuung der seitlichen Streulichtinformation der ersten Lymphozytenpopulation kleiner als ein entsprechender Schwellenwert ist, die Information, die anzeigt, dass ein Lymphoblast vorhanden ist, als die Information ausgegeben wird, die das Vorhandensein oder Nichtvorhandensein einer zweiten abnormalen Zelle anzeigt, und
wenn der Partikel mit einer ersten Charakteristik die Bedingungen 1) und 4) erfüllt und die Information über die Verteilung der seitlichen Streulichtinformation der ersten Lymphozytenpopulation gleich oder größer als der entsprechende Schwellenwert ist, wobei die Information, die das Vorhandensein eines Myeloblasten anzeigt, als die Information ausgegeben, die das Vorhandensein oder Nichtvorhandensein einer zweiten abnormalen Zelle anzeigt.

17. Analyseverfahren nach Anspruch 16, wobei die Information über die Verteilung der seitlichen Streulichtinformation ein CV-Wert der Intensität des seitlich gestreuten Lichts ist.

## Revendications

1. Procédé d'analyse d'un échantillon, comprenant :
acquérir des premières informations de fluorescence, des secondes informations de fluorescence, et des informations de lumière dispersée générées en irradiant de lumière une particule dans un échantillon de mesure coloré avec un premier colorant fluorescent et un second colorant fluorescent ;
spécifier une particule présentant une première caractéristique sur la base des premières informations de fluorescence, des secondes informations de fluorescence, et des informations de lumière dispersée ; et
délivrer en sortie des informations indiquant la présence ou l'absence d'une première cellule anormale, des informations indiquant la présence ou l'absence d'une deuxième cellule anormale, et des informations indiquant la présence ou l'absence d'une troisième cellule anormale sur la base des informations de la particule présentant une première caractéristique,
dans lequel l'échantillon de mesure est préparé en mélangeant un échantillon collecté à partir d'un sujet, le premier colorant fluorescent, et le second colorant fluorescent,
la première information de fluorescence est l'intensité de fluorescence en provenance du premier colorant fluorescent, et la seconde information de fluorescence est l'intensité de fluorescence en provenance du second colorant fluorescent, et
le premier colorant fluorescent est un colorant fluorescent qui se lie spécifiquement à l'ADN, et le second colorant fluorescent est un colorant fluorescent qui se lie spécifiquement à l'ARN.

2. Procédé d'analyse selon la revendication 1, dans lequel l'échantillon est un échantillon de sang.

3. Procédé d'analyse selon la revendication 2, dans lequel lors de l'acquisition, des informations de lumière dispersée latéralement et des informations de lumière dispersée vers l'avant sont acquises en tant qu'informations de lumière dispersée.

4. Procédé d'analyse selon la revendication 3, dans lequel
lors de la spécification, chaque sous-population de globules blancs comprenant une première population de lymphocytes est spécifiée à partir de particules dans l'échantillon de mesure sur la base des informations de lumière dispersée latéralement et des premières informations de fluorescence, et
chaque sous-population de globules blancs comprenant une seconde population de lymphocytes est spécifiée à partir de particules dans l'échantillon de mesure sur la base des informations de lumière dispersée latéralement et des secondes informations de fluorescence.

5. Procédé d'analyse selon la revendication 4, dans lequel
lors de la spécification, lorsque la seconde population de lymphocytes comprend une particule indiquant des secondes informations de fluorescence supérieures ou égales à une première valeur de seuil correspondant aux secondes informations de fluorescence, la particule présentant une première caractéristique est spécifiée sur la base des premières informations de fluorescence, et
la particule présentant une première caractéristique est une particule indiquant des premières informations de fluorescence supérieures à celles de la première population de lymphocytes.

6. Procédé d'analyse selon la revendication 5, dans lequel les informations de la particule présentant une première caractéristique comprennent un nombre de particules, des informations sur la dispersion des premières informations de fluorescence, et des informations de lumière dispersée vers l'avant.

7. Procédé d'analyse selon la revendication 6, dans lequel
lors de la délivrance en sortie, lorsque la particule présentant une première caractéristique satisfait les conditions suivantes 1) à 3) :
1) un nombre de particules présentant une première caractéristique est supérieur ou égal à une valeur de seuil correspondante ;
2) les informations sur la dispersion des premières informations de fluorescence de la particule présentant une première caractéristique sont supérieures ou égales à une valeur de seuil correspondante ; et
3) les informations de lumière dispersée vers l'avant de la particule présentant une première caractéristique sont supérieures ou égales à une valeur de seuil correspondante,
la particule présentant une première caractéristique est spécifiée en tant que première cellule anormale, et
des informations indiquant qu'une cellule aneuploïde d'ADN est présente sont délivrées en sortie en tant qu'informations indiquant la présence ou l'absence d'une première cellule anormale.

8. Procédé d'analyse selon la revendication 7, dans lequel l'information sur la dispersion des premières informations de fluorescence est une valeur CV de la première intensité de fluorescence, et l'information de lumière dispersée vers l'avant est l'intensité de lumière dispersée vers l'avant.

9. Procédé d'analyse selon la revendication 7, comprenant en outre : calculer un indice d'ADN de la cellule aneuploïde d'ADN ; et délivrer en sortie l'indice d'ADN.

10. Procédé d'analyse selon la revendication 6, dans lequel
lors de la délivrance en sortie, lorsque l'information de la particule présentant une première caractéristique satisfait les conditions suivantes 1) et 4) :
1) un nombre de particules présentant une première caractéristique est supérieur ou égal à une valeur de seuil correspondante ; et
4) les informations sur la dispersion des premières informations de fluorescence de la particule présentant une première caractéristique sont inférieures à une valeur de seuil correspondante,
la particule présentant une première caractéristique est spécifiée en tant que deuxième cellule anormale, et
des informations indiquant qu'un blaste est présent sont délivrées en sortie en tant qu'informations indiquant la présence ou l'absence d'une deuxième cellule anormale.

11. Procédé d'analyse selon la revendication 10, dans lequel l'information sur la dispersion des premières informations de fluorescence est une valeur CV de la première intensité de fluorescence.

12. Procédé d'analyse selon la revendication 6, dans lequel
lors de la délivrance en sortie, lorsqu'un nombre de particules présentant une première caractéristique est inférieur à une valeur de seuil correspondante, la particule présentant une première caractéristique est spécifiée en tant que troisième cellule anormale, et
les informations indiquant qu'un lymphocyte anormal est présent sont délivrées en sortie en tant qu'informations indiquant la présence ou l'absence d'une troisième cellule anormale.

13. Procédé d'analyse selon la revendication 6, dans lequel
lors de la délivrance en sortie, lorsque des informations indiquant la présence ou l'absence d'une quatrième cellule anormale sont en outre délivrées en sortie, et
l'information de la particule présentant une première caractéristique satisfait les conditions suivantes 1), 2) et 5) :
1) un nombre de particules présentant une première caractéristique est supérieur ou égal à une valeur de seuil correspondante ;
2) les informations sur la dispersion des premières informations de fluorescence de la particule présentant une première caractéristique sont supérieures ou égales à un seuil correspondant ; et
5) les informations de lumière dispersée vers l'avant de la particule présentant une première caractéristique sont inférieures à une valeur de seuil correspondante,
la particule présentant une première caractéristique est spécifiée en tant que quatrième cellule anormale, et
des informations indiquant la présence d'un érythroblaste immature ou d'un mégaloblaste sont délivrées en sortie en tant qu'informations indiquant la présence ou l'absence d'une quatrième cellule anormale.

14. Procédé d'analyse selon la revendication 13, dans lequel l'information sur la dispersion des premières informations de fluorescence est une valeur CV de la première intensité de fluorescence, et l'information de lumière dispersée vers l'avant est l'intensité de lumière dispersée vers l'avant.

15. Procédé d'analyse selon la revendication 10, dans lequel
le blaste est un lymphoblaste ou un myéloblaste, et
des informations indiquant qu'un lymphoblaste ou un myéloblaste est présent sont délivrées en sortie en tant qu'informations indiquant la présence ou l'absence d'une deuxième cellule anormale sur la base des informations de la particule présentant une première caractéristique et des informations de la première population de lymphocytes.

16. Procédé d'analyse selon la revendication 15, dans lequel
lors de la délivrance en sortie,
lorsque la particule présentant une première caractéristique satisfait les conditions 1) et 4) et les informations sur la dispersion des informations de lumière dispersée latéralement de la première population de lymphocytes sont inférieures à une valeur de seuil correspondante, des informations indiquant qu'un lymphoblaste est présent sont délivrées en sortie en tant qu'informations indiquant la présence ou l'absence d'une deuxième cellule anormale, et
lorsque la particule présentant une première caractéristique satisfait les conditions 1) et 4) et les informations sur la dispersion des informations de lumière dispersée latéralement de la première population de lymphocytes sont supérieures ou égales à la valeur de seuil correspondante, des informations indiquant qu'un myéloblaste est présent sont délivrées en sortie en tant qu'informations indiquant la présence ou l'absence d'une deuxième cellule anormale.

17. Procédé d'analyse selon la revendication 16, dans lequel l'information sur la dispersion des informations de lumière dispersée latéralement est une valeur CV de l'intensité de lumière dispersée latérale.
